(19) Europäisches Patentamt
European Patent Office
Office européen des brevets

(11) EP 0 657 426 B1

(12) # EUROPÄISCHE PATENTSCHRIFT

(45) Veröffentlichungstag und Bekanntmachung des Hinweises auf die Patenterteilung:
**22.07.1998 Patentblatt 1998/30**

(51) Int. Cl.[6]: **C07D 209/52**, C07D 209/60, C07D 491/04, C07D 495/04, A61K 31/40

(21) Anmeldenummer: **94115812.3**

(22) Anmeldetag: **07.10.1994**

(54) **Tricyclische Pyrrol-Derivate**
Tricyclic pyrrole derivatives
Dérivés tricycliques de pyrrole

(84) Benannte Vertragsstaaten:
**AT BE CH DE DK ES FR GB GR IE IT LI LU MC NL PT SE**

(30) Priorität: **22.10.1993 CH 3202/93**
**17.08.1994 CH 2529/94**

(43) Veröffentlichungstag der Anmeldung:
**14.06.1995 Patentblatt 1995/24**

(73) Patentinhaber:
**F. HOFFMANN-LA ROCHE AG**
**4002 Basel (CH)**

(72) Erfinder:
- **Bös, Michael**
  **CH-4310 Rheinfelden (CH)**
- **Wichmann, Jürgen**
  **D-79541 Lörrach (DE)**
- **Widmer, Ulrich**
  **CH-4310 Rheinfelden (CH)**

(74) Vertreter: **Poppe, Regina et al**
**F.Hoffmann-La Roche AG**
**Patent Department(PLP),**
**124 Grenzacherstrasse**
**4070 Basel (CH)**

(56) Entgegenhaltungen:
**EP-A- 0 521 368**
**WO-A-94/14777**
**US-A- 3 997 557**

EP 0 657 426 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft tricyclische Pyrrol-Derivate, im speziellen betrifft sie Pyrrol-Derivate der allgemeinen Formel

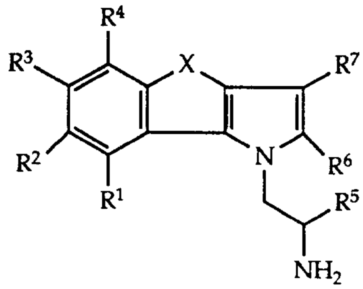

I

worin

| | |
|---|---|
| $R^1$ bis $R^4$ | je Wasserstoff, Halogen, **$C_1$-$C_7$**-Alkyl, Phenyl, **$C_5$-$C_6$**-Cycloalkyl oder **$C_1$-$C_7$**-Alkoxy und $R^2$ noch zusätzlich **$C_1$-$C_7$**-Alkoxycarbonyl, **Acetyloxy** oder Mesyloxy; |
| **$R^5$** | **$C_1$-$C_7$-Alkyl;** |
| **$R^6$ und** $R^7$ | je Wasserstoff oder **$C_1$-$C_7$**-Alkyl; |
| X | $-CH_2CH(C_6H_5)-$, $-CH{=}C(C_6H_5)-$, $-YCH_2-$, $-CH{=}CH-$ oder $-(CR^{11}R^{12})_n$; |
| $R^{11}$ und $R^{12}$ | je Wasserstoff, Phenyl, **$C_1$-$C_7$**-Alkyl oder Halogen; |
| n | 1 bis 3 und |
| Y | O oder S bedeuten, |

sowie pharmazeutisch annehmbare Salze von basischen Verbindungen der Formel I mit Säuren.

Diese Verbindungen und Salze sind neu und zeichnen sich durch wertvolle pharmakologische Eigenschaften aus.

Gegenstand der vorliegenden Erfindung sind Verbindungen der allgemeinen Formel I und pharmazeutisch annehmbare Salze davon als solche und als pharmazeutische Wirkstoffe, die Herstellung der Verbindungen und Salze der allgemeinen Formel I, ferner Arzneimittel, die diese Verbindungen und Salze der Formel I enthalten und die Herstellung dieser Arzneimittel, sowie die Verwendung von Verbindungen der allgemeinen Formel I und von pharmazeutisch anwendbaren Salzen davon bei der Bekämpfung bzw. Verhütung von Krankheiten bzw. bei der Verbesserung der Gesundheit, insbesondere bei der Bekämpfung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung entsprechender Arzneimittel.

Weiterhin sind Gegenstand der Erfindung die Verbindungen der allgemeinen Formel

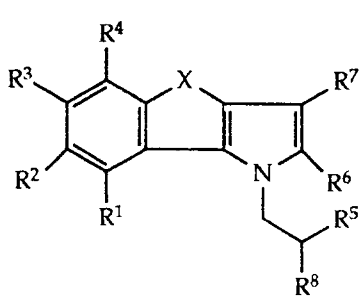

II

worin $R^1$ bis $R^7$ die oben genannten Bedeutungen haben und $R^8$ einen in eine Aminogruppe überführbaren Rest oder eine Hydroxygruppe bedeutet.

Diese Verbindungen sind wichtige Zwischenprodukte zur Herstellung der pharmazeutisch wertvollen Verbindungen der allgemeinen Formel I.

In der Europäische Patentschrift Nr. 521 388 werden bi- und tetracyclische Pyrrolopyrazine beschrieben, die beispielsweise mit Hilfe Von Zwischenprodukten hergestellt werden können, die strukturell ähnliche tricyclische Pyrrolderivate betreffen.

US 3,997,557 beschreibt substituierte N-Aminoalkyl-pyrrole, die in $R^6$-Position durch einen gegebenenfalls substituierten Phenylring substituiert sind.

Der in der vorliegenden Beschreibung verwendete Ausdruck "nieder" bezeichnet Reste mit höchstens 7, vorzugsweise bis 4 Kohlenwasserstoffatomen, "Alkyl" bezeichnet geradkettige oder verzweigte, gesättigte Kohlenwasserstoffreste wie Methyl, Ethyl, Propyl, Isopropyl oder t-Butyl, und "Alkoxy" bezeichnet eine über ein Sauerstoffatom gebundene Alkylgruppe, "Halogen" kann Cl, Br, F oder J bedeuten.

Der Ausdruck "pharmazeutisch annehmbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen.

$R^5$ kann zweckmassigerweise vorzugsweise Methyl bedeuten.

Bedeutet $R^5$ Methyl , sind Verbindungen besonders bevorzugt, worin $R^1$, $R^3$ und $R^4$ Wasserstoff und $R^2$ Halogen, **$C_1$-$C_7$** Alkyl oder Methoxy bedeuten.

Einige im Rahmen der vorliegenden Erfindung ganz besonders bevorzugte Vertreter der durch die allgemeine Formel I definierten Stoffklasse sind:

(S)-2-(4,4,7-Trimethyl-1,4-dihydro-indeno[1,2-b]pyrrol-1-yl)-1-methyl-ethylamin,

(S)-2-(7-Methoxy-4,4-dimethyl-1,4-dihydro-indeno[1,2-b]pyrrol-1-yl)-1-methyl-ethylamin,

(S)-2-(7-Ethyl-4,4-dimethyl-1,4-dihydro-indeno[1,2-b]pyrrol-1-yl)-1-methyl-ethylamin,

(S)-2-(7-Methyl-1,4-dihydro-indeno[1,2-b]pyrrol-1-yl)-1-methyl-ethylamin,

(S)-2-(7-Brom-1,4-dihydro-indeno[1,2-b]pyrrol-1-yl)-1-methyl-ethylamin,

(S)-2-(7-Methoxy-1,4-dihydro-indeno[1,2-b]pyrrol-1-yl)-1-methyl-ethyl amin,

(S)-2-(7-Chlor-1,4-dihydro-indeno[1,2-b]pyrrol-1-yl)-1-methyl-ethylamin,

(S)-2-(8-Methoxy-1H-benz[g]indol-1-yl)-1-methyl-ethylamin.

Verbindungen der allgemeinen Formel I sowie ihre pharmazeutisch annehmbaren Salze können erfindungsgemäss hergestellt werden, indem man

a) eine Verbindung der allgemeinen Formel

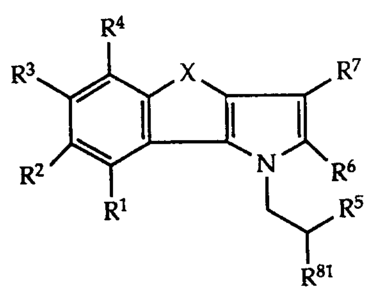

IIa

worin $R^1$ bis $R^7$ die oben angegebenen Bedeutungen haben und $R^{81}$ einen in eine Aminogruppe überführbaren Rest bedeutet,

in eine entsprechende Aminoverbindung überführt und
b) erwünschtenfalls die erhaltene Verbindung der Formel I in ein pharmazeutisch annehmbares Salz überführt.

Die Verbindungen der allgemeinen Formel IIa, worin $R^{81}$ einen in eine Aminogruppe überführbaren Rest, vorzugsweise eine Azidgruppe, eine Acetylaminogruppe oder eine andere geschützte Aminogruppe bedeutet, können wie weiter unten beschrieben nach an sich bekannten Methoden hergestellt werden. Ist der Rest $R^{81}$ eine Azidgruppe, erfolgt die Herstellung der Verbindungen der Formel I durch Reduktion. Diese kann mit komplexen Hydriden, wie z.B. Lithiumaluminiumhydrid oder durch katalytische Hydrierung an Metallkatalysatoren, wie z.B. Platin oder Palladium, durchgeführt werden. Verwendet man Lithiumaluminiumhydrid als Reduktionsmittel, sind vor allem wasserfreier Aether oder Tetrahydrofuran als Lösungsmittel geeignet. Zweckmässigerweise kann man wie folgt vorgehen: Nach dem Zutropfen der Verbindung der Formel IIa, worin $R^{81}$ eine Azidgruppe bedeutet, zu einer Lösung, bestehend aus dem wasserfreien Lösungsmittel und dem Hydrid, wird am Rückfluss gekocht, anschliessend mit wässriger Aether- oder THF-Lösung hydrolysiert und der Aluminium- und Lithiumhydroxid-Niederschlag mit wässriger Aether- oder THF-Lösung ausgekocht.

Die katalytische Hydrierung an Metallkatalysatoren, z.B. Platin oder Palladium, erfolgt zweckmässigerweise bei Raumtemperatur. Als Lösungsmittel sind dazu besonders geeignet: Wasser, Alkohole, Essigester, Dioxan oder Gemische dieser Lösungsmittel. Die Hydrierung erfolgt unter Wasserstoffatmosphäre zweckmässigerweise in einem Autoklaven oder in einer Schüttelapparatur. Bedeutet $R^{81}$ eine Acetylaminogruppe oder eine andere geschützte Aminogruppe, wie z.B. Trifluormethylcarbonylamino, erfolgt eine Ueberführung in die entsprechende Aminoverbindung durch Hydrolyse.

Die Hydrolyse zu den entsprechenden Aminoverbindungen der allgemeinen Formel I erfolgt nach an sich bekannten Methoden. Es eignen sich dafür Metallhydroxide, beispielsweise Natrium- oder Kaliumhydroxid, die in Anwesenheit von Wasser und einem mit Wasser mischbaren organischen Lösungsmittel, wie Ethylenglykol oder dergleichen, zu den Verbindungen der Formel I hydrolysieren.

Die Ueberführung der Verbindungen der Formel I in ihre entsprechenden Säureadditionssalze erfolgt im letzten Arbeitsgang, d.h. nach der Hydrierung oder Hydrolyse ohne Zwischenisolierung der Verbindungen der Formel I.

Aus Stabilitätsgründen eignen sich die Salze der Fumarsäure besonders gut für eine pharmazeutische Anwendung. Aber auch alle anderen in der Beschreibung erwähnten Säuren bilden pharmakologisch annehmbare Salze. Die Salzbildung erfolgt bei Raumtemperatur, als Lösungsmittel sind Alkohol-Ethergemische besonders geeignet.

Die Herstellung von Zwischenprodukten der Formel II, die für die Synthese der Verbindungen der allgemeinen Formel I benötigt werden, ist im Schema 1 dargestellt. Hierbei haben alle Substituenten $R^1$ bis $R^4$ die in der Formel I angegebenen Bedeutungen. $R^{51}$, $R^{61}$ und $R^{71}$ bedeuten Methyl. Me bedeutet Methyl und Ac ist Acetyl. X hat ebenfalls die in der Formel I angegebene Bedeutung, ausser -CH=CH-; die Herstellung von entsprechenden

Verbindungen, worin X -CH=CH- bedeutet, zeigt das Schema 2.

**Schema 1**

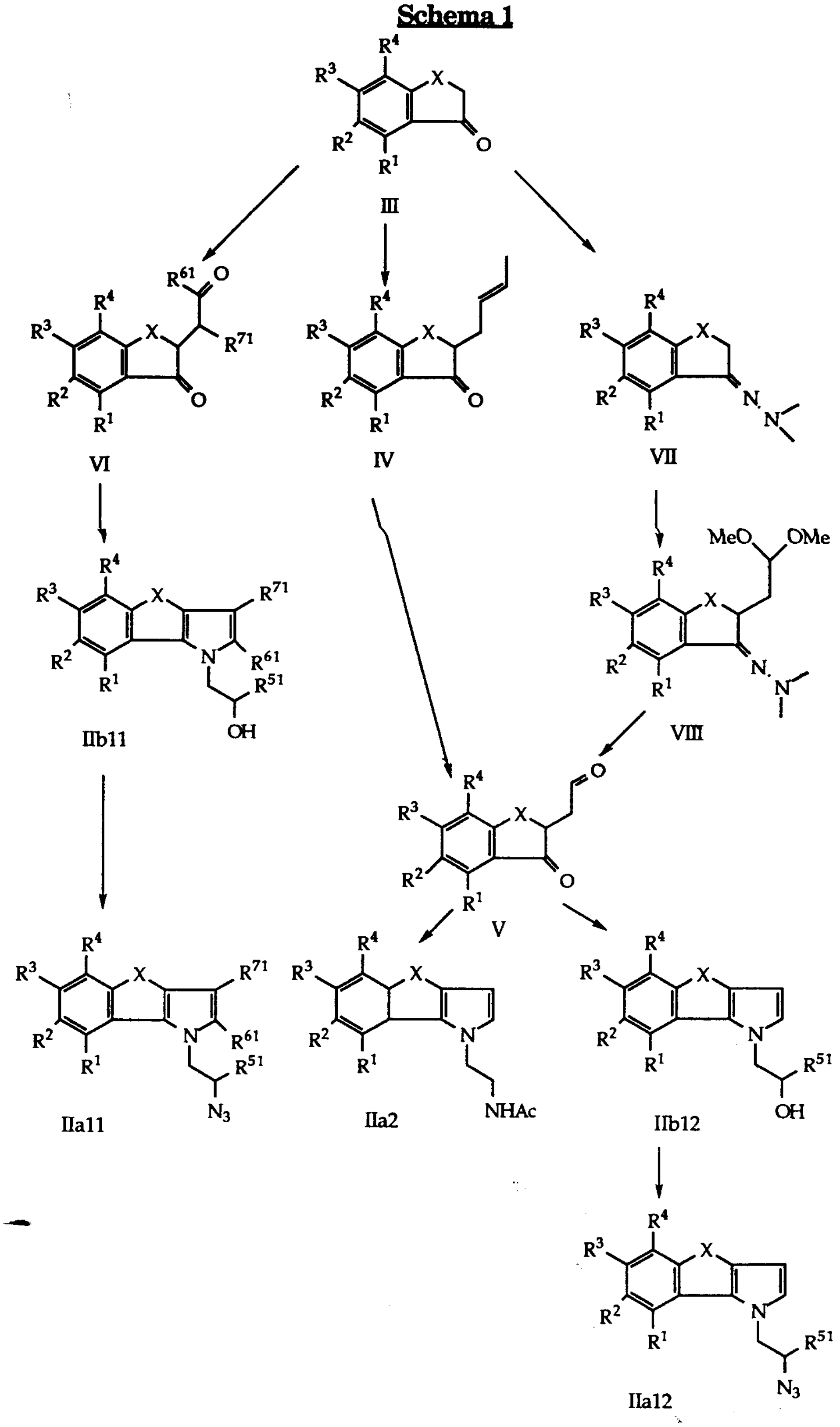

III, VI, IV, VII, IIb11, VIII, V, IIa11, IIa2, IIb12, IIa12

Die Herstellung der Verbindungen der Formel IIa2, ausgehend von der Verbindung III, erfolgt zweckmässigerweise wie folgt:

Eine Verbindung der Formel III wird mit 3-Buten-2-ol, 2,2-Dimethoxypropan und katalytischen Mengen p-Toluolsulfonsäure in wasserfreiem Toluol unter Rückfluss erhitzt. In einer weiteren Variante dient 2,2-Dimethoxypropan unter Verzicht auf Toluol als Lösungsmittel. Hierbei wird das Reaktionsgemisch an einem mit Molekularsieb gefüllten Wasserabscheider am Rückfluss erhitzt. Anschliessend werden die so entstandenen Verbindungen der Formel IV in die entsprechenden Oxo-ethylverbindungen überführt. Dazu wird die Verbindung IV in einem wasserfreien Lösungsmittel gelöst, beispielsweise in Dichlormethan und Methanol, und bei ca. -70°C mit einem Ozonstrom behandelt. Dann kann die Verbindung der Formel V zu den Pyrrol-Acetamino-Verbindungen cyclisiert werden. Zweckmässigerweise verwendet man N-Acetylethylendiamin und als Lösungsmittel Toluol oder Essigsäure. Nach erfolgter Umsetzung und Reinigung kann die Acetylgruppe, wie beschrieben, abgespalten und die Verbindung der Formel IIa2 in eine Verbindung der Formel I überführt werden.

Ausgehend von den Verbindungen der Formel V entsteht die Hydroxy-Verbindung der Formel IIb12 zweckmässigerweise durch Cyclisierung mit 1-Amino-2-propanol in wasserfreiem Toluol mit katalytischen Mengen p-Toluolsulfonsäure durch Erhitzen am Wasserabscheider. Anschliessend kann die Hydroxygruppe nach an sich bekannten Methoden in eine Abgangsgruppe überführt werden, beispielsweise durch die Umsetzung mit einem Sulfonsäurechlorid, vorzugsweise Methansulfonsäurechlorid, zum Sulfonat. Durch Behandlung mit einem Azid, vorzugsweise Natriumazid, in einem polaren Lösungsmittel, wie z.B. DMF, können Verbindungen der Formel IIb12 in die entsprechenden Azidoverbindungen der Formel IIa12 übergeführt werden, die, wie beschrieben, durch Reduktion der Azidgruppe in Verbindungen der Formel I überführt werden können.

Die Verbindungen der Formel V können gemäss Schema 1 auch auf andere Weise hergestellt werden.

Eine Verbindung der Formel III wird zweckmässigerweise mit N,N-Dimethylhydrazin unter Argon erhitzt und dann über eine Vigreuxkolonne destilliert. Die erhaltene Verbindung der Formel VII wird mit DMPU (1,3-Dimethyl-3,4,5,6-tetrahydro-2(1H)-pyrimidinon) versetzt, in wasserfreiem THF gelöst, auf ca. -75°C abgekühlt und anschliessend mit n-Butyllithium in Hexan sowie Bromacetaldehyddimethylacetal versetzt.

Die entstandene Verbindung der Formel VIII wird anschliessend mittels Phosphatpuffer und Kupfer(II)chloriddihydrat in THF in die Verbindung der Formel V überführt.

Gemäss Schema 1 können auch Verbindungen der Formel IIb11 dargestellt werden. Diese Verbindungen können dann, wie beschrieben, in die Verbindungen der Formel I überführt werden. Zweckmässigerweise kann man wie folgt vorgehen: Eine Verbindung der Formel III, gelöst in wasserfreiem THF, wird mit Diisopropylamin und n-Butyllithium versetzt und anschliessend mit Chloraceton bzw. 3-Chlor-2-butanon behandelt. Die entstandenen Verbindungen VI werden in wasserfreiem Toluol mit katalytischen Mengen p-Toluolsulfonsäure am Wasserabscheider erhitzt. Dann behandelt man mit 1-Amino-2-propanol zu den Verbindungen der Formel IIb11.

Das Schema 2 zeigt die Herstellung der Verbindungen der Formel IIa22, worin $R^1$ bis $R^7$ die oben angegebenen Bedeutungen haben und X -CH=CH- bedeutet. $R^{10}$ kann eine Methyl- oder Trifluormethylgruppe sein.

## Schema 2

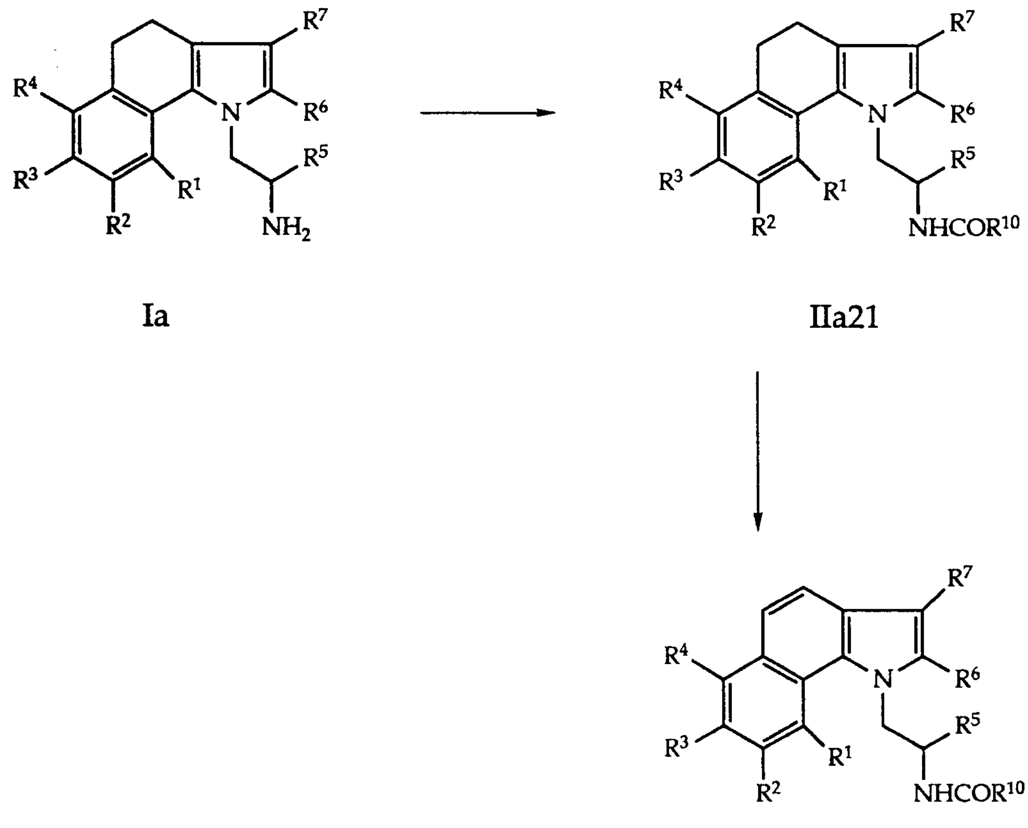

Ia

IIa21

IIa22

Zweckmässigerweise geht man wie folgt vor:

Eine Verbindung der Formel Ia wird in einer Lösung, bestehend aus Triethylamin und Trifluoressigsäureethylester in einem wasserfreien Lösungsmittel, vorzugsweise Methanol umgesetzt. Nach Abzug des Lösungsmittels wird der Rückstand in Dioxan aufgenommen und mit DDQ (2,3-Dichlor-5,6-dicyan-1,4-benzochinon) versetzt. Anschliessend kann die Schutzgruppe, wie beschrieben, von der Aminogruppe abgespalten werden.

Wie eingangs erwähnt, besitzen die Verbindungen der Formel I und ihre pharmazeutisch annehmbaren Salze wertvolle pharmakologische Eigenschaften. Sie sind in der Lage, an Serotonin-Rezeptoren zu binden und eignen sich daher zur Behandlung oder Verhütung von Krankheiten oder Störungen der eingangs erwähnten Art bzw. zur Herstellung entsprechender Arzneimittel.

Die Bindung der erfindungsgemässen Verbindungen der Formel I an die Serotonin-Rezeptoren wurde durch Standardmethoden in vitro bestimmt. Die Präparate wurden gemäss den nachfolgend angegebenen Tests geprüft:

Methode I :

a) Für die Bindung an den $5HT_{1A}$-Rezeptor gemäss dem $^3$H-8-OH-DPAT-Bindungs-Test nach der Methode von S.J. Peroutka, Biol. Psychiatry 20, 971-979 (1985).

b) Für die Bindung an den $5HT_{2C}$-Rezeptor gemäss dem $^3$H-Mesulergin-Bindungs-Test nach der Methode von A. Pazos et al., Europ. J. Pharmacol. 106, 539-546 bzw. D. Hoyer, Receptor Research 8, 59-81 (1988).

c) Für die Bindung an den $5HT_{2A}$-Rezeptor gemäss dem $^3$H-Ketanserin-Bindungs-Test nach der Methode von J.E. Leysen, Molecular Pharmacology 21, 301-304 (1981).

Es wurden die $IC_{50}$-Werte der zu prüfenden Substanzen bestimmt, d.h. diejenige Konzentration in nM, durch welche 50% des am Rezeptor gebundenen Liganden verdrängt werden.

Die so ermittelte Aktivität einiger erfindungsgemässer Verbindungen sowie diejenige einiger Vergleichsverbindungen ist aus nachfolgender Tabelle ersichtlich:

| | Testmethode a | Testmethode b | Testmethode c |
|---|---|---|---|
| Buspiron | 19.50 | 3700.0 | 990.0 |
| NAN-190 | 0.56 | 1800.0 | 581.0 |
| 5HT | 1.50 | 9.5 | 1730.0 |
| Metergolin | 4.80 | 5.5 | 64.9 |
| mCPP | 227.00 | 53.0 | 319.0 |
| RU 24969 | 8.0 | 159.0 | 2500.0 |
| CP 93129 | 1620.00 | 2780.0 | 29200.0 |
| Ritanserin | 5750.00 | 37.0 | 3.1 |
| Pirenperon | 2879.00 | 37.0 | 2.9 |
| 3 | 2830 | 11.4 | 2160 |
| 4 | 3230 | 49.3 | 2170 |
| 5 | 2560 | 20.2 | 591 |
| 7 | 2160 | 24.0 | 1200 |
| 21 | 1350 | 56.6 | 2540 |
| 22 | 1590 | 30.4 | 1400 |
| 27 | 1070 | 78.7 | 1630 |
| 29 | 298 | 58.1 | 479 |
| 30 | 337 | 28.1 | 874 |
| 32 | 1620 | 107.0 | 2640 |
| 50 | 1100 | 308.0 | 11300 |
| 51 | 825 | 116.0 | 2590 |
| 52 | 1260 | 371.0 | 8350 |
| 54 | 384 | 85.4 | 773 |
| 55 | 1750 | 146.0 | 1070 |
| 57 | 3460 | 143.0 | 854 |

3 = (S)-2-(7-Methoxy-1,4-dihydro-indeno[1,2-b]pyrrol-1-yl)-1-methyl-ethylamin
4 = (S)-2-(7-Chlor-1,4-dihydro-indeno[1,2-b]pyrrol-1-yl)-1-methyl-ethylamin
5 = (S)-2-(8-Methoxy-4,5-dihydro-1H-benz[g]indol-1-yl)-1-methyl-ethylamin
7 = (S)-2-(8-Methoxy-1H-benz[g]indol-1-yl)-1-methyl-ethylamin
21 = (RS)-2-(7-Chlor-1,4-dihydro-indeno[1,2-b]pyrrol-1-yl)-1-methyl-ethylamin
22 = (RS)-2-(7-Methoxy-1,4-dihydro-indeno[1,2-b]pyrrol-1-yl)-1-methyl-ethylamin
27 = (R)-2-(7-Methoxy-1,4-dihydro-indeno[1,2-b]pyrrol-1-yl)-1-methyl-ethylamin
29 = (R)-2-(8-Methoxy-4,5-dihydro-1H-benz[g]indol-1-yl)-1-methyl-ethylamin
30 = (R)-2-(8-Methoxy-1H-benz[g]indol-1-yl)-1-methyl-ethylamin
32 = (RS)-1-(1,4-Dihydro[1]benzopyrano[4,3-b]pyrrol-1-yl)-1-methyl-ethylamin
50 = (RS)-2-(6-Chlor-1,4-dihydro-indeno[1,2-b]pyrrol-1-yl)-1-methyl-ethylamin
51 = (RS)-1-(8-Methoxy-1,4-dihydro[1]benzopyrano[4,3-b]pyrrol-1-yl)-1-methyl-ethylamin
52 = (RS)-2-(6-Methoxy-1,4-dihydro-indeno[1,2-b]pyrrol-1-yl)-1-methyl-ethylamin
53 = (RS)-1-(1,4-Dihydro[1]benzothiopyrano[4,3-b]pyrrol-1-yl)-1-methyl-ethylamin
55 = (RS)-2-(8-Methoxy-1,4-dihydro-indeno[1,2-b]pyrrol-1-yl)-1-methyl-ethylamin
57 = (2RS/4RS)-2-(4-Phenyl-1,4-dihydro-indeno[1,2-b]pyrrol-1-yl)-1-methyl-ethylamin

Methode II:

a) Zur Bestimmung der Affinität einer Verbindung zum $5HT_{1A}$-Rezeptor wurden Verdrängungsversuche mit [3H]-5-HT (1 nM) als Radioligand an rekombinierten human-$5HT_{1A}$ Rezeptoren expremiert in 3T3-Zellen von Mäusen durchgeführt. Es wurden Membranen verwendet` die von $2x10^5$ Zellen erhalten wurden, sowie verschiedene Konzentrationen der jeweiligen Testverbindung.
b) Für die Bindung an den $5HT_{2C}$-Rezeptor gemäss dem [3H]-5-HT-Bindungs-Test nach der Methode von S.J. Peroutka et al., Brain Research 584, 191-196 (1992).
c) Für die Bindung an den $5HT_{2A}$-Rezeptor gemäss dem [3H]-DOB-Bindungs-Test nach der Methode von T. Branchek et. al., Molecular Pharmacology 38, 604-609 (1990).

Es sind die $p_{ki}$-Werte ($p_{ki} = -\log_{10} Ki$) der zu prüfenden Substanzen angegeben. Der ki-Wert ist durch folgende Formel definiert:

$$Ki = \frac{IC_{50}}{1+\frac{[L]}{K_D}}$$

wobei die $IC_{50}$-Werte diejenigen Konzentrationen der zu prüfenden Verbindungen in nM sind, durch welche 50% des

am Rezeptor gebundenen Liganden verdrängt werden. [L] ist die Konzentration des Liganden und der $K_D$-Wert die Dissoziationskonstante des Liganden.
Die so ermittelte Aktivität einiger erfindungsgemässer Verbindungen ist aus nachfolgender Tabelle ersichtlich:

| | Testmethode | | |
|---|---|---|---|
| | a | b | c |
| 1 | 5.66 | 8.49 | 7.27 |
| 2 | 5.39 | 9.44 | 8.03 |
| 3 | | 8.16 | 7.12 |
| 8 | 5.34 | 8.63 | 7.18 |
| 9 | 5.56 | 8.29 | 7.46 |
| 12 | 5.00 | 8.00 | 6.94 |
| 14 | 5.00 | 7.78 | 7.62 |
| 17 | 5.00 | 7.74 | 6.25 |
| **24** | 5.00 | 7.38 | 6.26 |
| 26 | 5.25 | 7.58 | 7.17 |
| **35** | 5.32 | 7.45 | 6.51 |
| **42** 44 | 5.73 | 7.10 | 6.57 |
| **48** | 5.62 | 6.97 | 6.35 |

1 = (S)-2-(4,4,7-Trimethyl-1,4-dihydro-indeno[1,2-b]pyrrol-1-yl)-1-methyl-ethylamin fumarat (1:1)
2 =(S)-2-(7-Methoxy-4,4-dimethyl-1,4-dihydro-indeno[1,2-b]pyrrol-1-yl)-1-methyl-ethylamin fumarat (1:1)
8 =(S)-2-(7-Ethyl-4,4-dimethyl-1,4-dihydro-indeno[1,2-b]pyrrol-1-yl)-1-methyl-ethylamin fumarat (1:1)
9 =(S)-2-(7-Fluor-4,4-dimethyl-1,4-dihydro-indeno[1,2-b]pyrrol-1-yl)-1-methyl-ethylamin fumarat (1:1)
12 =(S)-2-(7-Methoxy-4,4-diethyl-1,4-dihydro-indeno[1,2-b]pyrrol-1-yl)-1-methyl-ethylamin fumarat (1:0.5)
14 =(S)-2-(7-Phenyl-1,4-dihydro-indeno[1,2-b]pyrrol-1-yl)-1-methyl-ethylamin fumarat (1:0.5)
17 =(S)-2-(7-Ethyl-1,4-dihydro-indeno[1,2-b]pyrrol-1-yl)-1-methyl-ethylamin fumarat (1:0.5)
**24** =(S)-2-(6,7-Difluor-1,4-dihydro-indeno[1,2-b]pyrrol-1-yl)-1-methyl-ethylamin fumarat (1:0.5)
**25** =(S)-2-(6-Chlor-7-methoxy-4,4-dimethyl-1,4-dihydro-indeno[1,2-b]pyrrol-1-yl)-1-methyl-ethylamin-fumarat (1:0.5)
**35** =(RS)-2-(5-Chlor-1,4-dihydro-indeno[1,2-b]pyrrol-1-yl)-1-methyl-ethylamin fumarat (1:0.5)
**42** 44 =(S)-1-Methyl-2-(7'-methyl-1',4'-dihydro-spiro[cyclopentan-1,4'-indeno[1,2-b]pyrrol]-1-yl)-ethylamin fumarat (1:1)
**48** =(RS)-2-(5-Methyl-1,4-dihydro-indeno[1,2-b]pyrrol-1-yl)-1-methyl-ethylamin fumarat (1:0.5)

Peniserektionen (Ratten)

Es wurde gezeigt, dass die Peniserektion abhängig ist von der Stimulierung des $5HT_{2C}$ Receptors (vergl. Berendsen & Broekkamp, Eur. J. Pharmacol. 135, 179 - 184 (1987).
Innerhalb 45 Minuten nach Verabreichen der Testsubstanz an die Tiere wurde die Anzahl der Peniserektionen bestimmt. Die $ED_{50}$ ist die Dosis, die 50% dieser Erektionen hervorruft.

| Beispiel No. | $ED_{50}$ (mg/kg, s. c.) |
|---|---|
| 1 | 0,90 |
| 2 | 0,50 |
| 3 | 1,20 |

(fortgesetzt)

| Beispiel No. | $ED_{50}$ (mg/kg, s. c.) |
|---|---|
| 4 | 2,70 |
| 5 | 3,20 |

Die Verbindungen der Formel I und die pharmazeutisch annehmbaren Säureadditionssalze der Verbindungen der Formel I können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, parenteral, z.B. in Form von Injektionslösungen, oder nasal erfolgen.

Zur Herstellung von pharmazeutischen Präparaten können die Verbindungen der Formel I und die pharmazeutisch annehmbaren Säureadditionssalze der Verbindungen der Formel I mit pharmazeutisch inerten, anorganischen oder organischen Trägern verabreicht werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Glyzerin, pflanzliches Oel und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Arzneimittel, enthaltend eine Verbindung der Formel I oder ein pharmazeutisch annehmbares Säureadditionssalz davon und einen therapeutisch inerten Träger, sind ebenfalls Gegenstand der vorliegenden Erfindung, ebenso ein Verfahren zu deren Herstellung, das dadurch gekennzeichnet ist, dass man eine oder mehrere Verbindungen der Formel I und/oder pharmazeutisch annehmbare Säureadditionssalze zusammen mit einem oder mehreren therapeutisch inerten Trägern in eine galenische Darreichungsform bringt.

Erfindungsgemäss kann man Verbindungen der allgemeinen Formel I sowie deren pharmazeutisch annehmbare Säureadditionssalze bei der Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung entsprechender Arzneimittel verwenden. Die Dosierung kann innerhalb weiter Grenzen variieren und ist natürlich in jedem einzelnen Fall den individuellen Gegebenheiten anzupassen. Bei oraler Verabreichung liegt die Dosis in einem Bereich von etwa 0,01 mg pro Dosis bis etwa 500 mg pro Tag einer Verbindung der allgemeinen Formel I bzw. der entsprechenden Menge eines pharmazeutisch annehmbaren Säureadditionssalzes davon, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

a) Eine Lösung von 18.9 g (108 mmol) 3,3,6-Trimethyl-1-indanon, 22.4 ml (0.26 mol) 3-Buten-2-ol und 300 mg p-Toluolsulfonsäure in 200 ml 2,2-Dimethoxypropan wurde 64 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 6:1). Man erhielt neben 4.5 g Edukt 12.7 g (51%) (RS)-2-(2-Buten-1-yl)-3,3,6-trimethyl-1-indanon als gelbes Öl.

b) Durch eine auf -70° gekühlte Lösung von 12.7 g (55.6 mmol) (RS)-2-(2-Buten-1-yl)-3,3,6-trimethyl-1-indanon in 200 ml wasserfreiem Dichlormethan und 40 ml wasserfreiem Methanol leitete man unter Rühren während 60 Minuten einen Ozonstrom (2.5 g Ozon/Stunde). Anschliessend spülte man die Lösung während 5 Minuten mit Sauerstoff und während 10 Minuten mit Argon. Nach Zugabe von 6.12 ml (83.4 mmol) Dimethylsulfid rührte man 15

Stunden bei Raumtemperatur. Das Reaktionsgemisch wurde im Vakuum eingedampft, der Rückstand mit 150 ml Dichlormethan versetzt und nach Zugabe von 25 ml Wasser und 25 ml Trifluoressigsäure 2.5 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 11.3 g (94%) (RS)-2-(2-Oxoethyl)-3,3,6-trimethyl-1-indanon als schwach gelbes Öl.

c) Eine Lösung von 2.16 g (10 mmol) (RS)-2-(2-Oxoethyl)-3,3,6-trimethyl-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 1:2) gereinigt. Man erhielt 1.5 g (59%) (R)-1-(4,4,7-Trimethyl-1,4-dihydro-indeno[1,2-b]pyrrol-1-yl)-propan-2-ol als braunes Öl.

d) Zu einer auf 0° gekühlten Lösung von 1.5 g (5.87 mmol) (R)-1-(4,4,7-Trimethyl-1,4-dihydro-indeno[1,2-b]pyrrol-1-yl)-propan-2-ol und 3.27 ml (23.5 mmol) Triethylamin in 50 ml Dichlormethan gab man tropfenweise unter Rühren 0.91 ml (11.7 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhy-drogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Der erhaltene grüne Feststoff wurde in 50 ml wasserfreiem Dimethylformamid gelöst, mit 0.76 g (11.7 mmol) Natriumazid versetzt und das Reaktionsgemisch unter Rühren 15 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 1.13 g (68%) (S)-1-(2-Azido-propyl)-4,4,7-trimethyl-1,4-dihydro-indeno[1,2-b]pyrrol als rötliches Öl.

e) 1.1 g (3.92 mmol) (S)-1-(2-Azido-propyl)-4,4,7-trimethyl-1,4-dihydro-indeno[1,2-b]-pyrrol gelöst in 50 ml wasserfreiem Ethanol wurden an 110 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 455 mg (3.92 mmol) Fumarsäure in 15 mi Methanol versetzt. Man rührte 24 Stunden bei Raumtemperatur und filtrierte anschliessend die weissen Kristalle ab. Man erhielt 805 mg (77%) (S)-2-(4,4,7-Trimethyl-1,4-dihydro-indeno[1,2-b]pyrrol-1-yl)-1-methyl-ethylamin fumarat (1:1) mit Smp. 196°.

Beispiel 2

a) Eine Lösung von 12.1 g (63.7 mmol) 6-Methoxy-3,3-dimethyl-1-indanon, 11.1 mi (0.13 mol) 3-Buten-2-ol und 110 mg p-Toluolsulfonsäure in 110 ml 2,2-Dimethoxy-propan wurde 67 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt neben 4.64 g Edukt 5.86 g (38%) (RS)-2-(2-Buten-1-yl)-6-methoxy-3,3-dimethyl-1-indanon als gelbes Öl.

b) Durch eine auf -70° gekühlte Lösung von 5.86 g (24 mmol) (RS)-2-(2-Buten-1-yl)-6-methoxy-3,3-dimethyl-1-indanon in 100 ml wasserfreiem Dichlormethan und 20 ml wasserfreiem Methanol leitete man unter Rühren während 40 Minuten einen Ozonstrom (3 g Ozon/Stunde). Anschliessend spülte man die Lösung während 5 Minuten mit Sauerstoff und während 10 Minuten mit Argon. Nach Zugabe von 2.64 ml (36 mmol) Dimethylsulfid rührte man 4 Stunden bei Raumtemperatur. Das Reaktionsgemisch wurde im Vakuum eingedampft, der Rückstand mit 60 ml Dichlormethan versetzt und nach Zugabe von 10 ml Wasser und 10 ml Trifluoressigsäure 2 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 4.94 g (89%) (RS)-2-(2-Oxoethyl)-6-methoxy-3,3-dimethyl-1-indanon als schwach gelbes Öl.

c) Eine Lösung von 4.94 g (21.3 mmol) (RS)-2-(2-Oxoethyl)-6-methoxy-3,3-dimethyl-1-indanon und 220 mg p-Toluolsulfonsäure in 200 ml wasserfreiem Toluol wurde am Wasserabscheider erhitzt. Zu der siedenden Lösung tropfte man über einen Zeitraum von 5 Minuten eine Lösung von 6.39 g (85.2 mmol) (R)-1-Amino-2-propanol in 40 ml wasserfreiem Toluol. Anschliessend wurde weitere 45 Minuten gekocht, wobei man das Lösungsmittel auf ein Volumen von 40 ml reduzierte. Das erkaltete Reaktionsgemisch wurde durch Säulenchromatographie an Kieselgel (Diethylether/Hexan 7:3) gereinigt. Man erhielt 3.42 g (60%) (R)-1-(7-Methoxy-4,4-dimethyl-1,4-dihydro-indeno[1,2-b]pyrrol-1-yl)-propan-2-ol als braunes Öl.

d) Zu einer auf 0° gekühlten Lösung von 3.42 g (12.7 mmol) (R)-1-(7-Methoxy-4,4-dimethyl-1,4-dihydro-indeno[1,2-b]pyrrol-1-yl)-propan-2-ol und 7.3 ml (50.8 mmol) Triethylamin in 70 ml Dichlormethan gab man tropfenweise unter Rühren 2.0 ml (25.4 mmol) Methansulfonylchlorid und rührte weitere 1.5 Stunden bei dieser Temperatur. Das Reaktionsgemisch wurde anschliessend mit 170 ml Dichlormethan verdünnt, zweimal mit jeweils 90 ml gesättigter Natriumhy-drogencarbonatlösung gewaschen und die vereinigten Wasserphasen einmal mit 70 ml Dichlormethan extrahiert. Die vereinigten organischen Phasen wurden mit 90 ml gesättigter Natriumchloridlösung gewaschen, über Magnesiumsulfat getrocknet und im Vakuum eingedampft. Das erhaltene grüne Öl wurde in 70 ml wasserfreiem Dimethylformamid gelöst, mit 1.35 g (20.4 mmol) Natriumazid versetzt und das Reaktionsgemisch unter Rühren 20 Stunden auf 60° erwärmt. Nach dem Abkühlen goss man die Lösung auf 100 ml Wasser und extrahierte dreimal mit jeweils 100 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 1.89 g (50%) (S)-1-(2-Azido-propyl)-7-methoxy-4,4-dimethyl-1,4-dihydro-indeno[1,2-b]pyrrol als gelbes Öl.

e) 1.89 g (6.38 mmol) (S)-1-(2-Azido-propyl)-7-methoxy-4,4-dimethyl-1,4-dihydro-indeno-[1,2-b]-pyrrol gelöst in 100 ml wasserfreiem Ethanol wurden an 190 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 740 mg (6.38 mmol) Fumarsäure in 15 ml Methanol versetzt. Man rührte 15 Stunden bei Raumtemperatur und filtrierte anschliessend die weissen Kristalle ab. Man erhielt 1.46 g (60%) (S)-2-(7-Methoxy-4,4-dimethyl-1,4-dihydro-indeno[1,2-b]pyrrol-1-yl)-1-methyl-ethylamin fumarat (1:1) mit Smp. 181°.

Beispiel 3

a) Eine Lösung von 9.3 g (57.3 mmol) 6-Methoxy-1-indanon, 11.8 ml (0.14 mol) 3-Buten-2-ol und 100 mg p-Toluolsulfonsäure in 100 ml 2,2-Dimethoxy-propan wurde 64 Stunden an einem mit Molekularsieb (0.4 nm, 2mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt 9.2 g (74%) (RS)-2-(2-Buten-1-yl)-6-methoxy-1-indanon als gelbes Öl.

b) Durch eine auf -70° gekühlte Lösung von 15.2 g (70.3 mmol) (RS)-2-(2-Buten-1-yl)-6-methoxy-1-indanon in 250 ml wasserfreiem Dichlormethan und 50 ml wasserfreiem Methanol leitete man unter Rühren während 80 Minuten einen Ozonstrom (3 g Ozon/Stunde). Anschliessend spülte man die Lösung während 5 Minuten mit Sauerstoff und während 10 Minuten mit Argon. Nach Zugabe von 7.75 ml (105 mmol) Dimethylsulfid rührte man 15 Stunden bei Raumtemperatur. Das Reaktionsgemisch wurde im Vakuum eingedampft, der Rückstand mit 250 ml Dichlormethan versetzt und nach Zugabe von 25 ml Wasser und 25 ml Trifluoressigsäure 3 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt aus Diethylether/Hexan kristallisiert. Man erhielt 12 g (83%) (RS)-2-(2-Oxoethyl)-6-methoxy-1-indanon als schwach gelben Feststoff mit Smp. 59°.

c) Eine Lösung von 2 g (9.8 mmol) (RS)-2-(2-Oxoethyl)-6-methoxy-1-inda-non und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.94 g (39.2 mmol) (R)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.5 g (63%) (R)-1-(7-Methoxy-1,4-dihydro-indeno[1,2-b]pyrrol-1-yl)-propan-2-ol als Feststoff mit Smp. 75°.

d) Zu einer auf 0° gekühlten Lösung von 1.5 g (6.2 mmol) (R)-1-(7-Methoxy-1,4-dihydro-indeno[1,2-b]pyrrol-1-yl)-propan-2-ol und 3.45 ml (24.6 mmol) Triethylamin in 50 ml Dichlormethan gab man tropfenweise unter Rühren 0.96 ml (12.3 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 60 ml wasserfreiem Dimethylformamid gelöst, mit 0.77 g (11.8 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.0 g (60%) (S)-1-(2-Azido-propyl)-7-methoxy-1,4-dihydro-indeno[1,2-b]pyrrol als farbloses Öl.

e) 1 g (3.7 mmol) (S)-1-(2-Azido-propyl)-7-methoxy-1,4-dihydro-indeno[1,2-b]pyrrol gelöst in 50 ml wasserfreiem Ethanol wurde an 100 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 240 mg (2.07 mmol) Fumarsaure in 20 ml Methanol versetzt. Man rührte 18 Stunden bei Raumtemperatur und filtrierte anschliessend die weissen Kristalle ab. Man erhielt 870 mg (77%) (S)-2-(7-Methoxy-1,4-dihydro-indeno[1,2-b]pyrrol-1-yl)-1-methyl-ethylamin fumarat (1:0.5) mit Smp. 206°.

<u>Beispiel 4</u>

a) Eine Lösung von 20.2 g (0.12 mol) 6-Chlor-1-indanon, 25 ml (0.29 mol) 3-Buten-2-ol und 200 mg p-Toluolsulfonsäure in 25 ml 2,2-Dimethoxypropan und 200 ml wasserfreiem Toluol wurde 16 Stunden unter Rückfluss gekocht. Das Reaktionsgemisch wurde anschliessend im Vakuum eingeengt und durch Säulenchromatographie an Kieselgel gereinigt (Hexan/ Diethylether 5:1). Man erhielt 10.3 g (39%) (RS)-2-(2-Buten-1-yl)-6-chlor-1-indanon als gelbes Öl.

b) Durch eine auf -70° gekühlte Lösung von 10.3 g (46.7 mmol) (RS)-2-(2-Buten-1-yl)-6-chlor-1-indanon in 200 ml wasserfreiem Dichlormethan und 100 ml wasserfreiem Methanol leitete man unter Rühren während 45 Minuten einen Ozonstrom (3 g Ozon/Stunde). Anschliessend spülte man die Lösung während 5 Minuten mit Sauerstoff und während 10 Minuten mit Argon. Nach Zugabe von 5.13 ml (70 mmol) Dimethylsulfid rührte man 16 Stunden bei Raumtemperatur. Das Reaktionsgemisch wurde im Vakuum eingedampft, der Rückstand mit 60 ml Dichlormethan versetzt und nach Zugabe von 15 ml Wasser und 15 ml Trifluoressigsäure 3 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt aus Essigester/Hexan kristallisiert. Man erhielt 8.29 g (85%) (RS)-2-(2-Oxoethyl)-6-chlor-1-indanon als weissen Feststoff mit Smp. 80°.

c) Eine Lösung von 2.5 g (12.0 mmol) (RS)-2-(2-Oxoethyl)-6-chlor-1-inda-non und 100 mg p-Toluolsulfonsäure in 120 ml wasserfreiem Toluol wurde am Wasserabscheider erhitzt. Zu der siedenden Lösung tropfte man über einen Zeitraum von 5 Minuten eine Lösung von 3.6 g (47.9 mmol) (R)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 2.42 g (81%) (R)-1-(7-Chlor-1,4-dihydro-indeno[1,2-b]pyrrol-1-yl)-propan-2-ol als braunes Öl, welches direkt in die nächste Reaktion eingesetzt wurde.

d) Zu einer auf 0° gekühlten Lösung von 2.42 g (9.8 mmol) (R)-1-(7-Chlor-1,4-dihydro-indeno[1,2-b]pyrrol-1-yl)-propan-2-ol und 5.48 ml (39.1 mmol) Triethylamin in 70 ml Dichlormethan gab man tropfenweise unter Rühren 1.53 ml (19.54 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organi-schen Phasen wurden mit 70 ml gesättigter Natriumchloridlösung gewa-schen, über Magnesiumsulfat getrocknet und im Vakuum eingedampft. Das erhaltene braune Öl wurde in 50 ml wasserfreiem Dimethylformamid gelöst, mit 1.27 g (19.54 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether und einmal mit 70 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.5 g (56%) (S)-1-(2-Azido-propyl)-7-chlor-1,4-dihydro-indeno[1,2-b]pyrrol als Öl.

e) 1.5 g (5.5 mmol) (S)-1-(2-Azido-propyl)-7-chlor-1,4-dihydro-indeno[1,2-b]pyrrol gelöst in 50 ml wasserfreiem Ethanol wurden an 150 mg Platinoxid 14 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 306 mg (2.64 mmol) Fumarsäure in 20 ml Methanol versetzt. Man rührte 3 Stunden bei Raumtemperatur und filtrierte anschliessend die weissen Kristalle ab. Man erhielt 1.12 g (67%) (S)-2-(7-Chlor-1,4-dihydro-indeno[1,2-b]pyrrol-1-yl)-1-methyl-ethylamin fumarat (1:0.5) mit Smp. 197°.

Beispiel 5

a) Eine Lösung von 2 g (9.2 mmol) (RS)-2-(2-Oxoethyl)-7-methoxy-1-tetra-lon und 100 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 2.78 g (37 mmol) (R)-1-Amino-2-propanol in 20 ml wasserfreiem Toluol. Anschliessend wurde weitere 30 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:1) gereinigt. Man erhielt 2.25 g (94%) (R)-1-(4,5-Dihydro-8-methoxy-1H-benz[g]indol-1-yl)-propan-2-ol als braunes Öl, welches direkt in die nächste Reaktion eingesetzt wurde.

b) Zu einer auf 0° gekühlten Lösung von 2.25 g (9 mmol) (R)-1-(4,5-Dihydro-8-methoxy-1H-benz[g]indol-1-yl)-propan-2-ol und 5 ml (36 mmol) Triethylamin in 60 ml Dichlormethan gab man tropfenweise unter Rühren 1.4 ml (18 mmol) Methansulfonylchlorid und rührte weitere 1.5 Stunden bei dieser Temperatur. Das Reaktionsgemisch wurde anschliessend mit 300 ml Diethylether verdünnt, zweimal mit jeweils 100 ml gesättigter Natriumhydrogencarbonatlösung gewaschen und die vereinigten Wasserphasen einmal mit 100 ml Diethylether extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 1.17 g (18 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.78 g (71%) (S)-1-(2-Azido-propyl)-4,5-dihydro-8-methoxy-1H-benz[g]indol als farbloses Öl.

c) 2.78 g (9.8 mmol) (S)-1-(2-Azido-propyl)-4,5-dihydro-8-methoxy-1H-benz[g]indol gelöst in 100 ml wasserfreiem Ethanol wurden an 280 mg Platinoxid 16 Stunden hydriert. Es wurde anschliessend vom Katalysator abfiltriert, mit Ethanol nachgewaschen und das Lösungsmittel im Vakuum abgezogen. 700 mg (2.7 mmol) des erhaltenen farblosen Öles wurden in 100 ml wasserfreiem Diethylether gelöst, filtriert und unter Rühren mit einer Lösung von 317 mg (2.7 mmol) Fumarsäure in 20 ml Methanol versetzt. Man rührte 2 Stunden bei Raumtemperatur und filtrierte anschliessend die weissen Kristalle ab. Man erhielt 820 mg (81%) (S)-2-(4,5-Dihydro-8-methoxy-1H-benz[g]indol-1-yl)-1-methyl-ethylamin fumarat (1:1) mit Smp. 193°.

Beispiel 6

a) Eine Lösung von 19.7 g (0.13 mol) 6-Fluor-1-indanon, 27.0 ml (0.31 mol) 3-Buten-2-ol und 200 mg p-Toluolsulfonsäure in 200 ml 2,2-Dimethoxy- propan wurde 67 Stunden an einem mit Molekularsieb (0.4 nm, 2mm Perl-form) gefüllten Wasserabscheider unter Rückfluss gekocht. Das Reaktions-gemisch wurde anschliessend im Vakuum eingeengt und durch Säulenchromatographie an Kieselgel gereinigt (Hexan/ Diethylether 4:1). Man erhielt 18.9 g (71%) (RS)-2-(2-Buten-1-yl)-6-fluor-1-indanon als gelbes Öl.

b) Durch eine auf -70° gekühlte Lösung von 18.9 g (92.5 mmol) (RS)-2-(2-Buten-1-yl)-6-fluor-1-indanon in 300 ml wasserfreiem Dichlormethan und 60 ml wasserfreiem Methanol leitete man unter Rühren während 100 Minuten einen Ozonstrom (3 g Ozon/Stunde). Anschliessend spülte man die Lösung während 5 Minuten mit Sauerstoff und während 10 Minuten mit Argon. Nach Zugabe von 10.2 ml (140 mmol) Dimethylsulfid rührte man 16 Stunden bei Raumtemperatur. Das Reaktionsgemisch wurde im Vakuum eingedampft, der Rückstand mit 300 ml Dichlormethan versetzt und nach Zugabe von 43 ml Wasser und 43 ml Trifluoressigsäure 3 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 200 ml Dichlormethan extrahiert. Die vereinigten organischen Phasen wurden über Magnesiumsulfat getrocknet, im Vakuum eingeengt und das erhaltene Rohprodukt aus Diethylether/Hexan kristallisiert. Man erhielt 16.5 g (92%) (RS)-2-(2-Oxo-ethyl)-6-fluor-1-indanon als weissen Feststoff mit Smp. 62°.

c) Eine Lösung von 1.92 g (10 mmol) (RS)-2-(2-Oxoethyl)-6-fluor-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.73 g (75%) (R)-1-(7-Fluor-1,4-dihydro-indeno[1,2-b]pyrrol-1-yl)-propan-2-ol als braunes Öl,

welches ohne weitere Reinigung in die nächste Reaktion eingesetzt wurde.

d) Zu einer auf 0° gekühlten Lösung von 1.73 g (7.5 mmol) (R)-1-(7-Fluor-1,4-dihydro-indeno[1,2-b]pyrrol-1-yl)-propan-2-ol und 4.2 ml (29.9 mmol) Triethylamin in 60 ml Dichlormethan gab man tropfenweise unter Rühren 1.17 ml (15.0 mmol) Methansulfonylchlorid und rührte weitere 1.5 Stunden bei dieser Temperatur. Das Reaktionsgemisch wurde anschliessend mit 150 ml Diethylether verdünnt, zweimal mit jeweils 60 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsalfat getrocknet und im Vakuum eingedampft. Das erhaltene braune Öl wurde in 50 ml wasserfreiem Dimethylformamid gelöst, mit 973 mg (15.0 mmol) Natriumazid versetzt und das Reaktionsgemisch unter Rühren 15 Stunden auf 50° erwärmt. Nach dem Abkühlen goss man die Lösung auf 110 ml Wasser und extrahierte zweimal mit jeweils 110 ml Diethylether und einmal mit 60 ml Essigester. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatograpie an Kieselgel (Toluol) gereinigt. Man erhielt 750 mg (39%) (S)-1-(2-Azido-propyl)-7-fluor-1,4-dihydro-indeno[1,2-b]pyrrol als farbloses Öl.

e) 750 mg (2.9 mmol) (S)-1-(2-Azido-propyl)-7-fluor-1,4-dihydro-indeno[1,2-b]pyrrol gelöst in 40 ml wasserfreiem Ethanol wurden an 75 mg Platinoxid 15 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 370 mg (1.46 mmol) Fumarsäure in 20 ml Methanol versetzt. Man rührte 3 Stunden bei Raumtemperatur und filtrierte anschliessend die weissen Kristalle ab. Man erhielt 460 mg (54%) (S)-2-(7-Fluor-1,4-dihydro-indeno[1,2-b]pyrrol-1-yl)-1-methyl-ethylamin fumarat (1:0.5) mit Smp. 194°.

Beispiel 7

a) Eine Lösung von 1.5 g (5.9 mmol) (S)-2-(4,5-Dihydro-8-methoxy-1H-benz[g]indol-1-yl)-1-methyl-ethylamin, 0.74 g (7.3 mmol)Triethylamin und 1.04 g (7.3 mmol) Trifluoressigsäureethylester in 100 ml wasserfreiem Methanol wurde 27 Stunden bei Raumtemperatur gerührt. Nachdem man das Lösungsmittel im Vakuum abgezogen hatte, nahm man den Rückstand mit 100 ml wasserfreiem Dioxan auf, gab 1.56 g (6.9 mmol) DDQ hinzu und kochte 1.5 Stunden unter Rückfluss. Anschliessend engte man das Reaktionsgemisch im Vakuum ein und reinigte den Rückstand durch Säulenchromatographie an Kieselgel (Dichlormethan/Aceton 4:1). Man erhielt 1.2 g (59%) (S)-N-[2-(8-Methoxy-1H-benz[g]indol-1-yl)-1-methyl-ethyl]-trifluoracetamid als hellbrauen Feststoff, der ohne weitere Umkristallisation in die nächste Reaktion eingesetzt wurde.

b) Eine Mischung aus 1.2 g (3.4 mmol) (S)-N-[2-(8-Methoxy-1H-benz[g]indol-1-yl)-1-methyl-ethyl]-trifluoracetamid, 1.2 g (21mmol) Kaliumhydroxid in 3 ml Wasser und 50 ml Methanol wurde 3 Stunden unter Rückfluss gekocht. Das Reaktionsgemisch wurde anschliessend in 100 ml 1N Natron-lauge gegossen, zweimal mit jeweils 100 ml Diethylether und einmal mit 100 ml Essigester extrahiert, die vereinigten organischen Phasen wurden einmal mit 150 ml gewaschen und über Magnesiumsulfat getrocknet. Nach dem Einengen im Vakuum, wurde der Rückstand in 100 ml wasserfreiem Diethylether gelöst, filtriert und unter Rühren mit einer Lösung von 398 mg (3.4 mmol) Fumarsäure in 30 ml Methanol versetzt. Man rührte 16 Stunden bei Raumtemperatur und filtrierte anschliessend die weissen Kristalle ab. Man erhielt 780 mg (73%) (S)-2-(8-Methoxy-1H-benz[g]indol-1-yl)-1-methyl-ethylamin fumarat (1:0.5) mit Smp. 208°.

Beispiel 8

a) Eine Lösung von 9.1 g (48.3 mmol) 6-Ethyl-3,3-dimethyl-1-indanon, 9.98 ml (0.12 mol) 3-Buten-2-ol und 250 mg p-Toluolsulfonsäure in 100 ml 2,2-Dimethoxy-propan wurde 88 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 6:1). Man erhielt neben 2.0 g Edukt 8.2 g (70%) (RS)-2-(2-Buten-1-yl)-6-ethyl-3,3-dimethyl-1-indanon als gelbes Öl.

b) Durch eine auf -70° gekühlte Lösung von 8.2 g (33.8 mmol) (RS)-2-(2-Buten-1-yl)-6-ethyl-3,3-diimethyl-1-indanon in 120 ml wasserfreiem Dichlormethan und 30 ml wasserfreiem Methanol leitete man unter Rühren während 40 Minuten einen Ozonstrom (3 g Ozon/Stunde). Anschliessend spülte man die Lösung während 5 Minuten mit Sauerstoff und während 10 Minuten mit Argon. Nach Zugabe von 3.72 ml (50.7 mmol) Dimethylsulfid rührte man 16 Stunden bei Raumtemperatur. Das Reaktionsgemisch wurde im Vakuum eingedampft, der Rückstand mit 100 ml Dichlormethan versetzt und nach Zugabe von 15 ml Wasser und 15 ml Trifluoressigsäure 2 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organi-

schen Phasen wurden über Magnesiumsulfat getrocknet und im Vakuum eingeengt. Man erhielt 7.55 g (97%) (RS)-2-(2-Oxoethyl)-6-ethyl-3,3-diimethyl-1-indanon als schwach gelbes Öl.

c) Eine Lösung von 2.3 g (10 mmol) (RS)-2-(2-Oxoethyl)-6-ethyl-3,3-dimethyl-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 40 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 3:2) gereinigt. Man erhielt 2.45 g (91%) (R)-1-(7-Ethyl-4,4-dimethyl-1,4-dihydro-indeno[1,2-b]pyrrol-1-yl)-propan-2-ol als rotes Öl.

d) Zu einer auf 0° gekühlten Lösung von 2.4 g (8.91 mmol) (R)-1-(7-Ethyl-4,4-dimethyl-1,4-dihydro-indeno[1,2-b]pyrrol-1-yl)-propan-2-ol und 4.97 ml (35.6 mmol) Triethylamin in 50 ml Dichlormethan gab man tropfenweise unter Rühren 1.39 ml (17.9 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 90 ml gesättigter Natriumchloridlösung gewaschen, über Magnesiumsulfat getrocknet und im Vakuum eingedampft. Das erhaltene grüne Öl wurde in 50 ml wasserfreiem Dimethylformamid gelöst, mit 1.16 g (17.8 mmol) Natriumazid versetzt und das Reaktionsgemisch unter Rühren 17 Stunden auf 60° erwärmt. Nach dem Abkühlen goss man die Lösung auf 70 ml Wasser und extrahierte dreimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Diethylether 4:1) gereinigt. Man erhielt 1.44 g (55%) (S)-1-(2-Azido-propyl)-7-ethyl-4,4-dimethyl-1,4-dihydro-indeno[1,2-b]pyrrol als rotes Öl.

e) 1.44 g (4.89 mmol) (S)-1-(2-Azido-propyl)-7-ethyl-4,4-dimethyl-1,4-dihydro-indeno-[1,2-b]-pyrrol gelöst in 50 ml wasserfreiem Ethanol wurden an 140 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene farblose Öl wurde in 120 ml wasserfreiem Diethylether gelöst, filtriert und unter Rühren mit einer Lösung von 568 mg (4.89 mmol) Fumarsäure in 15 ml Methanol versetzt. Man rührte 15 Stunden bei Raumtemperatur und filtrierte anschliessend die weissen Kristalle ab. Man erhielt 1.04 g (55%) (S)-2-(7-Ethyl-4,4-dimethyl-1,4-dihydro-indeno[1,2-b]pyrrol-1-yl)-1-methyl-ethylamin fumarat (1:1) mit Smp. 178°.

Beispiel 9

a) Eine Lösung von 10.0 g (56.1 mmol) 6-Fluor-3,3-dimethyl-1-indanon, 11.1 ml (0.13 mol) 3-Buten-2-ol und 200 mg p-Toluolsulfonsäure in 200 ml 2,2-Dimethoxy-propan wurde 96 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 8:1). Man erhielt neben 11.9 g Edukt 3.38 g (26%) (RS)-2-(2-Buten-1-yl)-6-fluor-3,3-dimethyl-1-indanon als gelbes Öl.

b) Durch eine auf -70° gekühlte Lösung von 3.38 g (14.6 mmol) (RS)-2-(2-Buten-1-yl)-6-fluor-3,3-diimethyl-1-indanon in 75 ml wasserfreiem Dichlormethan und 15 ml wasserfreiem Methanol leitete man unter Rühren während 30 Minuten einen Ozonstrom (1.5 g Ozon/Stunde). Anschliessend spülte man die Lösung während 5 Minuten mit Sauerstoff und während 10 Minuten mit Argon. Nach Zugabe von 1.6 ml (21.8 mmol) Dimethylsulfid rührte man 17 Stunden bei Raumtemperatur. Das Reaktionsgemisch wurde im Vakuum eingedampft, der Rückstand mit 40 ml Dichlormethan versetzt und nach Zugabe von 5 ml Wasser und 5 ml Trifluoressigsäure 2 Stunden bei Raumtemperatur gerührt. Man goss das Gemisch anschliessend auf 90 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 3.04 g (95%) (RS)-2-(2-Oxoethyl)-6-fluor-3,3-diimethyl-1-indanon als schwach gelbes Öl.

c) Eine Lösung von 3.04 g (13.8 mmol) (RS)-2-(2-Oxoethyl)-6-fluor-3,3-dimethyl-1-indanon und 110 mg p-Toluolsulfonsäure in 100 ml wasserfreiem Toluol wurde am Wasserabscheider erhitzt. Zu der siedenden Lösung tropfte man über einen Zeitraum von 5 Minuten eine Lösung von 4.14 g (55.2 mmol) (R)-1-Amino-2-propanol in 40 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 2.0 g (56%) (R)-1-(7-Fluor-4,4-dimethyl-1,4-dihydro-indeno[1,2-b]pyrrol-1-yl)-propan-2-ol als braunes Öl.

d) Zu einer auf 0° gekühlten Lösung von 2.0 g (8.15 mmol) (R)-1-(7-Fluor-4,4-dimethyl-1,4-dihydro-indeno[1,2-b]pyrrol-1-yl)-propan-2-ol und 4.52 ml (32.6 mmol) Triethylamin in 60 ml Dichlormethan gab man tropfenweise

unter Rühren 1.27 ml (16.3 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 60 ml gesättigter Natriumhydrogencarbonatlösung gewaschen und die vereinigten Wasserphasen einmal mit 60 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 1.07 g (16.3 mmol) Natriumazid versetzt und das Reaktionsgemisch unter Rühren 16 Stunden auf 60° erwärmt. Nach dem Abkühlen goss man die Lösung auf 80 ml Wasser und extrahierte dreimal mit jeweils 70 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.22 g (54%) (S)-1-(2-Azido-propyl)-7-fluor-4,4-dimethyl-1,4-dihydro-indeno[1,2-b]pyrrol als gelbes Öl.

e) 1.22 g (4.29 mmol) (S)-1-(2-Azido-propyl)-7-fluor-4,4-dimethyl-1,4-dihydro-indeno-[1,2-b]-pyrrol gelöst in 100 ml wasserfreiem Ethanol wurden an 120 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 150 ml wasserfreiem Diethylether gelöst, filtriert und unter Rühren mit einer Lösung von 498 mg (4.29 mmol) Fumarsäure in 20 ml Methanol versetzt. Man rührte 15 Stunden bei Raumtemperatur und filtrierte anschliessend die weissen Kristalle ab. Man erhielt 1.12 g (70%) (S)-2-(7-Fluor-4,4-dimethyl-1,4-dihydro-indeno[1,2-b]pyrrol-1-yl)-1-methyl-ethylamin fumarat (1:1) mit Smp. 211°.

Beispiel 10

a) Eine Lösung von 14.2 g (97,3 mmol) 6-Methyl-1-indanon, 20.1 ml (0.23 mol) 3-Buten-2-ol und 140 mg p-Toluolsulfonsäure in 140 ml 2,2-Dimethoxy-propan wurde 69 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt 16.3 g (83%) (RS)-2-(2-Buten-1-yl)-6-methyl-1-indanon als gelbes Öl.

b) Durch eine auf -70° gekühlte Lösung von 16.3 g (81.4 mmol) (RS)-2-(2-Buten-1-yl)-6-methyl-1-indanon in 300 ml wasserfreiem Dichlormethan und 60 ml wasserfreiem Methanol leitete man unter Rühren während 80 Minuten einen Ozonstrom (3 g Ozon/Stunde). Anschliessend spülte man die Lösung während 5 Minuten mit Sauerstoff und während 10 Minuten mit Argon. Nach Zugabe von 8.95 ml (122 mmol) Dimethylsulfid rührte man 15 Stunden bei Raumtemperatur. Das Reaktionsgemisch wurde im Vakuum eingedampft, der Rückstand mit 300 ml Dichlormethan versetzt und nach Zugabe von 40 ml Wasser und 40 ml Trifluoressigsäure 3 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 200 ml Dichlormethan extrahiert. Die vereinigten organischen Phasen wurden über Magnesiumsulfat getrocknet und im Vakuum eingeengt. Das erhaltene Rohprodukt wurde aus Diethylether/Hexan umkristallisiert. Man erhielt 12.7 g (82%) (RS)-2-(2-Oxoethyl)-6-methyl-1-indanon als schwach gelben Feststoff mit Smp. 53-54°.

c) Eine Lösung von 2.82 g (15 mmol) (RS)-2-(2-Oxoethyl)-6-methyl-1-indanon und 100 mg p-Toluolsulfonsäure in 100 ml wasserfreiem Toluol wurde am Wasserabscheider erhitzt. Zu der siedenden Lösung tropfte man über einen Zeitraum von 5 Minuten eine Lösung von 4.51 g (60 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 2.7 g (79%) (R)-1-(7-Methyl-1,4-dihydro-indeno[1,2-b]pyrrol-1-yl)-propan-2-ol als braungrünes Ol.

d) Zu einer auf 0° gekühlten Lösung von 2.7 g (11.9 mmol) (R)-1-(7-Methyl-1,4-dihydro-indeno[1,2-b]pyrrol-1-yl)-propan-2-ol und 6.7 ml (47.6 mmol) Triethylamin in 75 ml Dichlormethan gab man tropfenweise unter Rühren 1.8 ml (23.8 mmol) Methansulfonylchlorid und rührte weitere 1.5 Stunden bei dieser Temperatur. Das Reaktionsgemisch wurde anschliessend mit 200 ml Dichlormethan verdünnt, zweimal mit jeweils 90 ml gesättigter Natriumhydrogencarbonatlösung gewaschen und die vereinigten Wasserphasen einmal mit 90 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 70 ml wasserfreiem Dimethylformamid gelöst, mit 1.55 g (23.8 mmol) Natriumazid versetzt und das Reaktionsgemisch unter Rühren 15 Stunden auf 60° erwärmt. Nach dem Abkühlen goss man die Lösung auf 150 ml Wasser und extrahierte zweimal mit jeweils 150 ml Essigester. Die vereinigten organischen Phasen wusch man je einmal mit 120 ml Wasser und mit 120 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.5 g (50%) (S)-1-(2-Azido-propyl)-7-methyl-1,4-dihydro-indeno[1,2-b]pyrrol als schwach gelbes Öl.

e) 1.5 g (5.94 mmol) (S)-1-(2-Azido-propyl)-7-methyl-1,4-dihydro-indeno-[1,2-b]pyrrol gelöst in 50 ml wasserfreiem Ethanol wurden an 150 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 150 ml wasserfreiem Diethylether gelöst, filtriert und unter Rühren mit einer Lösung von 345 mg (2.97 mmol) Fumarsäure in 25 ml Methanol versetzt. Man rührte 17 Stunden bei Raumtemperatur und filtrierte anschliessend die weissen Kristalle ab. Man erhielt 1.1 g (65%) (S)-2-(7-Methyl-1,4-dihydro-indeno[1,2-b]pyrrol-1-yl)-1-methyl-ethylamin fumarat (1:0.5) mit Smp. 194°.

Beispiel 11

a) Eine Lösung von 10.0 g (47.4 mmol) 6-Brom-1-indanon, 9.79 ml (0.11 mol) 3-Buten-2-ol und 100 mg p-Toluolsulfonsäure in 100 ml 2,2-Dimethoxy-propan wurde 71 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 1:1). Man erhielt neben 3.07 g Edukt 9.86 g (78%) (RS)-2-(2-Buten-1-yl)-6-brom-1-indanon als gelbes Öl.

b) Durch eine auf -70° gekühlte Lösung von 9.86 g (37.2 mmol) (RS)-2-(2-Buten-1-yl)-6-brom-1-indanon in 150 ml wasserfreiem Dichlormethan und 30 ml wasserfreiem Methanol leitete man unter Rühren während 60 Minuten einen Ozonstrom (2 g Ozon/Stunde). Anschliessend spülte man die Lösung während 5 Minuten mit Sauerstoff und während 10 Minuten mit Argon. Nach Zugabe von 4.09 ml (55.8 mmol) Dimethylsulfid rührte man 15 Stunden bei Raumtemperatur. Das Reaktionsgemisch wurde im Vakuum eingedampft, der Rückstand mit 150 ml Dichlormethan versetzt und nach Zugabe von 20 ml Wasser und 20 ml Trifluoressigsäure 2 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Das erhaltene Rohprodukt wurde aus Essigester/Hexan umkristallisiert. Man erhielt 7.5 g (80%) (RS)-2-(2-Oxoethyl)-6-brom-1-indanon als schwach gelben Feststoff mit Smp. 84°.

c) Eine Lösung von 2.0 g (7.9 mmol) (RS)-2-(2-Oxoethyl)-6-brom-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 2.37 g (31.6 mmol) (R)-1-Amino-2-propanol in 10 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 4:1) gereinigt. Man erhielt 1.19 g (52%) (R)-1-(7-Brom-1,4-dihydro-indeno[1,2-b]pyrrol-1-yl)-propan-2-ol als braunes Öl.

d) Zu einer auf 0° gekühlten Lösung von 1.19 g (4.07 mmol) (R)-1-(7-Brom-1,4-dihydro-indeno[1,2-b]pyrrol-1-yl)-propan-2-ol und 2.27 ml (16.3 mmol) Triethylamin in 40 ml Dichlormethan gab man tropfenweise unter Rühren 0.63 ml (8.14 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 40 ml wasserfreiem Dimethylformamid gelöst, mit 0.53 g (8.14 mmol) Natriumazid versetzt und das Reaktionsgemisch unter Rühren 15 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.95 g (74%) (S)-1-(2-Azido-propyl)-7-brom-1,4-dihydro-indeno[1,2-b]pyrrol als farbloses Öl.

e) 0.95 g (2.99 mmol) (S)-1-(2-Azido-propyl)-7-brom-1,4-dihydro-indeno-[1,2-b]pyrrol gelöst in 40 ml wasserfreiem Ethanol wurden an 95 mg Platinoxid 4 Stunden hydriert. Es wurde anschliessend vom Katalysator abfiltriert, mit Ethanol nachgewaschen und das Lösungsmittel im Vakuum abgezogen. Das Produktgemisch wurde an Kieselgel (Methanol/Dichlormethan 5:95) getrennt. Das erhaltene farblose Öl (507 mg) wurde in 50 ml wasserfreiem Diethylether gelöst, filtriert und unter Rühren mit einer Lösung von 101 mg (0.87 mmol) Fumarsäure in 10 ml Methanol versetzt. Man rührte 17 Stunden bei Raumtemperatur und filtrierte anschliessend die weissen Kristalle ab. Man erhielt 503 mg (50%) (S)-2-(7-Brom-1,4-dihydro-indeno[1,2-b]pyrrol-1-yl)-1-methyl-ethylamin fumarat (1:0.5) mit Smp. 197°.

Beispiel 12

a) Eine Lösung von 22.0 g (101 mmol) 6-Methoxy-3,3-diethyl-1-indanon, 20.8 ml (0.24 mol) 3-Buten-2-ol und 220 mg p-Toluolsulfonsäure in 220 ml 2,2-Dimethoxy-propan wurde 87 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 6:1). Man erhielt neben 15.7 g Edukt 4.1 g (15%) (RS)-2-(2-Buten-1-yl)-6-methoxy-3,3-diethyl-1-indanon als gelbes Öl.
b) Durch eine auf -70° gekühlte Lösung von 4.1 g (15.1 mmol) (RS)-2-(2-Buten-1-yl)-6-methoxy-3,3-diethyl-1-indanon in 60 ml wasserfreiem Dichlormethan und 15 ml wasserfreiem Methanol leitete man unter Rühren während 25 Minuten einen Ozonstrom (2 g Ozon/Stunde). Anschliessend spülte man die Lösung während 5 Minuten mit Sauerstoff und während 10 Minuten mit Argon. Nach Zugabe von 1.66 ml (22.6 mmol) Dimethylsulfid rührte man 22 Stunden bei Raumtemperatur. Das Reaktionsgemisch wurde im Vakuum eingedampft, der Rückstand mit 50 ml Dichlormethan versetzt und nach Zugabe von 8 ml Wasser und 8 ml Trifluoressigsäure 2.5 Stunden bei Raumtemperatur gerührt. Man goss das Gemisch anschliessend auf 70 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 3.9 g (99%) (RS)-2-(2-Oxoethyl)-6-methoxy-3,3-diethyl-1-indanon als schwach gelbes Öl.
c) Eine Lösung von 3.9 g (15 mmol) (RS)-2-(2-Oxoethyl)-6-methoxy-3,3-diethyl-1-indanon und 120 mg p-Toluolsulfonsäure in 100 ml wasserfreiem Toluol wurde am Wasserabscheider erhitzt. Zu der siedenden Lösung tropfte man über einen Zeitraum von 5 Minuten eine Lösung von 4.5 g (60 mmol) (R)-1-Amino-2-propanol in 30 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 1:1) gereinigt. Man erhielt 2.73 g (61%) (R)-1-(7-Methoxy-4,4-diethyl-1,4-dihydro-indeno[1,2-b]pyrrol-1-yl)-propan-2-ol als rotbraunes Öl.
d) Zu einer auf 0° gekühlten Lösung von 2.73 g (9.12 mmol) (R)-1-(7-Methoxy-4,4-diethyl-1,4-dihydro-indeno[1,2-b]pyrrol-1-yl)-propan-2-ol und 5.08 ml (36.5 mmol) Triethylamin in 70 ml Dichlormethan gab man tropfenweise unter Rühren 1.42 ml (18.2 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 1.18 g (18.2 mmol) Natriumazid versetzt und das Reaktionsgemisch unter Rühren 15 Stunden auf 60° erwärmt. Nach dem Abkühlen goss man die Lösung auf 70 ml Wasser und extrahierte dreimal mit jeweils 80 ml Essigester. Die vereinigten organischen Phasen wusch man je einmal mit 70 mi Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 2.33 g (79%) (S)-1-(2-Azido-propyl)-7-methoxy-4,4-diethyl-1,4-dihydro-indeno[1,2-b]pyrrol als gelbes Öl.
e) 2.25 g (6.94 mmol) (S)-1-(2-Azido-propyl)-7-methoxy-4,4-diethyl-1,4-dihydro-indeno-[1,2-b]-pyrrol gelöst in 100 ml wasserfreiem Ethanol wurden an 225 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 406 mg (3.5 mmol) Fumarsäure in 10 ml Methanol versetzt. Man rührte 17 Stunden bei Raumtemperatur und filtrierte anschliessend die weissen Kristalle ab. Man erhielt 1.93 g (78%) (S)-2-(7-Methoxy-4,4-diethyl-1,4-dihydro-indeno[1,2-b]pyrrol-1-yl)-1-methyl-ethylamin fumarat (1:0.5) mit Smp. 189°.

Beispiel 13

a) Eine Lösung von 20.0 g (133 mmol) 5-Fluor-1-indanon, 27.5 ml (0.32 mol) 3-Buten-2-ol und 200 mg p-Toluolsulfonsäure in 200 ml 2,2-Dimethoxy-propan wurde 63 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt 18.6 g (68%) (RS)-2-(2-Buten-1-yl)-5-fluor-1-indanon als gelbes Öl.
b) Durch eine auf -70° gekühlte Lösung von 18.5 g (90.6 mmol) (RS)-2-(2-Buten-1-yl)-5-fluor-1-indanon in 300 ml wasserfreiem Dichlormethan und 50 ml wasserfreiem Methanol leitete man unter Rühren während 85 Minuten einen Ozonstrom (3.5 g Ozon/Stunde). Anschliessend spülte man die Lösung während 5 Minuten mit Sauerstoff und während 10 Minuten mit Argon. Nach Zugabe von 9.9 ml (135 mmol) Dimethylsulfid rührte man 17 Stunden

bei Raumtemperatur. Das Reaktionsgemisch wurde im Vakuum eingedampft, der Rückstand mit 220 ml Dichlormethan versetzt und nach Zugabe von 40 ml Wasser und 40 ml Trifluoressigsäure 4.5 Stunden bei Raumtemperatur gerührt. Man goss das Gemisch anschliessend auf 170 ml Wasser und neutralisierte unter Rühren durch spatelweise Zugabe von Natriumhydrogencarbonat. Es wurden weitere 110 ml Wasser zugegeben, die Phasen getrennt und die Wasserphase zweimal mit jeweils 170 ml Dichlormethan extrahiert. Die vereinigten organischen Phasen wurden über Magnesiumsulfat getrocknet und im Vakuum eingeengt. Man erhielt ein gelbes Öl, welches aus Diethylether/Hexan kristallisiert wurde. Man erhielt 13.6 g (78%) (RS)-2-(2-Oxoethyl)-5-fluor-1-indanon als schwach gelben Feststoff mit Smp. 56°.

c) Eine Lösung von 2.88 g (15 mmol) (RS)-2-(2-Oxoethyl)-5-fluor-1-indanon und 100 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 4.51 g (60 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 2.75 g (79%) (R)-1-(6-Fluor-1,4-dihydro-indeno[1,2-b]pyrrol-1-yl)-propan-2-ol als braunes Öl.

d) Zu einer auf 0° gekühlten Lösung von 2.75 g (11.9 mmol) (R)-1-(6-Fluor-1,4-dihydro-indeno[1,2-b]pyrrol-1-yl)-propan-2-ol und 6.7 ml (47.6 mmol) Triethylamin in 70 ml Dichlormethan gab man tropfenweise unter Rühren 1.8 ml (23.8 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 90 ml gesättigter Natriumhydrogencarbonatlösung gewaschen und die vereinigten Wasserphasen einmal mit 90 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 60 ml wasserfreiem Dimethylformamid gelöst, mit 1.55 g (12.5 mmol) Natriumazid versetzt und das Reaktionsgemisch unter Rühren 17 Stunden auf 60° erwärmt. Nach dem Abkühlen goss man die Lösung auf 80 ml Wasser und extrahierte dreimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.23 g (40%) (S)-1-(2-Azido-propyl)-6-fluor-1,4-dihydro-indeno[1,2-b]pyrrol als schwach gelbes Öl.

e) 1.23 g (4.80 mmol) (S)-1-(2-Azido-propyl)-6-fluor-1,4-dihydro-indeno-[1,2-b]pyrrol gelöst in 60 ml wasserfreiem Ethanol wurden an 125 mg Platinoxid 6 Stunden hydriert. Es wurde anschliessend vom Katalysator abfiltriert, mit Ethanol nachgewaschen und das Lösungsmittel im Vakuum abgezogen. Das erhaltene farblose Öl wurde in 130 ml wasserfreiem Diethylether gelöst, filtriert und unter Rühren mit einer Lösung von 279 mg (2.40 mmol) Fumarsäure in 25 ml Methanol versetzt. Man rührte 15 Stunden bei Raumtemperatur und filtrierte anschliessend die weissen Kristalle ab. Man erhielt 1.0 g (72%) (S)-2-(6-Fluor-1,4-dihydro-indeno[1,2-b]pyrrol-1-yl)-1-methyl-ethylamin fumarat (1:0.5) mit Smp. 200°.

<u>Beispiel 14</u>

a) Eine Lösung von 13.2 g (63.4 mmol) 6-Phenyl-1-indanon, 11 ml (0.13 mol) 3-Buten-2-ol und 110 mg p-Toluolsulfonsäure in 110 ml 2,2-Dimethoxy-propan wurde 48 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt 12.0 g (72%) (RS)-2-(2-Buten-1-yl)-6-phenyl-1-indanon als schwach gelben Feststoff, der ohne weitere Umkristallisation in die nächste Reaktion eingesetzt wurde.

b) Durch eine auf -70° gekühlte Lösung von 12.0 g (45.8 mmol) (RS)-2-(2-Buten-1-yl)-6-phenyl-1-indanon in 180 ml wasserfreiem Dichlormethan und 40 ml wasserfreiem Methanol leitete man unter Rühren während 75 Minuten einen Ozonstrom (3 g Ozon/Stunde). Anschliessend spülte man die Lösung während 5 Minuten mit Sauerstoff und während 10 Minuten mit Argon. Nach Zugabe von 5.04 ml (68.7 mmol) Dimethylsulfid rührte man 15 Stunden bei Raumtemperatur. Das Reaktionsgemisch wurde im Vakuum eingedampft, der Rückstand mit 120 ml Dichlormethan versetzt und nach Zugabe von 30 ml Wasser und 30 ml Trifluoressigsäure 2 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 200 ml Dichlormethan extrahiert. Die vereinigten organischen Phasen wurden über Magnesiumsulfat getrocknet und im Vakuum eingeengt. Man erhielt 11.2 g (97%) (RS)-2-(2-Oxoethyl)-6-phenyl-1-indanon als schwach gelbes Öl.

c) Eine Lösung von 2.5 g (10 mmol) (RS)-2-(2-Oxoethyl)-6-phenyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.5 g (52%) (R)-1-(7-Phenyl-1,4-dihydro-indeno[1,2-b]pyrrol-1-yl)-propan-2-ol als braunes Öl.

d) Zu einer auf 0° gekühlten Lösung von 1.5 g (5.2 mmol) (R)-1-(7-Phenyl-1,4-dihydro-indeno[1,2-b]pyrrol-1-yl)-propan-2-ol und 3 ml (20.8 mmol) Triethylamin in 50 ml Dichlormethan gab man tropfenweise unter Rühren 0.82 ml (10.4 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Der erhaltene grüne Feststoff wurde in 50 ml wasserfreiem Dimethylformamid gelöst, mit 0.53 g (8.2 mmol) Natriumazid versetzt und das Reaktionsgemisch unter Rühren 15 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 80 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.64 g (39%) (S)-1-(2-Azido-propyl)-7-phenyl-1,4-dihydro-indeno[1,2-b]pyrrol als braunes Öl.

e) 0.64 g (2.04 mmol) (S)-1-(2-Azido-propyl)-7-phenyl-1,4-dihydro-indeno-[1,2-b]pyrrol gelöst in 30 ml wasserfreiem Ethanol wurden an 65 mg Platinoxid 2 Stunden hydriert. Es wurde anschliessend vom Katalysator abfiltriert, mit Ethanol nachgewaschen und das Lösungsmittel im Vakuum abgezogen. Der erhaltene grünliche Feststoff wurde in 80 ml wasserfreiem Diethylether gelöst, filtriert und unter Rühren mit einer Lösung von 118 mg (1.02 mmol) Fumarsäure in 20 ml Methanol versetzt. Man rührte 15 Stunden bei Raumtemperatur und filtrierte anschliessend die beigen Kristalle ab. Man erhielt 0.37 g (53%) (S)-2-(7-Phenyl-1,4-dihydro-indeno[1,2-b]pyrrol-1-yl)-1-methyl-ethylamin fumarat (1:0.5) mit Smp. 202-204°.

Beispiel 15

a) Eine Lösung von 16 g (108 mmol) 6-Hydroxy-1-indanon, 22.3 ml (0.26 mol) 3-Buten-2-ol und 160 mg p-Toluolsulfonsäure in 170 ml 2,2-Dimethoxy-propan wurde 38 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 4:1). Man erhielt neben 7.5 g Edukt 6.21 g (28%) (RS)-2-(2-Buten-1-yl)-6-hydroxy-1-indanon als gelbes Öl.

b) Eine Lösung von 5.0 g (24.7 mmol) (RS)-2-(2-Buten-1-yl)-6-hydroxy-1-indanon, 4.1 ml (54.4 mmmol) Ethylbromid, 6.83 g (49.4 mmol) Kaliumcarbonat und 10 ml N,N-Dimethylformamid in 70 ml Aceton wurde 35 Stunden auf 35° erhitzt. Nach dem Abkühlen goss man die Lösung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Man erhielt 5.6 g (98%) (RS)-2-(2-Buten-1-yl)-6-ethoxy-1-indanon als rotbraunes Öl, welches direkt in die nächste Reaktion eingesetzt wurde.

c) Durch eine auf -70° gekühlte Lösung von 5.6 g (24.3 mmol) (RS)-2-(2-Buten-1-yl)-6-ethoxy-1-indanon in 150 ml wasserfreiem Dichlormethan und 30 ml wasserfreiem Methanol leitete man unter Rühren während 90 Minuten einen Ozonstrom (3 g Ozon/Stunde). Anschliessend spülte man die Lösung während 5 Minuten mit Sauerstoff und während 10 Minuten mit Argon. Nach Zugabe von 2.72 ml (37.1 mmol) Dimethylsulfid rührte man 15 Stunden bei Raumtemperatur. Das Reaktionsgemisch wurde im Vakuum eingedampft, der Rückstand mit 170 ml Dichlormethan versetzt und nach Zugabe von 25 ml Wasser und 25 ml Trifluoressigsäure 3 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 3.4 g (64%) (RS)-2-(2-Oxoethyl)-6-ethoxy-1-indanon als rotbraunes Öl.

d) Eine Lösung von 1.6 g (7.33 mmol) (RS)-2-(2-Oxoethyl)-6-ethoxy-1-indanon und 70 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.2 g (29.3 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 4:1) gereinigt. Man erhielt 1.03 g (52%) (RS)-1-(7-Ethoxy-1,4-dihydro-indeno[1,2-b]pyrrol-1-yl)-propan-2-ol als braunes Öl.

e) Zu einer auf 0° gekühlten Lösung von 1.03 g (3.8 mmol) (RS)-1-(7-Ethoxy-1,4-dihydro-indeno[1,2-b]pyrrol-1-yl)-propan-2-ol und 2.1 ml (15.2 mmol) Triethylamin in 50 ml Dichlormethan gab man tropfenweise unter Rühren 0.59

ml (7.6 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 30 ml wasserfreiem Dimethylformamid gelöst, mit 0.6 g (9.2 mmol) Natriumazid versetzt und das Reaktionsgemisch unter Rühren 20 Stunden auf 60° erwärmt. Nach dem Abkühlen goss man die Losung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Losung im Vakuum ein. Das erhaltene braune Öl wurde durch Säulenchromatographie an Kieselgel (Essigester/Hexan 1:10) gereinigt. Man erhielt 948 mg (72%) (RS)-1-(2-Azido-propyl)-7-ethoxy-1,4-dihydro-indeno[1,2-b]pyrrol als schwach gelbliches Öl.

f) 0.94 g (3.32 mmol) (RS)-1-(2-Azido-propyl)-7-ethoxy-1,4-dihydro-indeno-[1,2-b]pyrrol gelöst in 80 ml wasserfreiem Ethanol wurden an 94 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 198 mg (1.7 mmol) Fumarsäure in 20 ml Methanol versetzt. Man rührte 17 Stunden bei Raumtemperatur und filtrierte anschliessend die weissen Kristalle ab. Man erhielt 581 mg (56%) (RS)-2-(7-Ethoxy-1,4-dihydro-indeno[1,2-b]pyrrol-1-yl)-1-methylethylamin fumarat (1:0.5) mit Smp. 212-214°.

Beispiel 16

a) Eine Lösung von 4.74 g (21.6 mmol) (RS)-2-(2-Buten-1-yl)-6-hydroxy-1-indanon, 5.18 ml (47.6 mmmol) Isobutylbromid und 5.98 g (43.3 mmol) Kaliumcarbonat in 40 ml N,N-Dimethylformamid wurde 48 Stunden auf 60° erhitzt. Nach dem Abkühlen goss man die Lösung auf 100 ml Wasser und extrahierte zweimal mit jeweils 70 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Man erhielt 5.2 g (93%) (RS)-2-(2-Buten-1-yl)-6-Isobutoxy-1-indanon als braunes Öl, welches direkt in die nächste Reaktion eingesetzt wurde.

b) Durch eine auf -70° gekühlte Lösung von 5.1 g (19.7 mmol) (RS)-2-(2-Buten-1-yl)-6-isobutoxy-1-indanon in 150 ml wasserfreiem Dichlormethan und 30 ml wasserfreiem Methanol leitete man unter Rühren während 90 Minuten einen Ozonstrom (2 g Ozon/Stunde). Anschliessend spülte man die Lösung während 5 Minuten mit Sauerstoff und während 10 Minuten mit Argon. Nach Zugabe von 2.16 ml (29.4 mmol) Dimethylsulfid rührte man 15 Stunden bei Raumtemperatur. Das Reaktionsgemisch wurde im Vakuum eingedampft, der Rückstand mit 170 ml Dichlormethan versetzt und nach Zugabe von 25 ml Wasser und 25 ml Trifluoressigsäure 3 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 2.3 g (48%) (RS)-2-(2-Oxoethyl)-6-isobutoxy-1-indanon als rotbraunes Öl.

c) Eine Lösung von 2.3 g (9.34 mmol) (RS)-2-(2-Oxoethyl)-6-isobutoxy-1-indanon und 70 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.93 g (37.3 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 1:4) gereinigt. Man erhielt 1.52 g (57%) (RS)-1-(7-Isobutoxy-1,4-dihydro-indeno[1,2-b]pyrrol-1-yl)-propan-2-ol als braunes Öl.

d) Zu einer auf 0° gekühlten Lösung von 1.5 g (5.26 mmol) (RS)-1-(7-Isobutoxy-1,4-dihydro-indeno[1,2-b]pyrrol-1-yl)-propan-2-ol und 2.93 ml (21.0 mmol) Triethylamin in 50 ml Dichlormethan gab man tropfenweise unter Rühren 0.81 ml (10.5 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 30 ml wasserfreiem Dimethylformamid gelöst, mit 0.68 g (10.5 mmol) Natriumazid versetzt und das Reaktionsgemisch unter Rühren 15 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum

ein. Das erhaltene braune Öl wurde durch Säulenchromatographie an Kieselgel (Essigester/Hexan 1:4) gereinigt. Man erhielt 0.43 g (26%) (RS)-1-(2-Azido-propyl)-7-isobutoxy-1,4-dihydro-indeno[1,2-b]pyrrol als schwach gelbliches Öl.

e) 0.43 g (1.38 mmol) (RS)-1-(2-Azido-propyl)-7-isobutoxy-1,4-dihydro-indeno-[1,2-b]-pyrrol gelöst in 50 ml wasserfreiem Ethanol wurden an 50 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 80 mg (0.69 mmol) Fumarsäure in 10 ml Methanol versetzt. Man rührte 17 Stunden bei Raumtemperatur und filtrierte anschliessend die weissen Kristalle ab. Man erhielt 0.25 mg (53%) (RS)-2-(7-Isobutoxy-1,4-dihydro-indeno[1,2-b]pyrrol-1-yl)-1-methyl-ethylamin fumarat (1:0.5) mit Smp. 178°.

Beispiel 17

a) Eine Lösung von 11.8 g (73.7 mmol) 6-Ethyl-1-indanon, 15.4 ml (0.18 mol) 3-Buten-2-ol und 110 mg p-Toluolsulfonsäure in 110 ml 2,2-Dimethoxy-propan wurde 46 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt 7.92 g (50%) (RS)-2-(2-Buten-1-yl)-6-ethyl-1-indanon als braunes Öl.
b) Durch eine auf -70° gekühlte Lösung von 7.92 g (37.0 mmol) (RS)-2-(2-Buten-1-yl)-6-ethyl-1-indanon in 150 ml wasserfreiem Dichlormethan und 30 ml wasserfreiem Methanol leitete man unter Rühren während 40 Minuten einen Ozonstrom (3 g Ozon/Stunde). Anschliessend spülte man die Lösung während 5 Minuten mit Sauerstoff und während 10 Minuten mit Argon. Nach Zugabe von 3.36 ml (45.8 mmol) Dimethylsulfid rührte man 15 Stunden bei Raumtemperatur. Das Reaktionsgemisch wurde im Vakuum eingedampft, der Rückstand mit 150 ml Dichlormethan versetzt und nach Zugabe von 30 ml Wasser und 30 ml Trifluoressigsäure 3 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 6.94 g (93%) (RS)-2-(2-Oxoethyl)-6-ethyl-1-indanon als gelbes Öl.
c) Eine Lösung von 2.02 g (10 mmol) (RS)-2-(2-Oxoethyl)-6-ethyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.7 g (71%) (R)-1-(7-Ethyl-1,4-dihydro-indeno[1,2-b]pyrrol-1-yl)-propan-2-ol als braunes Öl.
d) Zu einer auf 0° gekühlten Lösung von 1.7 g (7.04 mmol) (R)-1-(7-Ethyl-1,4-dihydro-indeno[1,2-b]pyrrol-1-yl)-propan-2-ol und 3.9 ml (28.2 mmol) Triethylamin in 55 ml Dichlormethan gab man tropfenweise unter Rühren 1.1 ml (14.1 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 50 ml wasserfreiem Dimethylformamid gelöst, mit 0.84 g (12.8 mmol) Natriumazid versetzt und das Reaktionsgemisch unter Rühren 16 Stunden auf 60° erwärmt. Nach dem Abkühlen goss man die Lösung auf 120 ml Wasser und extrahierte zweimal mit jeweils 120 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.74 g (40%) (S)-1-(2-Azido-propyl)-7-ethyl-1,4-dihydro-indeno[1,2-b]pyrrol als gelbes Öl.
e) 0.74 g (2.7 mmol) (S)-1-(2-Azido-propyl)-7-ethyl-1,4-dihydro-indeno-[1,2-b]pyrrol gelöst in 30 ml wasserfreiem Ethanol wurden an 80 mg Platinoxid 15 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 345 mg (2.97 mmol) Fumarsäure in 10 ml Methanol versetzt. Man rührte 17 Stunden bei Raumtemperatur und filtrierte anschliessend die weissen Kristalle ab. Man erhielt 0.45 g (56%) (S)-2-(7-Ethyl-1,4-dihydro-indeno[1,2-b]pyrrol-1-yl)-1-methyl-ethylamin fumarat (1:0.5) mit Smp. 189-190°.

Beispiel 18

a) Eine Lösung von 8 g (42 mmol) 6-Methoxycarbonyl-1-indanon, 8 ml (0.1 mol) 3-Buten-2-ol und 100 mg p-Toluolsulfonsäure in 80 ml 2,2-Dimethoxy-propan wurde 28 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 3:1). Man erhielt neben 1.3 g Edukt 8.5g (83%) (RS)-2-(2-Buten-1-yl)-6-methoxycarbonyl-1-indanon als gelbes Öl.

b) Durch eine auf -70° gekühlte Lösung von 10.5 g (42.9 mmol) (RS)-2-(2-Buten-1-yl)-6-methoxycarbonyl-1-indanon in 150 ml wasserfreiem Dichlormethan leitete man unter Rühren während 45 Minuten einen Ozonstrom (3 g Ozon/Stunde). Anschliessend spülte man die Lösung während 5 Minuten mit Sauerstoff und während 10 Minuten mit Argon. Nach Zugabe von 4.74 ml (64.4 mmol) Dimethylsulfid rührte man 18 Stunden bei Raumtemperatur. Das Reaktionsgemisch wurde im Vakuum eingedampft und durch Säulenchromatographie an Kieselgel gereinigt (Diethylether). Durch Umkristallisation des erhattenen Feststoffes erhielt man 4.5 g (45%) (RS)-2-(2-Oxoethyl)-6-methoxycarbonyl-1-indanon als schwach gelben Feststoff mit Smp. 92°.

c) Eine Lösung von 2.32 g (10 mmol) (RS)-2-(2-Oxoethyl)-6-methoxycarbonyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 2.2 g (81%) (RS)-1-(7-Methoxycarbonyl-1,4-dihydro-indeno[1,2-b]pyrrol-1-yl)-propan-2-ol als braunes Öl.

d) Zu einer auf 0° gekühlten Lösung von 2.2 g (8.1 mmol) (RS)-1-(7-Methoxycarbonyl-1,4-dihydro-indeno[1,2-b]pyrrol-1-yl)-propan-2-ol und 4.55 ml (32.4 mmol) Triethylamin in 60 ml Dichlormethan gab man tropfenweise unter Rühren 1.26 ml (16.2 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 1.05 g (16.2 mmol) Natriumazid versetzt und das Reaktionsgemisch unter Rühren 15 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 950 mg (40%) (RS)-1-(2-Azido-propyl)-7-methoxycarbonyl-1,4-dihydro-indeno[1,2-b]pyrrol als schwach braunes Öl.

e) 0.95 g (3.2 mmol) (RS)-1-(2-Azido-propyl)-7-methoxycarbonyl-1,4-dihydro-indeno-[1,2-b]-pyrrol gelöst in 70 ml wasserfreiem Methanol wurden an 95 mg Platinoxid 4 Stunden hydriert. Es wurde anschliessend vom Katalysator abfiltriert, mit Methanol nachgewaschen und das Lösungsmittel im Vakuum abgezogen. Das erhaltene farblose Öl wurde in 110 ml wasserfreiem Diethylether gelöst, filtriert und unter Rühren mit einer Lösung von 186 mg (1.6 mmol) Fumarsäure in 20 ml Methanol versetzt. Man rührte 3 Stunden bei Raumtemperatur und filtrierte anschliessend die weissen Kristalle ab. Man erhielt 770 mg (73%) (RS)-2-(7-Methoxycarbonyl-1,4-dihydro-indeno[1,2-b]pyrrol-1-yl)-1-methyl-ethylamin fumarat (1:0.5) mit Smp. 199-200°.

Beispiel 19

a) Zu einer auf 0° gekühlten Lösung von 3.55 g (10.7 mmol) (RS)-1-(2-Azido-propyl)-7-mesyloxycarbonyl-1,4-dihydro-indeno[1,2-b]pyrrol 90 ml wasserfreiem Diethylether und 90 ml wasserfreiem Tetrahydrofuran tropfte man über 5 Minuten 5.87 ml (11.7 mmol) einer 2 M Phenyllithiumlösung Nach 15 Minuten bei dieser Temperatur tropfte man nochmals 5.87 ml der Phenyllithiumlösung hinzu und rührte weitere 5 Minuten. Anschliessend versetzte man das Reaktionsgemisch mit 50 ml einer gesättigten Ammoniumchloridlösung und 25 ml Wasser. Es wurde einmal mit 50 ml Essigester extrahiert, die Wasserphase mit 1 N Salzsäure leicht sauer gestellt und dreimal mit jeweils 100 ml Essigester extrahiert. Die vereinigten organischen Phasen wurden einmal mit 100 ml gesättigter Natriumchloridlösung gewaschen, über Magnesiumsulfat getrocknet und im Vakuum eingeengt. Man erhielt 2.7 g (99 %) (RS)-1-(2-Azido-propyl)-7-hydroxy-1,4-dihydro-indeno[1,2-b]pyrrol als braunes Öl.

b) Zu einer Lösung von 1.05 g (4.13 mmol) (RS)-1-(2-Azido-propyl)-7-hydroxy-1,4-dihydro-indeno[1,2-b]pyrrol und 0.6 ml (7.4 mmol) Pyridin in 30 ml Dichlormethan gab man unter Rühren 0.7 ml (7.4 mmol) Acetanhydrid und rührte weitere 16 Stunden. Anschliessend versetzte man das Reaktionsgemisch mit 50 ml Dichlormethan und 40 ml Wasser, trennte die organische Phase ab und wusch diese einmal mit 50 ml gesättigter Natriumchloridlösung. Nach

dem Trocknen über Magnesiumsulfat, wurde im Vakuum eingeengt und das Rohprodukt durch Säulenchromatographie an Kieselgel (Essigester/Hexan 1:2) gereinigt. Man erhielt 0.9 g (85%) (RS)-1-(2-Azido-propyl)-7-acetoxy-1,4-dihydro-indeno[1,2-b]pyrrol als schwach oranges Öl.

c) 0.84 g (2.84 mmol) (RS)-1-(2-Azido-propyl)-7-acetoxy-1,4-dihydro-indeno-[1,2-b]-pyrrol gelöst in 100 ml wasserfreiem Ethanol wurden an 85 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 178 mg (1.54 mmol) Fumarsäure in 20 ml Methanol versetzt. Man rührte 4 Stunden bei Raumtemperatur und filtrierte anschliessend die weissen Kristalle ab. Man erhielt 400 mg (43%) (RS)-2-(7-Acetoxy-1,4-dihydro-indeno[1,2-b]pyrrol-1-yl)-1-methyl-ethylamin fumarat (1:0.5) mit Smp. 187-188°.

Beispiel 20

a) Eine Lösung von 1.88 g (10 mmol) (RS)-2-(2-Oxoethyl)-6-methyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 0.99 g (44%) (RS)-1-(7-Methyl-1,4-dihydro-indeno[1,2-b]pyrrol-1-yl)-propan-2-ol als braunes Öl.

b) Zu einer auf 0° gekühlten Lösung von 0.98 g (4.3 mmol) (RS)-1-(7-Methyl-1,4-dihydro-indeno[1,2-b]pyrrol-1-yl)-propan-2-ol und 2.4 ml (17.2 mmol) Triethylamin in 50 ml Dichlormethan gab man tropfenweise unter Rühren 0.67 ml (8.6 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 40 ml wasserfreiem Dimethylformamid gelöst, mit 0.56 g (8.6 mmol) Natriumazid versetzt und das Reaktionsgemisch unter Rühren 16 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 737 mg (68%) (RS)-1-(2-Azido-propyl)-7-methyl-1,4-dihydro-indeno[1,2-b]pyrrol als farbloses Öl.

c) 0.73 g (2.89 mmol) (RS)-1-(2-Azido-propyl)-7-methyl-1,4-dihydro-indeno-[1,2-b]-pyrrol gelöst in 40 ml wasserfreiem Ethanol wurden an 75 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 168 mg (1.45 mmol) Fumarsäure in 10 ml Methanol versetzt. Man rührte 17 Stunden bei Raumtemperatur und filtrierte anschliessend die weissen Kristalle ab. Man erhielt 596 mg (73%) (RS)-2-(7-Methyl-1,4-dihydro-indeno[1,2-b]pyrrol-1-yl)-1-methyl-ethylamin fumarat (1:0.5) mit Smp. 194°.

Beispiel 21

a) Eine Lösung von 2.08 g (10 mmol) (RS)-2-(2-Oxoethyl)-6-chlor-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 30 ml reduzierte. Das erkaltete Reaktionsgemisch wurde durch Säulenchromatographie an Kieselgel (Essigester/Toluol 3:7) gereinigt. Man erhielt 1.52 g (61%) (RS)-1-(7-Chlor-1,4-dihydro-indeno[1,2-b]pyrrol-1-yl)-propan-2-ol als braunes Öl, welches direkt in die nächste Reaktion eingesetzt wurde.

b) Zu einer auf 0°C gekühlten Lösung von 1.52 g (6.1 mmol) (RS)-1-(7-Chlor-1,4-dihydro-indeno[1,2-b]pyrrol-1-yl)-propan-2-ol und 3.4 ml (24.5 mmol) Triethylamin in 40 ml Dichlormethan gab man tropfenweise unter Rühren 0.95 ml (12.3 mmol) Methansulfonylchlorid und rührte weitere 1.5 Stunden bei dieser Temperatur. Das Reaktionsgemisch wurde anschliessend mit 280 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 30 ml wasserfreiem Dimethylformamid gelöst, mit 718 mg (11.0 mmol) Natriumazid versetzt und das Reaktionsgemisch unter Rühren 17

Stunden auf 60° erwärmt. Nach dem Abkühlen goss man die Lösung auf 140 ml Wasser und extrahierte zweimal mit jeweils 140 ml Diethylether und einmal mit 140 ml Essigester. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.0 g (60%) (RS)-1-(2-Azido-propyl)-7-chlor-1,4-dihydro-indeno[1,2-b]-pyrrol als Öl.

c) 1.0 g (3.7 mmol) (RS)-1-(2-Azido-propyl)-7-chlor-1,4-dihydro-indeno[1,2-b]pyrrol gelöst in 40 ml wasserfreiem Ethanol wurde an 100 mg Platinoxid 17 Stunden hydriert. Es wurde anschliessend vom Katalysator abfiltriert, mit Ethanol nachgewaschen und das Lösungsmittel im Vakuum abgezogen. Das erhaltene farblose Öl wurde in 75 ml wasserfreiem Diethylether gelöst, filtriert und unter Rühren mit einer Lösung von 195 mg (1.68 mmol) Fumarsäure in 15 ml Methanol versetzt. Man rührte 19 Stunden bei Raumtemperatur und filtrierte anschliessend die weissen Kristalle ab. Man erhielt 945 mg (85%) (RS)-2-(7-Chlor-1,4-dihydro-indeno[1,2-b]pyrrol-1-yl)-1-methyl-ethylamin fumarat (1:0.5) mit Smp. 206°.

Beispiel 22

a) Eine Lösung von 2 g (9.8 mmol) (RS)-2-(2-Oxoethyl)-6-methoxy-1-inda-non und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.94 g (39.2 mmol) (RS)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.7 g (71%) (RS)-1-(7-Methoxy-1,4-dihydro-indeno[1,2-b]pyrrol-1-yl)-propan-2-ol als Feststoff mit Smp. 76°.

b) Zu einer auf 0° C gekühlten Lösung von 1 g (4.1 mmol) (RS)-1-(7-Methoxy-1,4-dihydro-indeno[1,2-b]pyrrol-1-yl)-propan-2-ol und 2.3 ml (16.4 mmol) Triethylamin in 30 ml Dichlormethan gab man tropfenweise unter Rühren 0.64 ml (8.2 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.53 g (8.2 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 750 mg (68%) (RS)-1-(2-Azido-propyl)-7-methoxy-1,4-dihydro-indeno[1,2-b]pyrrol als farbloses Öl.

c) 1 g (3.7 mmol) (RS)-1-(2-Azido-propyl)-7-methoxy-1,4-dihydro-indeno-[1,2-b]pyrrol gelöst in 50 ml wasserfreiem Ethanol wurde an 100 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 216 mg (1.8 mmol) Fumarsäure in 20 ml Methanol versetzt. Man rührte 18 Stunden bei Raumtemperatur und filtrierte anschliessend die weissen Kristalle ab. Man erhielt 882 mg (79%) (RS)-2-(7-Methoxy-1,4-dihydro-indeno[1,2-b]pyrrol-1-yl)-1-methyl-ethylamin fumarat (1:0.5) mit Smp. 203°.

Beispiel **23**

a) Eine Lösung von 2.0 g (10.4 mmol) (RS)-2-(2-Oxoethyl)-5-fluor-1-indanon und 85 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.12 g (41.6 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 2:3) gereinigt. Man erhielt 1.66 g (69%) (RS)-1-(6-Fluor-1,4-dihydro-indeno[1,2-b]pyrrol-1-yl)-propan-2-ol als braunes Öl.

b) Zu einer auf 0° gekühlten Lösung von 1.66 g (7.17 mmol) (RS)-1-(6-Fluor-1,4-dihydro-indeno[1,2-b]pyrrol-1-yl)-propan-2-ol und 3.98 ml (28.7 mmol) Triethylamin in 60 ml Dichlormethan gab man tropfenweise unter Rühren 1.12 ml (14.3 mmol) Methansulfonylchlorid und rührte weitere 1.5 Stunden bei dieser Temperatur. Das Reaktionsgemisch wurde anschliessend mit 70 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.82 g (12.5 mmol) Natriumazid versetzt und das Reaktionsgemisch unter Rühren 17 Stunden auf 60° erwärmt. Nach dem Abkühlen goss man die Lösung auf 80 ml Wasser und extrahierte dreimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.45 g (24%) (RS)-1-(2-Azido-propyl)-6-fluor-1,4-dihydro-indeno[1,2-b]pyrrol als gelbes Öl.

c) 0.44 g (1.71 mmol) (RS)-1-(2-Azido-propyl)-6-fluor-1,4-dihydro-indeno-[1,2-b]pyrrol gelöst in 30 ml wasserfreiem Ethanol wurden an 45 mg Platinoxid 5 Stunden hydriert. Es wurde anschliessend vom Katalysator abfiltriert, mit Ethanol nachgewaschen und das Lösungsmittel im Vakuum abgezogen. Das erhaltene farblose Öl wurde in 40 ml wasserfreiem Diethylether gelöst, filtriert und unter Rühren mit einer Lösung von 100 mg (0.86 mmol) Fumarsäure in 7 ml Methanol versetzt. Man rührte 15 Stunden bei Raumtemperatur und filtrierte anschliessend die weissen Kristalle ab. Man erhielt 0.39 g (80%) (RS)-2-(6-Fluor-1,4-dihydro-indeno[1,2-b]pyrrol-1-yl)-1-methyl-ethylamin fumarat (1:0.5) mit Smp. 202°.

Beispiel **24**

a) Eine Lösung von 10.6 g (63.3 mmol) 5,6-Difluor-1-indanon, 13.1 ml (0.15 mol) 3-Buten-2-ol und 110 mg p-Toluolsulfonsäure in 110 ml 2,2-Dimethoxy-propan wurde 64 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 2:1). Man erhielt neben 3.6 g Edukt 5.32 g (38%) (RS)-2-(2-Buten-1-yl)-5,6-difluor-1-indanon als braunes Öl.

b) Durch eine auf -70° gekühlte Lösung von 5.3 g (23.8 mmol) (RS)-2-(2-Buten-1-yl)-5,6-difluor-1-indanon in 125 ml wasserfreiem Dichlormethan und 25 ml wasserfreiem Methanol leitete man unter Rühren während 40 Minuten einen Ozonstrom (2 g Ozon/Stunde). Anschliessend spülte man die Lösung während 5 Minuten mit Sauerstoff und während 10 Minuten mit Argon. Nach Zugabe von 2.64 ml (36 mmol) Dimethylsulfid rührte man 16 Stunden bei Raumtemperatur. Das Reaktionsgemisch wurde im Vakuum eingedampft, der Rückstand mit 60 ml Dichlormethan versetzt und nach Zugabe von 10 ml Wasser und 10 ml Trifluoressigsäure 2 Stunden bei Raumtemperatur gerührt. Man goss das Gemisch anschliessend auf 80 ml Wasser und neutralisierte unter Rühren durch spatelweise Zugabe von Natriumhydrogencarbonat. Es wurden weitere 40 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt ein gelbes Öl, welches aus Diethylether/Hexan kristallisiert wurde. Man erhielt 3.82 g (76%) (RS)-2-(2-Oxoethyl)-5,6-difluor-1-indanon als weissen Feststoff mit Smp. 78-81°.

c) Eine Lösung von 2.1 g (10 mmol) (RS)-2-(2-Oxoethyl)-5,6-difluor-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.13 g (45%) (R)-1-(6,7-Difluor-1,4-dihydro-indeno[1,2-b]pyrrol-1-yl)-propan-2-ol als braunen Feststoff, der ohne weitere Umkristallisation in die nächste Reaktion eingesetzt wurde.

d) Zu einer auf 0° gekühlten Lösung von 1.13 g (4.5 mmol) (R)-1-(6,7-Difluor-1,4-dihydro-indeno[1,2-b]pyrrol-1-yl)-propan-2-ol und 2.48 ml (18 mmol) Triethylamin in 50 ml Dichlormethan gab man tropfenweise unter Rühren 0.7 ml (9 mmol) Methansulfonylchlorid und rührte weitere 2 Stunden bei dieser Temperatur. Das Reaktionsgemisch wurde anschliessend mit 70 ml Dichlormethan verdünnt, zweimal mit jeweils 60 ml gesättigter Natriumhydrogencarbonatlösung gewaschen und die vereinigten Wasserphasen einmal mit 60 ml Dichlormethan extrahiert. Die vereinigten organischen Phasen wurden mit 90 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.58 g (8.98 mmol) Natriumazid versetzt und das Reaktionsgemisch unter Rühren 17 Stunden auf 60° erwärmt. Nach dem Abkühlen goss man die Lösung auf 70 ml Wasser und extrahierte dreimal mit jeweils 70 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 0.97 g (79%) (S)-1-(2-Azido-propyl)-6,7-difluor-1,4-dihydro-indeno[1,2-b]pyrrol als farbloses Öl.

e) 0.97 g (3.5 mmol) (S)-1-(2-Azido-propyl)-6,7-difluor-1,4-dihydro-indeno-[1,2-b]pyrrol gelöst in 50 ml wasserfreiem Ethanol wurden an 100 mg Platinoxid 18 Stunden hydriert. Es wurde anschliessend vom Katalysator abfiltriert, mit Ethanol nachgewaschen und das Lösungsmittel im Vakuum abgezogen. Der erhaltene weisse Feststoff wurde in 75 ml wasserfreiem Diethylether gelöst, filtriert und unter Rühren mit einer Lösung von 406 mg (3.5 mmol)

Fumarsäure in 20 ml Methanol versetzt. Man rührte 22 Stunden bei Raumtemperatur und filtrierte anschliessend die weissen Kristalle ab. Man erhielt 0.78 g (61%) (S)-2-(6,7-Difluor-1,4-dihydro-indeno[1,2-b]pyrrol-1-yl)-1-methyl-ethylamin fumarat (1:1) mit Smp. 215-217°.

Beispiel **25**

a) Eine Lösung von 14.5 g (64.5 mmol) 5-Chlor-6-methoxy-3,3-dimethyl-1-indanon, 13.3 ml (0.15 mol) 3-Buten-2-ol und 300 mg p-Toluolsulfonsäure in 150 ml 2,2-Dimethoxy-propan wurde 71 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 6:1). Man erhielt neben 2.25 g Edukt 11.9 g (66%) (RS)-2-(2-Buten-1-yl)-5-chlor-6-methoxy-3,3-dimethyl-1-indanon als schwach gelben Feststoff mit Smp. 86°.

b) Durch eine auf -70° gekühlte Lösung von 11.9 g (42.7 mmol) (RS)-2-(2-Buten-1-yl)-5-chlor-6-methoxy-3,3-dimethyl-1-indanon in 160 ml wasserfreiem Dichlormethan und 40 ml wasserfreiem Methanol leitete man unter Rühren während 50 Minuten einen Ozonstrom (3 g Ozon/Stunde). Anschliessend spülte man die Lösung während 5 Minuten mit Sauerstoff und während 10 Minuten mit Argon. Nach Zugabe von 4.7 ml (64 mmol) Dimethylsulfid rührte man 16 Stunden bei Raumtemperatur. Das Reaktionsgemisch wurde im Vakuum eingedampft, der Rückstand mit 120 ml Dichlormethan versetzt und nach Zugabe von 20 ml Wasser und 20 ml Trifluoressigsäure 2 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Das erhaltene Öl wurde aus Hexan/Essigester kristallisiert. Man erhielt 10.3 g (90%) (RS)-2-(2-Oxoethyl)-5-chlor-6-methoxy-3,3-dimethyl-1-indanon als schwach gelben Feststoff mit Smp. 102-103°.

c) Eine Lösung von 6.67 g (25 mmol) (RS)-2-(2-Oxoethyl)-5-chlor-6-methoxy-3,3-dimethyl-1-indanon und 150 mg p-Toluolsulfonsäure in 200 ml wasserfreiem Toluol wurde am Wasserabscheider erhitzt. Zu der siedenden Lösung tropfte man über einen Zeitraum von 5 Minuten eine Lösung von 7.51 g (0.1 mol) (R)-1-Amino-2-propanol in 40 ml wasserfreiem Toluol. Anschliessend wurde weitere 45 Minuten gekocht, wobei man das Lösungsmittel auf ein Volumen von 40 ml reduzierte. Das erkaltete Reaktionsgemisch wurde durch Säulenchromatographie an Kieselgel (Essigester/Hexan 1:1) gereinigt. Man erhielt 7.0 g (92%) (R)-1-(6-Chlor-7-methoxy-4,4-dimethyl-1,4-dihydro-indeno[1,2-b]pyrrol-1-yl)-propan-2-ol als braunes Öl.

d) Zu einer auf 0° gekühlten Lösung von 1.84 g (6.0 mmol) (R)-1-(6-Chlor-7-methoxy-4,4-dimethyl-1,4-dihydro-indeno[1,2-b]pyrrol-1-yl)-propan-2-ol und 3.35 ml (24 mmol) Triethylamin in 50 ml Dichlormethan gab man tropfenweise unter Rühren 0.93 ml (12 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 90 ml gesättigter Natriumhydrogencarbonatlösung gewaschen und die vereinigten Wasserphasen einmal mit 70 ml Dichlormethan extrahiert. Die vereinigten organischen Phasen wurden mit 90 ml gesättigter Natriumchloridlösung gewaschen, über Magnesiumsulfat getrocknet und im Vakuum eingedampft. Das erhaltene grüne Öl wurde in 50 ml wasserfreiem Dimethylformamid gelöst, mit 0.78 g (12 mmol) Natriumazid versetzt und das Reaktionsgemisch unter Rühren 17 Stunden auf 60° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 3:1) gereinigt. Man erhielt 1.58 g (80%) (S)-1-(2-Azido-propyl)-6-chlor-7-methoxy-4,4-dimethyl-1,4-dihydro-indeno[1,2-b]pyrrol als rotes Öl.

e) 1.56 g (4.72 mmol) (S)-1-(2-Azido-propyl)-6-chlor-7-methoxy-4,4-dimethyl-1,4-dihydro-indeno-[1,2-b]-pyrrol gelöst in 100 ml wasserfreiem Ethanol wurden an 160 mg Platinoxid 16 Stunden hydriert. Es wurde anschliessend vom Katalysator abfiltriert, mit Ethanol nachgewaschen und das Lösungsmittel im Vakuum abgezogen. Das erhaltene schwach gelbe Öl wurde in 125 ml wasserfreiem Diethylether gelöst, filtriert und unter Rühren mit einer Lösung von 548 mg (4.72 mmol) Fumarsäure in 15 ml Methanol versetzt. Man rührte 19 Stunden bei Raumtemperatur und filtrierte anschliessend die leicht gelben Kristalle ab. Man erhielt 1.57 g (80%) (S)-2-(6-Chlor-7-methoxy-4,4-dimethyl-1,4-dihydro-indeno[1,2-b]pyrrol-1-yl)-1-methyl-ethylamin fumarat (1:1) mit Smp. 186-188°.

Beispiel **26**

a) Eine Lösung von 1.92 g (10 mmol) (RS)-2-(2-Oxoethyl)-6-fluor-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.0 g (40 mmol) (S)-1-Amino-2-propanol in 20 ml wasserfreiem Toluol.

Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 2:3) gereinigt. Man erhielt 1.51 g (65%) (S)-1-(7-Fluor-1,4-dihydro-indeno[1,2-b]pyrrol-1-yl)-propan-2-ol als braunes Öl.

b) Zu einer auf 0° gekühlten Lösung von 1.51 g (6.5 mmol) (S)-1-(7-Fluor-1,4-dihydro-indeno[1,2-b]pyrrol-1-yl)-propan-2-ol und 3.64 ml (26.1 mmol) Triethylamin in 40 ml Dichlormethan gab man tropfenweise unter Rühren 0.51 ml (13.1 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 60 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 30 ml wasserfreiem Dimethylformamid gelöst, mit 711 mg (10.9 mmol) Natriumazid versetzt und das Reaktionsgemisch unter Rühren 16 Stunden auf 50° erwärmt. Nach dem Abkühlen goss man die Lösung auf 110 ml Wasser und extrahierte zweimal mit jeweils 110 ml Essigester. Die vereinigten organischen Phasen wusch man je einmal mit 90 ml Wasser und mit 90 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 631 mg (38%) (R)-1-(2-Azido-propyl)-7-fluor-1,4-dihydro-indeno[1,2-b]pyrrol als Öl.

c) 620 mg (2.4 mmol) (R)-1-(2-Azido-propyl)-7-fluor-1,4-dihydro-indeno[1,2-b]pyrrol gelöst in 30 ml wasserfreiem Ethanol wurden an 62 mg Platinoxid 15 Stunden hydriert. Es wurde anschliessend vom Katalysator abfiltriert, mit Ethanol nachgewaschen und das Lösungsmittel im Vakuum abgezogen. Das erhaltene farblose Öl wurde in 75 ml wasserfreiem Diethylether gelöst, filtriert und unter Rühren mit einer Lösung von 140 mg (1.2 mmol) Fumarsäure in 15 ml Methanol versetzt. Man rührte 4.5 Stunden bei Raumtemperatur und filtrierte anschliessend die weissen Kristalle ab. Man erhielt 409 mg (53%) (R)-2-(7-Fluor-1,4-dihydro-indeno[1,2-b]pyrrol-1-yl)-1-methyl-ethylamin fumarat (1:1) mit Smp. 174°.

## Beispiel **27**

a) Eine Lösung von 2 g (9.8 mmol) (RS)-2-(2-Oxoethyl)-6-methoxy-1-inda-non und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.94 g (39.2 mmol) (S)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.5 g (63%) (S)-1-(7-Methoxy-1,4-dihydro-indeno[1,2-b]pyrrol-1-yl)-propan-2-ol als Feststoff mit Smp. 74°.

b) Zu einer auf 0° gekühlten Lösung von 1.5 g (6.2 mmol) (S)-1-(7-Methoxy-1,4-dihydro-indeno[1,2-b]pyrrol-1-yl)-propan-2-ol und 3.45 ml (24.6 mmol) Triethylamin in 50 ml Dichlormethan gab man tropfenweise unter Rühren 0.96 ml (12.3 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 60 ml wasserfreiem Dimethylformamid gelöst, mit 0.71 g (10.9 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.0 g (60%) (R)-1-(2-Azido-propyl)-7-methoxy-1,4-dihydro-indeno[1,2-b]pyrrol als farbloses Öl.

c) 1 g (3.7 mmol) (R)-1-(2-Azido-propyl)-7-methoxy-1,4-dihydro-indeno[1,2-b]pyrrol gelöst in 50 ml wasserfreiem Ethanol wurde an 100 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 240 mg (2.07 mmol) Fumarsäure, gelöst in 20 ml Methanol, versetzt. Man rührte 18 Stunden bei Raumtemperatur und filtrierte anschliessend die weissen Kristalle ab. Man erhielt 760 mg (68%) (R)-2-(7-Methoxy-1,4-dihydro-indeno[1,2-b]pyrrol-1-yl)-1-methylethylamin fumarat (1:0.5) mit Smp. 207°.

## Beispiel **28**

a) Eine Lösung von 2.5 g (12.0 mmol) (RS)-2-(2-Oxoethyl)-6-chlor-1-inda-non und 100 mg p-Toluolsulfonsäure in 120 ml wasserfreiem Toluol wurde am Wasserabscheider erhitzt. Zu der siedenden Lösung tropfte man über einen

Zeitraum von 5 Minuten eine Lösung von 3.6 g (47.9 mmol) (S)-1-Amino-2-propanol in 20 ml wasserfreiem Toluol. Anschliessend wurde wei-tere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.56 g (53%) (S)-1-(7-Chlor-1,4-dihydro-indeno[1,2-b]pyrrol-1-yl)-propan-2-ol als braunes Öl, welches direkt in die nächste Reaktion eingesetzt wurde.

b) Zu einer auf 0° gekühlten Lösung von 1.55 g (6.3 mmol) (S)-1-(7-Chlor-1,4-dihydro-indeno[1,2-b]pyrrol-1-yl)-propan-2-ol und 3.5 ml (25.0 mmol) Triethylamin in 40 ml Dichlormethan gab man tropfenweise unter Rühren 0.97 ml (12.5 mmol) Methansulfonylchlorid und rührte weitere 1.5 Stunden bei dieser Temperatur. Das Reaktionsgemisch wurde anschliessend mit 200 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 30 ml wasser-freiem Dimethylformamid gelöst, mit 815 mg (12.5 mmol) Natriumazid versetzt und das Reaktionsgemisch unter Rühren 17 Stunden auf 60° erwärmt. Nach dem Abkühlen goss man die Lösung auf 140 ml Wasser und extrahierte zweimal mit jeweils 140 ml Essigester. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 833 mg (49%) (R)-1-(2-Azido-propyl)-7-chlor-1,4-dihydro-indeno[1,2-b]pyrrol als Öl.

c) 750 mg (2.8 mmol) (R)-1-(2-Azido-propyl)-7-chlor-1,4-dihydro-indeno-[1,2-b]pyrrol gelöst in 30 ml wasserfreiem Ethanol wurden an 75 mg Platin-oxid 16 Stunden hydriert. Es wurde anschliessend vom Katalysator abge-nutscht, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 154 mg (1.3 mmol) Fumarsäure in 15 ml Methanol versetzt. Man rührte 6 Stunden bei Raumtemperatur und filtrierte anschliessend die weissen Kristalle ab. Man erhielt 633 mg (76%) (R)-2-(7-Chlor-1,4-dihydro-indeno[1,2-b]pyrrol-1-yl)-1-methyl-ethylamin fumarat (1:0.5) mit Smp. 195°.

Beispiel **29**

a) Eine Lösung von 3 g (13.9 mmol) (RS)-2-(2-Oxoethyl)-7-methoxy-1-tetralon und 150 mg p-Toluolsulfonsäure in 130 ml wasserfreiem Toluol wurde am Wasserabscheider erhitzt. Zu der siedenden Lösung tropfte man über einen Zeitraum von 5 Minuten eine Lösung von 4.16 g (55.5 mmol) (S)-1-Amino-2-propanol in 20 ml wasserfreiem Toluol. Anschliessend wurde weitere 30 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:1) gereinigt. Man erhielt 3.0 g (84%) (S)-1-(4,5-Dihydro-8-methoxy-1H-benz[g]indol-1-yl)-propan-2-ol als braunes Öl, welches direkt in die nächste Reaktion eingesetzt wurde.

b) Zu einer auf 0° gekühlten Lösung von 3 g (11.7 mmol) (S)-1-(4,5-Dihydro-8-methoxy-1H-benz[g]indol-1-yl)-propan-2-ol und 6.55 ml (46.7 mmol) Triethylamin in 80 ml Dichlormethan gab man tropfenweise unter Rühren 1.8 ml (23.3 mmol) Methansulfonylchlorid und rührte weitere 1.5 Stunden bei dieser Temperatur. Das Reaktionsgemisch wurde anschlies-send mit 300 ml Diethylether verdünnt, zweimal mit jeweils 100 ml gesättigter Natriumhydrogencarbonatlösung gewaschen und die vereinigten Wasserphasen einmal mit 100 ml Diethylether extrahiert. Die vereinigten organischen Phasen wurden mit 100 ml gesättigter Natriumchloridlösung gewaschen, über Magnesiumsulfat getrocknet und im Vakuum eingedampft. Das erhaltene Öl wurde in 70 ml wasserfreiem Dimethylformamid gelöst, mit 1.52 g (23.3 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.9 g (58%) (R)-1-(2-Azido-propyl)-4,5-dihydro-8-methoxy-1-H-benz[g]indol als schwach gelbes Öl.

c) 1.9 g (6.4 mmol) (R)-1-(2-Azido-propyl)-4,5-dihydro-8-methoxy-1H-benz[g]indol gelöst in 100 ml wasserfreiem Ethanol wurden an 190 mg Platinoxid 16 Stunden unter hydriert. Es wurde anschliessend vom Katalysator abfiltriert, mit Ethanol nachgewaschen und das Lösungsmittel im Vakuum abgezogen. Man erhielt 1.64 g (95%) (R)-2-(4,5-Dihydro-8-methoxy-1H-benz[g]indol-1-yl)-1-methyl-ethylamin als farbloses Öl, von dem 440 mg (1.7 mmol) in 70 ml wasserfreiem Diethylether gelöst, filtriert und unter Rühren mit einer Lösung von 200 mg (1.7 mmol) Fumarsäure in 20 ml Methanol versetzt wurden Man rührte 2 Stunden bei Raumtemperatur und filtrierte anschliessend die weissen Kristalle ab. Man erhielt 450 mg (70%) (R)-2-(4,5-Dihydro-8-methoxy-1H-benz[g]indol-1-yl)-1-methylethylamin fumarat (1:1) mit Smp. 194°.

Beispiel **30**

a) Eine Lösung von 1.2 g (4.7 mmol) (R)-2-(4,5-Dihydro-8-methoxy-1H-benz[g]indol-1-yl)-1-methyl-ethylamin, 0.52 g (5.1 mmol) Triethylamin und 0.83 g (5.8 mmol) Trifluoressigsäureethylester in 50 ml wasserfreiem Methanol wurde 27 Stunden bei Raumtemperatur gerührt. Nachdem man das Lösungsmittel im Vakuum abgezogen hatte, nahm man den Rückstand mit 70 ml wasserfreiem Dioxan auf, gab 1.8 g (7.9 mmol) DDQ hinzu und kochte 1.5 Stunden unter Rückfluss. Anschliessend engte man das Reak-tionsgemisch im Vakuum ein und reinigte den Rückstand durch Säulen-chromatographie an Kieselgel (Dichlormethan/Aceton 4:1). Man erhielt 0.97 g (59%) (R)-N-[2-(8-Methoxy-1H-benz[g]indol-1-yl)-1-methyl-ethyl]-trifluor-acetamid als hellbraunen Feststoff, der ohne weitere Umkristallisation in die nächste Reaktion eingesetzt wurde.

b) Eine Mischung aus 0.97 g (2.8 mmol) (R)-N-[2-(8-Methoxy-1H-benz[g]indol-1-yl)-1-methyl-ethyl]-trifluoracetamid, 1 g (17.8 mmol) Kaliumhydroxid, 2 ml Wasser und 40 ml Methanol wurde 15 Stunden unter Rückfluss gekocht. Das Reaktionsgemisch wurde anschliessend in 80 ml 1N Natronlauge gegossen, zweimal mit jeweils 80 ml Diethylether und einmal mit 80 ml Essigester extrahiert, die vereinigten organischen Phasen wurden einmal mit 120 ml gesättigter Natriumchlorid-Lösung gewaschen und über Magnesiumsulfat getrocknet. Nach dem Einengen im Vakuum, wurde der Rückstand in 100 ml wasserfreiem Diethylether gelöst, filtriert und unter Rühren mit einer Lösung von 322 mg (2.77 mmol) Fumarsäure in 30 ml Methanol versetzt. Man rührte 3 Stunden bei Raumtemperatur und filtrierte anschliessend die weissen Kristalle ab. Man erhielt 600 mg (69%) (R)-2-(8-Methoxy-1H-benz[g]indol-1-yl)-1-methyl-ethylamin fumarat (1:0.5) mit Smp. 209°.

Beispiel **31**

a) Eine Lösung von 20 g (104 mmol) 5,6-Dimethoxy-1-indanon, 21.5 ml (0.25 mol) 3-Buten-2-ol und 200 mg p-Toluolsulfonsäure in 21.5 ml 2,2-Dimethoxy-propan und 200 ml wasserfreiem Toluol wurde 24 Stunden unter Rückfluss gekocht. Das Reaktionsgemisch wurde anschliessend im Vakuum eingeengt und durch Säulenchromatographie an Kieselgel (Hexan/ Essig-ester 3:2). gereinigt. Man erhielt 6.8 g (27%) (RS)-2-(2-Buten-1-yl)-5,6-dime-thoxy-1-indanon als gelbes Öl.

b) Durch eine auf -70° gekühlte Lösung von 6.8 g (27.6 mmol) (RS)-2-(2-Buten-1-yl)-5,6-dimethoxy-1-indanon in 100 ml wasserfreiem Dichlormethan und 20 ml wasserfreiem Methanol leitete man unter Rühren während 30 Minuten einen Ozonstrom (3 g Ozon/Stunde). Anschliessend spülte man die Lösung während 5 Minuten mit Sauerstoff und während 10 Minuten mit Argon. Nach Zugabe von 4 ml (54.3 mmol) Dimethylsulfid rührte man 15 Stunden bei Raumtemperatur. Das Reaktionsgemisch wurde im Vakuum eingedampft, der Rückstand mit 250 ml Dichlormethan versetzt und nach Zugabe von 10 ml Wasser und 10 ml Trifluoressigsäure 1.5 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 70 ml Wasser zugegeben, die Phasen getrennt und die Wasserphase zweimal mit jeweils 120 ml Dichlormethan extrahiert. Die vereinigten organischen Phasen wurden über Magnesiumsulfat getrocknet, im Vakuum eingeengt und das erhaltene Rohprodukt aus Diethylether/Hexan kristallisiert. Man erhielt 4.7 g (73%) (RS)-2-(2-Oxoethyl)-5,6-dimethoxy-1-indanon als schwach gelben Feststoff mit Smp. 122°.

c) Eine Lösung von 2 g (8.5 mmol) (RS)-2-(2-Oxoethyl)-5,6-dimethoxy-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.56 g (34.2 mmol) (RS)-1-Amino-2-propanol in 20 ml wasserfreiem Toluol. Anschliessend wurde weitere 30 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 2:3) gereinigt. Man erhielt 0.91 g (40%) (RS)-1-(1,4-Dihydro-6,7-dimethoxy-indeno[1,2-b]pyrrol-1-yl)-propan-2-ol als Öl.

d) Zu einer auf 0° gekühlten Lösung von 0.91 g (3.3 mmol) (RS)-1-(1,4-Dihydro-6,7-dimethoxy-indeno[1,2-b]pyrrol-1-yl)-propan-2-ol und 1.86 ml (13.4 mmol) Triethylamin in 25 ml Dichlormethan gab man tropfenweise unter Rühren 0.52 ml (6.7 mmol) Methansulfonylchlorid und rührte weitere 1.5 Stunden bei dieser Temperatur. Das Reaktionsgemisch wurde anschliessend mit 140 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natrium-chloridlösung gewaschen, über Magnesiumsulfat getrocknet und im Vakuum eingedampft. Das erhaltene braune Öl wurde in 40 ml wasserfreiem Dimethylformamid gelöst, mit 0.43 g (6.7 mmol) Natriumazid versetzt und das Reaktionsgemisch unter Rühren 15 Stunden auf 60° erwärmt. Nach dem Abkühlen goss man die Lösung auf 140 ml Wasser und extrahierte zweimal mit jeweils 140 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol/Essigester 4:1)

gereinigt. Man erhielt 400 mg (40%) (RS)-1-(2-Azido-propyl)-1,4-dihydro-6,7-dimethoxy-indeno[1,2-b]pyrrol als Öl, welches ohne weitere Reinigung in die nächste Reaktion eingesetzt wurde.

e) 0.4 g (1.3 mmol) (RS)-1-(2-Azido-propyl)-1,4-dihydro-6,7-dimethoxy-indeno[1,2-b]pyrrol gelöst in 20 ml wasserfreiem Ethanol wurden an 40 mg Platinoxid 16 Stunden hydriert. Es wurde anschliessend vom Katalysator abfiltriert, mit Ethanol nachgewaschen und das Lösungsmittel im Vakuum abgezogen. Das erhaltene farblose Öl wurde in 25 ml wasserfreiem Diethylether gelöst, filtriert und unter Rühren mit einer Lösung von 62.2 mg (0.54 mmol) Fumarsäure in 5 ml Methanol versetzt. Man rührte 18 Stunden bei Raumtemperatur und filtrierte anschliessend die weissen Kristalle ab. Man erhielt 314 mg (71%) (RS)-2-(1,4-Dihydro-6,7-dimethoxy-indeno[1,2-b]pyrrol-1-yl)-1-methyl-ethylamin fumarat (1:0.5) mit Smp. 203-205°.

Beispiel **32**

a) Eine Lösung von 48 g (0.324 mol) 4-Chromanon, 67 ml (0.78 mol) 3-Buten-2-ol und 500 mg p-Toluolsulfonsäure in 67 ml 2,2-Dimethoxypropan und 500 ml wasserfreiem Toluol wurde 46 Stunden unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt 24.7 g (38%) (RS)-2-(2-Buten-1-yl)-4-chromanon als gelbes Oel.

b) Durch eine auf -70° gekühlte Lösung von 24.6 g (0.12 mol) (RS)-2-(2-Buten-1-yl)-4-chromanon in 450 ml wasserfreiem Dichlormethan und 150 ml wasserfreiem Methanol leitete man unter Rühren während 2 Stunden einen Ozonstrom (3.5 g Ozon/Stunde). Anschliessend spülte man die Lösung während 5 Minuten mit Sauerstoff und während 15 Minuten mit Argon. Nach Zugabe von 13.4 ml (0.18 mol) Dimethylsulfid rührte man 20 Stunden bei Raumtemperatur. Das Reaktionsgemisch wurde anschliessend im Vakuum eingedampft, der Rückstand mit 250 ml Dichlormethan versetzt und nach Zugabe von 40 ml Wasser und 40 ml Trifluoressigsäure 4 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 70 ml Wasser zugegeben, die Phasen getrennt und die Wasserphase zweimal mit jeweils 120 ml Dichlormethan extrahiert. Die vereinigten organischen Phasen wurden über Magnesiumsulfat getrocknet und im Vakuum eingeengt. Man erhielt 22.7 g (99%) 2-(2-Oxoethyl)-4-chromanon als gelbes Öl, welches ohne weitere Reinigung in die nächste Reaktion eingesetzt wurde.

c) Eine Lösung von 1.9 g (10 mmol) (RS)-2-(2-Oxoethyl)-4-chromanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 35 Minuten gekocht, wobei man das Lösungsmittel auf ein Volumen von 20 ml reduzierte. Das erkaltete Reaktionsgemisch wurde durch Säulenchromato-graphie an Kieselgel (Essigester/Toluol 2:3) gereinigt. Man erhielt 1.82 g (79%) (RS)-1-(1,4-Dihydro-[1]benzopyrano[4,3-b]pyrrol-1-yl)-propan-2-ol als gelbes Öl.

d) Zu einer auf 0° gekühlten Lösung von 1.82 g (7.9 mmol) (RS)-1-(1,4-Dihydro-[1]benzopyrano[4,3-b]pyrrol-1-yl)-propan-2-ol und 4.4 ml (31.7 mmol) Triethylamin in 50 ml wasserfreiem Dichlormethan gab man tropfenweise unter Rühren 1.23 ml (15.9 mmol) Methansulfonylchlorid und rührte weitere 1.5 Stunden bei dieser Temperatur. Das Reaktionsgemisch wurde anschliessend mit 280 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 140 ml gesättigter Natrium-chloridlösung gewaschen, über Magnesiumsulfat getrocknet und im Vakuum eingedampft. Das erhaltene braune Öl wurde in 50 ml wasserfreiem Dimethylformamid gelöst, mit 1.03 g (15.9 mmol) Natriumazid versetzt und das Reaktionsgemisch unter Rühren 17 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.44 g (71%) (RS)-1-(2-Azido-propyl)-1,4-dihydro-[1]benzopyrano[4,3-b]pyrrol als farb-loses Öl.

e) 1.44 g (5.7 mmol) (RS)-1-(2-Azido-propyl)-1,4-dihydro-[1]benzopyrano-[4,3-b]-pyrrol gelöst in 60 ml wasserfreiem Ethanol wurden an 150 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 329 mg (2.83 mmol) Fumarsäure in 20 ml Methanol versetzt. Man rührte 18 Stunden bei Raumtemperatur und filtrierte anschliessend die weissen Kristalle ab. Man erhielt 1.42 g (88%) (RS)-1-(1,4-Dihydro-[1]benzopyrano-[4,3-b]pyrrol-1-yl)-1-methyl-ethylamin fumarat (1:0.5) mit Smp. 208-209°.

Beispiel **33**

a) Zu einer auf -70° gekühlten Lösung von 2.96 g (18.3 mmol) 6-Methoxy-1-indanon in 300 ml wasserfreiem Tetrahydrofuran gab man unter Rühren tropfenweise eine aus 3.12 ml (22 mmol) Diisopropylamin und 13.8 ml (22 mmol) 1.6 N n-Butyllithium in Hexan frisch hergestellte Lithiumdiisopropylamid-lösung in 40 ml wasserfreiem Tetrahydrofuran. Man rührte weitere 30 Minuten bei dieser Temperatur und tropfte anschliessend über 10 Minuten eine Lösung von 2.03 ml (20.2 mmol) 3-Chlor-2-butenon in 40 ml wasserfreiem Tetrahydrofuran gelöst hinzu. Man liess das Reaktionsgemisch über 30 Minuten auf 0° kommen und rührte weitere 30 Minuten bei dieser Temperatur. Anschliessend goss man das Reaktionsgemisch auf 150 ml Eis, gab 150 ml gesättigte Natriumchloridlösung dazu und trennte die organische Phase ab. Die Wasserphase wurde einmal mit 400 ml Diethylether extrahiert, die vereinigten organischen Phasen einmal mit 200 ml gesättigter Natriumchloridlösung gewaschen, über Magnesiumsulfat getrocknet und im Vakuum eingeengt. Das erhaltene rote Öl wurde durch Säulenchromatographie an Kieselgel (Hexan/Diethylether 3:2) gereinigt. Man erhielt neben 0.93 g Edukt 1.56 g (37%) rac-6-Methoxy-2-(3-oxo-2-butyl)-1-indanon als gelbes Öl.

b) Eine Lösung von 1.5 g (6.46 mmol) rac-6-Methoxy-2-(3-oxo-2-butyl)-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 1.94 g (25.8 mol) (R)-1-Amino-2-propanol in 20 ml wasserfreiem Toluol. Anschliessend wurde weitere 85 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 2:3) gereinigt. Man erhielt 1.23 g (70%) (R)-1-(7-Methoxy-2,3-dimethyl-1,4-dihydro-indeno[1,2-b]pyrrol-1-yl)-propan-2-ol als gelbes Öl.

c) Zu einer auf 0° gekühlten Lösung von 1.22 g (4.5 mmol) (R)-1-(7-Methoxy-2,3-dimethyl-1,4-dihydro-indeno[1,2-b]pyrrol-1-yl)-propan-2-ol und 2.5 ml (18 mmol) Triethylamin in 50 ml Dichlormethan gab man tropfenweise unter Rühren 0.7 ml (9.0 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 60 ml gesättigter Natriumhydrogencarbonatlösung gewaschen und die vereinigten Wasserphasen einmal mit 60 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.58 g (9.0 mmol) Natriumazid versetzt und das Reaktionsgemisch unter Rühren 18 Stunden auf 80° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.64 g (48%) (S)-1-(2-Azido-propyl)-7-methoxy-2,3-dimethyl-1,4-dihydro-indeno[1,2-b]pyrrol als schwach gelbes Öl.

d) 0.63 g (2.12 mmol) (S)-1-(2-Azido-propyl)-7-methoxy-2,3-dimethyl-1,4-dihydro-indeno-[1,2-b]-pyrrol gelöst in 40 ml wasserfreiem Ethanol wurden an 63 mg Platinoxid 2.5 Stunden hydriert. Es wurde anschliessend vom Katalysator abfiltriert, mit Ethanol nachgewaschen und das Lösungsmittel im Vakuum abgezogen. Das erhaltene schwach gelbe Öl wurde in 50 ml wasserfreiem Diethylether gelöst, filtriert und unter Rühren mit einer Lösung von 123 mg (1.06 mmol) Fumarsäure in 10 ml Methanol versetzt. Man rührte 16 Stunden bei Raumtemperatur und filtrierte anschliessend die leicht gelben Kristalle ab. Man erhielt 528 mg (76%) (S)-2-(7-Methoxy-2,3-dimethyl-1,4-dihydro-indeno[1,2-b]pyrrol-1-yl)-1-methyl-ethylamin fumarat (1:0.5) mit Smp. 197°.

Beispiel **34**

a) Zu einer auf -70° gekühlten Lösung von 2.96 g (18.3 mmol) 6-Methoxy-1-indanon in 300 ml wasserfreiem Tetrahydrofuran gab man unter Rühren tropfenweise eine aus 3.12 ml (22 mmol) Diisopropylamin und 13.8 ml (22 mmol) 1.6 N n-Butyllithium in Hexan frisch hergestellte Lithiumdiisopropylamid-lösung in 40 ml wasserfreiem Tetrahydrofuran. Man rührte weitere 30 Minuten bei dieser Temperatur und tropfte anschliessend über 10 Minuten eine Lösung von 1.62 ml (20.2 mmol) Chloraceton in 40 ml wasserfreiem Tetrahydrofuran gelöst hinzu. Man liess das Reaktionsgemisch über 90 Minuten auf Raumtemperatur kommen und rührte weitere 45 Minuten bei dieser Temperatur. Anschliessend goss man das Reaktionsgemisch auf 100 ml Eis, gab 100 ml gesättigte Natriumchloridlösung dazu und trennte die organische Phase ab. Die Wasserphase wurde einmal mit 300 ml Diethylether extrahiert, die vereinigten organischen Phasen einmal mit 100 ml gesättigter Natriumchloridlösung gewaschen, über Magnesiumsulfat getrocknet und im Vakuum eingeengt. Das erhaltene rote Öl wurde durch Säulenchromatographie an Kieseigel (Hexan/Diethylether 3:2) gereinigt. Man erhielt 2.24 g (56%) (RS)-6-Methoxy-2-(2-oxopropyl)-1-indanon als gelben Feststoff, der ohne weitere Umkristallisation in die nächste Reaktion eingesetzt wurde.

b) Eine Lösung von 1.45 g (6.64 mmol) (RS)-6-Methoxy-2-(2-oxopropyl)-1-indanon und 60 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.0 g (26.6 mmol) (R)-1-Amino-2-propanol in 20 ml wasserfreiem Toluol. Anschliessend wurde weitere 90 Minuten gekocht, wobei man das Lösungsmittel auf ein Volumen von 20 ml reduzierte. Das erkaltete Reaktionsgemisch wurde durch Säulenchromatographie an Kieselgel (Essig-

ester/Toluol 2:3) gereinigt. Man erhielt 1.05 g (61%) (R)-1-(7-Methoxy-2-methyl-1,4-dihydro-indeno[1,2-b]pyrrol-1-yl)-propan-2-ol als gelben Feststoff mit Smp. 110°.

c) Zu einer auf 0° gekühlten Lösung von 0.8 g (3.1 mmol) (R)-1-(7-Methoxy-2-methyl-1,4-dihydro-indeno[1,2-b]pyrrol-1-yl)-propan-2-ol und 1.73 ml (12.4 mmol) Triethylamin in 40 ml Dichlormethan gab man tropfenweise unter Rühren 0.48 ml (6.2 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 60 ml gesättigter Natriumhydrogencarbonatlösung gewaschen und die vereinigten Wasserphasen einmal mit 60 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 25 ml wasserfreiem Dimethylformamid gelöst, mit 0.40 g (6.2 mmol) Natriumazid versetzt und das Reaktionsgemisch unter Rühren 16 Stunden auf 60° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.44 g (50%) (S)-1-(2-Azido-propyl)-7-methoxy-2-methyl-1,4-dihydro-indeno[1,2-b]pyrrol als schwach gelbes Öl.

d) 0.44 g (1.56 mmol) (S)-1-(2-Azido-propyl)-7-methoxy-2-methyl-1,4-dihydro-indeno-[1,2-b]-pyrrol gelöst in 35 ml wasserfreiem Ethanol wurden an 45 mg Platinoxid 16 Stunden hydriert. Es wurde anschliessend vom Katalysator abfiltriert, mit Ethanol nachgewaschen und das Lösungsmittel im Vakuum abgezogen. Das erhaltene schwach gelbe Öl wurde in 35 ml wasserfreiem Diethylether gelöst, filtriert und unter Rühren mit einer Lösung von 90 mg (0.78 mmol) Fumarsäure in 7 ml Methanol versetzt. Man rührte 18 Stunden bei Raumtemperatur und filtrierte anschliessend die leicht gelben Kristalle ab. Man erhielt 414 mg (84%) (S)-2-(7-Methoxy-2-methyl-1,4-dihydro-indeno[1,2-b]pyrrol-1-yl)-1-methyl-ethylamin fumarat (1:0.5) mit Smp. 199°.

<u>Beispiel **35**</u>

a) Eine Lösung von 14.0 g (84 mmol) 4-Chlor-1-indanon, 17.3 ml (0.20 mol) 3-Buten-2-ol und 140 mg p-Toluolsulfonsäure in 140 ml 2,2-Dimethoxy-propan wurde 64 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexar/Diethylether 6:1). Man erhielt 15.2 g (81%) (RS)-2-(2-Buten-1-yl)-4-chlor-1-indanon als gelbes Öl.

b) Durch eine auf -70° gekühlte Lösung von 15.1 g (68.4 mmol) (RS)-2-(2-Buten-1-yl)-4-chlor-1-indanon in 200 ml wasserfreiem Dichlormethan und 40 ml wasserfreiem Methanol leitete man unter Rühren während 90 Minuten einen Ozonstrom (3 g Ozon/Stunde). Anschliessend spülte man die Lösung während 5 Minuten mit Sauerstoff und während 10 Minuten mit Argon. Nach Zugabe von 7.55 ml (103 mmol) Dimethylsulfid rührte man 20 Stunden bei Raumtemperatur. Das Reaktionsgemisch wurde im Vakuum eingedampft, der Rückstand mit 200 ml Dichlormethan versetzt und nach Zugabe von 25 ml Wasser und 25 ml Trifluoressigsäure 2 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 120 ml Dichlormethan extrahiert. Die vereinigten organischen Phasen wurden über Magnesiumsulfat getrocknet und im Vakuum eingeengt. Man erhielt 13.9 g (97%) (RS)-2-(2-Oxoethyl)-4-chlor-1-indanon als schwach gelbes Öl.

c) Eine Lösung von 2.08 g (10.0 mmol) (RS)-2-(2-Oxoethyl)-4-chlor-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 1:2) gereinigt. Man erhielt 1.47 g (59%) (RS)-1-(5-Chlor-1,4-dihydro-indeno[1,2-b]pyrrol-1-yl)-propan-2-ol als braunes Öl.

d) Zu einer auf 0° gekühlten Lösung von 1.47 g (5.93 mmol) (RS)-1-(5-Chlor-1,4-dihydro-indeno[1,2-b]pyrrol-1-yl)-propan-2-ol und 3.3 ml (23.7 mmol) Triethylamin in 50 ml Dichlormethan gab man tropfenweise unter Rühren 0.92 ml (11.9 mmol) Methansulfonylchlorid und rührte weitere 1.5 Stunden bei dieser Temperatur. Das Reaktionsgemisch wurde anschliessend mit 50 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.77 g (11.9 mmol) Natriumazid versetzt und das Reaktionsgemisch unter Rühren 17 Stunden auf 60° erwärmt. Nach dem Abkühlen goss man die Lösung auf 80 ml Wasser und extrahierte dreimal mit jeweils 100 ml Essigester. Die vereinigten organischen Phasen wusch man je einmal mit 70 ml Wasser und mit 70 ml

gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 1.0 g (62%) (RS)-1-(2-Azido-propyl)-5-chlor-1,4-dihydro-indeno[1,2-b]pyrrol als schwach gelbes Öl.

e) 1.0 g (3.66 mmol) (RS)-1-(2-Azido-propyl)-5-chlor-1,4-dihydro-indeno-[1,2-b]pyrrol gelöst in 50 ml wasserfreiem Ethanol wurden an 100 mg Platinoxid 5 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 212 mg (1.83 mmol) Fumarsäure in 16 ml Methanol versetzt. Man rührte 18 Stunden bei Raumtemperatur und filtrierte anschliessend die weissen Kristalle ab. Man erhielt 1.05 g (94%) (RS)-2-(5-Chlor-1,4-dihydro-indeno[1,2-b]pyrrol-1-yl)-1-methyl-ethylamin fumarat (1:0.5) mit Smp. 182°.

Beispiel **36**

a) Eine Lösung von 1.8 g (8.25 mmol) (RS)-6-Methoxy-2-(2-oxopropyl)-1-indanon und 70 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.48 g (33 mmol) (RS)-1-Amino-2-propanol in 20 ml wasserfreiem Toluol. Anschliessend wurde weitere 90 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.37 g (65%) (RS)-1-(7-Methoxy-2-methyl-1,4-dihydro-indeno[1,2-b]pyrrol-1-yl)-propan-2-ol als braunen Feststoff, der direkt in die nächste Reaktion eingesetzt wurde.

b) Zu einer auf 0° gekühlten Lösung von 1.35 g (5.25 mmol) (RS)-1-(7-Methoxy-2-methyl-1,4-dihydro-indeno[1,2-b]pyrrol-1-yl)-propan-2-ol und 2.92 ml (21.0 mmol) Triethylamin in 60 ml Dichlormethan gab man tropfenweise unter Rühren 0.81 ml (10.5 mmol) Methansulfonylchlorid und rührte weitere 1.5 Stunden bei dieser Temperatur. Das Reaktionsgemisch wurde anschliessend mit 120 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 90 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.68 g (10.5 mmol) Natriumazid versetzt und das Reaktionsgemisch unter Rühren 23 Stunden auf 80° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.93 g (63%) (RS)-1-(2-Azido-propyl)-7-methoxy-2-methyl-1,4-dihydro-indeno[1,2-b]pyrrol als schwach gelbes Öl.

c) 0.92 g (3.26 mmol) (RS)-1-(2-Azido-propyl)-7-methoxy-2-methyl-1,4-dihydro-indeno-[1,2-b]-pyrrol gelöst in 70 ml wasserfreiem Ethanol wurden an 90 mg Platinoxid 16 Stunden hydriert. Es wurde anschliessend vom Katalysator abfiltriert, mit Ethanol nachgewaschen und das Lösungsmittel im Vakuum abgezogen. Das erhaltene schwach gelbe Öl wurde in 70 ml wasserfreiem Diethylether gelöst, filtriert und unter Rühren mit einer Lösung von 189 mg (1.63 mmol) Fumarsäure in 15 ml Methanol versetzt. Man rührte 18 Stunden bei Raumtemperatur und filtrierte anschliessend die leicht gelben Kristalle ab. Man erhielt 800 mg (78%) (RS)-2-(7-Methoxy-2-methyl-1,4-dihydro-indeno[1,2-b]pyrrol-1-yl)-1-methyl-ethylamin fumarat (1:0.5) mit Smp. 187-188°.

Beispiel **37**

a) Eine Lösung von 11.2 g (64.1 mmol) 6-iso-Propyl-1-indanon, 13.3 ml (0.15 mol) 3-Buten-2-ol und 110 mg p-Toluolsulfonsäure in 110 ml 2,2-Dimethoxy-propan wurde 89 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt neben 6.6 g Edukt 5.6 g (38%) (RS)-2-(2-Buten-1-yl)-6-iso-propyl-1-indanon als gelbes Öl.

b) Durch eine auf -70° gekühlte Lösung von 5.6 g (24.5 mmol) (RS)-2-(2-Buten-1-yl)-6-iso-propyl-1-indanon in 125 ml wasserfreiem Dichlormethan und 25 ml wasserfreiem Methanol leitete man unter Rühren während 50 Minuten einen Ozonstrom (2 g Ozon/Stunde). Anschliessend spülte man die Lösung während 5 Minuten mit Sauerstoff und während 10 Minuten mit Argon. Nach Zugabe von 2.7 ml (36.8 mmol) Dimethylsulfid rührte man 15 Stunden bei Raumtemperatur. Das Reaktionsgemisch wurde im Vakuum eingedampft, der Rückstand mit 60 ml Dichlormethan versetzt und nach Zugabe von 10 ml Wasser und 10 ml Trifluoressigsäure 3 Stunden bei Raumtemperatur gerührt. Man goss das Gemisch anschliessend auf 50 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 200 ml Dichlormethan extrahiert. Die vereinigten organischen Phasen wurden

über Magnesiumsulfat getrocknet und im Vakuum eingeengt. Man erhielt 5.08 g (95%) (RS)-2-(2-Oxoethyl)-6-iso-propyl-1-indanon als gelbes Öl.

c) Eine Lösung von 2.16 g (10 mmol) (RS)-2-(2-Oxoethyl)-6-iso-proyl-1-indanon und 80 mg p-Toluolsulfonsäure in 60 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.4 g (55%) (RS)-1-(7-iso-Propyl-1,4-dihydro-indeno[1,2-b]pyrrol-1-yl)-propan-2-ol als braunes Öl.

d) Zu einer auf 0° gekühlten Lösung von 1.38 g (5.4 mmol) (RS)-1-(7-iso-Propyl-1,4-dihydro-indeno[1,2-b]pyrrol-1-yl)-propan-2-ol und 3.01 ml (21.6 mmol) Triethylamin in 50 ml Dichlormethan gab man tropfenweise unter Rühren 0.84 ml (10.8 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.7 g (10.8 mmol) Natriumazid versetzt und das Reaktionsgemisch unter Rühren 17 Stunden auf 60° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.08 g (72%) (RS)-1-(2-Azido-propyl)-7-iso-propyl-1,4-dihydro-indeno[1,2-b]pyrrol als schwach gelbes Öl.

e) 1.06 g (3.78 mmol) (RS)-1-(2-Azido-propyl)-7-iso-propyl-1,4-dihydro-indeno[1,2-b]-pyrrol gelöst in 50 ml wasserfreiem Ethanol wurden an 110 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 219 mg (1.89 mmol) Fumarsäure in 15 ml Methanol versetzt. Man rührte 15 Stunden bei Raumtemperatur und filtrierte anschliessend die weissen Kristalle ab. Man erhielt 918 mg (78%) (RS)-2-(7-iso-Propyl-1,4-dihydro-indeno[1,2-b]pyrrol-1-yl)-1-methyl-ethylamin fumarat (1:0.5) mit Smp. 203°.

Beispiel **38**

a) Eine Lösung von 2.16 g (10 mmol) (RS)-2-(2-Oxoethyl)-6-iso-propyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.78 g (70%) (R)-1-(7-iso-Propyl-1,4-dihydro-indeno[1,2-b]pyrrol-1-yl)-propan-2-ol als braunes Öl.

b) Zu einer auf 0° gekühlten Lösung von 1.78 g (6.97 mmol) (R)-1-(7-iso-Propyl-1,4-dihydro-indeno[1,2-b]pyrrol-1-yl)-propan-2-ol und 3.86 ml (27.9 mmol) Triethylamin in 50 ml Dichlormethan gab man tropfenweise unter Rühren 1.08 ml (13.9 mmol) Methansulfonylchlorid und rührte weitere 1.5 Stunden bei dieser Temperatur. Das Reaktionsgemisch wurde anschliessend mit 70 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.91 g (13.9 mmol) Natriumazid versetzt und das Reaktionsgemisch unter Rühren 16 Stunden auf 60° erwärmt. Nach dem Abkühlen goss man die Lösung auf 150 ml Wasser und extrahierte zweimal mit jeweils 200 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.12 g (57%) (S)-1-(2-Azido-propyl)-7-iso-propyl-1,4-dihydro-indeno[1,2-b]pyrrol als farbloses Öl.

c) 1.12 g (3.99 mmol) (S)-1-(2-Azido-propyl)-7-iso-propyl-1,4-dihydro-indeno-[1,2-b]-pyrrol gelöst in 100 ml wasserfreiem Ethanol wurden an 112 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 232 mg (2.0 mmol) Fumarsäure in 10 ml Methanol versetzt. Man rührte 5 Stunden bei Raumtemperatur und filtrierte anschliessend die weissen Kristalle ab. Man erhielt 371 mg (29%) (S)-2-(7-iso-Propyl-1,4-dihydro-indeno[1,2-b]pyrrol-1-yl)-1-methyl-ethylamin fumarat (1:0.57) mit Smp. 179-181°.

Beispiel **39**

a) Eine Lösung von 11.0 g (58.0 mmol) 6-tert-Butyl-1-indanon, 12.5 ml (145 mmol) 3-Buten-2-ol und 110 mg p-Toluolsulfonsäure in 110 ml 2,2-Dimethoxy-propan wurde 41 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 6:1). Man erhielt 7.35 g (53%) (RS)-2-(2-Buten-1-yl)-6-tert-butyl-1-indanon als gelbes Öl.

b) Durch eine auf -70° gekühlte Lösung von 7.35 g (30.5 mmol) (RS)-2-(2-Buten-1-yl)-6-tert-butyl-1-indanon in 150 ml wasserfreiem Dichlormethan und 30 ml wasserfreiem Methanol leitete man unter Rühren während 35 Minuten einen Ozonstrom (3 g Ozon/Stunde). Anschliessend spülte man die Lösung während 5 Minuten mit Sauerstoff und während 10 Minuten mit Argon. Nach Zugabe von 3.36 ml (45.8 mmol) Dimethylsulfid rührte man 17 Stunden bei Raumtemperatur. Das Reaktionsgemisch wurde im Vakuum eingedampft, der Rückstand mit 100 ml Dichlormethan versetzt und nach Zugabe von 15 ml Wasser und 15 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 6.44 g (92%) (RS)-2-(2-Oxoethyl)-6-tert-butyl-1-indanon als gelbes Öl.

c) Eine Lösung von 2.3 g (10 mmol) (RS)-2-(2-Oxoethyl)-6-tert-butyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.9 g (70%) (R)-1-(7-tert-Butyl-1,4-dihydro-indeno[1,2-b]pyrrol-1-yl)-propan-2-ol als braunes Öl.

d) Zu einer auf 0° gekühlten Lösung von 1.9 g (7.05 mmol) (R)-1-(7-tert-Butyl-1,4-dihydro-indeno[1,2-b]pyrrol-1-yl)-propan-2-ol und 3.9 ml (28.2 mmol) Triethylamin in 55 ml Dichlormethan gab man tropfenweise unter Rühren 1.1 ml (14.1 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.83 g (12.6 mmol) Natriumazid versetzt und das Reaktionsgemisch unter Rühren 16 Stunden auf 60° erwärmt. Nach dem Abkühlen goss man die Lösung auf 120 ml Wasser und extrahierte zweimal mit jeweils 120 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.82 g (44%) (S)-1-(2-Azido-propyl)-7-tert-butyl-1,4-dihydro-indeno[1,2-b]pyrrol als schwach braunes Öl.

e) 0.82 g (2.8 mmol) (S)-1-(2-Azido-propyl)-7-tert-butyl-1,4-dihydro-indeno-[1,2-b]pyrrol gelöst in 30 ml wasserfreiem Ethanol wurden an 80 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 163 mg (1.4 mmol) Fumarsäure in 10 ml Methanol versetzt. Man rührte 15 Stunden bei Raumtemperatur und filtrierte anschliessend die schwach rosafarbenen Kristalle ab. Man erhielt 0.33 g (36%) (S)-2-(7-tert-Butyl-1,4-dihydro-indeno[1,2-b]pyrrol-1-yl)-1-methyl-ethylamin fumarat (1:0.5) mit Smp. 188-190°.

Beispiel **40**

a) Eine Lösung von 9.73 g (45 mmol) 5'-Methoxy-2',3'-dihydro-spiro[cyclopentan-1,1'-[1H]inden]-3'-on, 9.3 ml (108 mmol) 3-Buten-2-ol und 100 mg p-Toluolsulfonsäure in 100 ml 2,2-Dimethoxy-propan wurde 90 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 7:1). Man erhielt neben 4.0 g Edukt 6.24 g (51%) (RS)-2'-(2-Buten-1-yl)-5'-methoxy-2',3'-dihydro-spiro[cyclopentan-1,1'-[1H]inden]-3'-on als gelbes Öl.

b) Durch eine auf -70° gekühlte Lösung von 6.2 g (22.9 mmol) (RS)-2'-(2-Buten-1-yl)-5'-methoxy-2',3'-dihydro-spiro[cyclopentan-1,1'-[1H]inden]-3'-on in 80 ml wasserfreiem Dichlormethan und 20 ml wasserfreiem Methanol

leitete man unter Rühren während 40 Minuten einen Ozonstrom (2.5 g Ozon/Stunde). Anschliessend spülte man die Lösung während 5 Minuten mit Sauerstoff und während 10 Minuten mit Argon. Nach Zugabe von 2.52 ml (34.4 mmol) Dimethylsulfid rührte man 17 Stunden bei Raumtemperatur. Das Reaktionsgemisch wurde im Vakuum eingedampft, der Rückstand mit 100 ml Dichlormethan versetzt und nach Zugabe von 15 ml Wasser und 15 ml Trifluoressigsäure 2.5 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 5.74 g (97%) (RS)-2'-(2-Oxoethyl)-5'-methoxy-2',3'-dihydro-spiro[cyclopentan-1,1'-[1H]inden]-3'-on als schwach gelbes Öl.

c) Eine Lösung von 2.58 g (10 mmol) (RS)-2'-(2-Oxoethyl)-5'-methoxy-2',3'-dihydro-spiro[cyclopentan-1,1'-[1H]inden]-3'-on und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 1:1) gereinigt. Man erhielt 1.98 g (67%) (R)-1-[7'-Methoxy-1',4'-dihydro-spiro[cyclopentan-1,4'-indeno[1,2-b]pyrrol]-1'-yl]-propan-2-ol als gelbes Öl.

d) Zu einer auf 0° gekühlten Lösung von 1.95 g (6.6 mmol) (R)-1-[7'-Methoxy-1',4'-dihydro-spiro[cyclopentan-1,4'-indeno[1,2-b]pyrrol]-1'-yl]-propan-2-ol und 3.65 ml (26.2 mmol) Triethylamin in 50 ml Dichlormethan gab man tropfenweise unter Rühren 1.02 ml (13.1 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene grüne Öl wurde in 50 ml wasserfreiem Dimethylformamid gelöst, mit 0.86 g (13.2 mmol) Natriumazid versetzt und das Reaktionsgemisch unter Rühren 5 Stunden auf 70° erwärmt. Nach dem Abkühlen goss man die Lösung auf 70 ml Wasser und extrahierte dreimal mit jeweils 100 ml Essigester. Die vereinigten organischen Phasen wusch man je einmal mit 70 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 1.45 g (68%) (S)-1'-(2-Azido-propyl)-7'-methoxy-1',4'-dihydro-spiro[cyclopentan]-1,4'-indeno[1,2-b]pyrrol als schwach gelbes Öl.

e) 1.45 g (4.5 mmol) (S)-1'-(2-Azido-propyl)-7'-methoxy-1',4'-dihydro-spiro[cyclopentan]-1,4'-indeno[1,2-b]pyrrol gelöst in 60 ml wasserfreiem Ethanol wurden an 145 mg Platinoxid 14 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 522 mg (4.5 mmol) Fumarsäure in 10 ml Methanol versetzt. Man rührte 5 Stunden bei Raumtemperatur und filtrierte anschliessend die weissen Kristalle ab. Man erhielt 1.47 g (79%) (S)-1-Methyl-2-(7'-methoxy-1',4'-dihydro-spiro[cyclopentan-1,4'-indeno[1,2-b]pyrrol]-1-yl)-ethylamin fumarat (1:1) mit Smp. 183-185°.

Beispiel **41**

a) Eine Lösung von 25.0 g (116 mmol) 5'-Methyl-2',3'-dihydro-spiro[cyclohexan-1,1'-[1H]inden]-3'-on, 24.1 ml (280 mmol) 3-Buten-2-ol und 250 mg p-Toluolsulfonsäure in 250 ml 2,2-Dimethoxy-propan wurde 88 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 6:1). Man erhielt neben 8.4 g Edukt 18.4 g (59%) (RS)-2'-(2-Buten-1-yl)-5'-methyl-2',3'-dihydro-spiro[cyclohexan-1,1'-[1H]inden]-3'-on als gelbes Öl.

b) Durch eine auf -70° gekühlte Lösung von 18.4 g (68.5 mmol) (RS)-2'-(2-Buten-1-yl)-5'-methyl-2',3'-dihydro-spiro[cyclohexan-1,1'-[1H]inden]-3'-on in 300 ml wasserfreiem Dichlormethan und 60 ml wasserfreiem Methanol leitete man unter Rühren während 75 Minuten einen Ozonstrom (3 g Ozon/Stunde). Anschliessend spülte man die Lösung während 5 Minuten mit Sauerstoff und während 10 Minuten mit Argon. Nach Zugabe von 7.55 ml (103 mmol) Dimethylsulfid rührte man 16 Stunden bei Raumtemperatur. Das Reaktionsgemisch wurde im Vakuum eingedampft, der Rückstand mit 200 ml Dichlormethan versetzt und nach Zugabe von 30 ml Wasser und 30 ml Trifluoressigsäure 3 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 17.0 g (97%) (RS)-2'-(2-Oxoethyl)-5'-methyl-2',3'-dihydro-spiro[cyclohexan-1,1'-[1H]inden]-3'-on

als gelbes Öl.

c) Eine Lösung von 2.56 g (10 mmol) (RS)-2'-(2-Oxoethyl)-5'-methyl-2',3'-dihydro-spiro[cyclohexan-1,1'-[1H]inden]-3'-on und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 2:3) gereinigt. Man erhielt 2.45 g (83%) (R)-1-[7'-Methyl-1',4'-dihydro-spiro[cyclohexan-1,4'-indeno[1,2-b]pyrrol]-1'-yl]-propan-2-ol als braunes Öl.

d) Zu einer auf 0° gekühlten Lösung von 2.45 g (8.3 mmol) (R)-1-[7'-Methyl-1',4'-dihydro-spiro[cyclohexan-1,4'-indeno[1,2-b]pyrrol]-1'-yl]-propan-2-ol und 4.57 ml (33.2 mmol) Triethylamin in 50 ml Dichlormethan gab man tropfenweise unter Rühren 1.3 ml (16.6 mmol) Methansulfonylchlorid und rührte weitere 2.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 75 ml wasserfreiem Dimethylformamid gelöst, mit 1.08 g (16.6 mmol) Natriumazid versetzt und das Reaktionsgemisch unter Rühren 16 Stunden auf 60° erwärmt. Nach dem Abkühlen goss man die Lösung auf 140 ml Wasser und extrahierte dreimal mit jeweils 100 ml Essigester. Die vereinigten organischen Phasen wusch man je einmal mit 90 ml Wasser und mit 90 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt (S)-1'-(2-Azido-propyl)-7'-methyl-1',4'-dihydro-spiro[cyclohexan]-1,4'-indeno[1,2-b]pyrrol 1.76 g (66%) als schwach rotes Öl.

e) 1.76 g (5.49 mmol) (S)-1'-(2-Azido-propyl)-7'-methyl-1',4'-dihydro-spiro[cyclohexan]-1,4'-indeno[1,2-b]pyrrol gelöst in 100 ml wasserfreiem Ethanol wurden an 170 mg Platinoxid 4 Stunden hydriert. Es wurde anschliessend vom Katalysator abfiltriert, mit Ethanol nachgewaschen und das Lösungsmittel im Vakuum abgezogen. Das erhaltene schwach braune Öl wurde in 100 ml wasserfreiem Diethylether gelöst, filtriert und unter Rühren mit einer Lösung von 637 mg (5.49 mmol) Fumarsäure in 10 ml Methanol versetzt. Man rührte 22 Stunden bei Raumtemperatur und filtrierte anschliessend die weissen Kristalle ab. Man erhielt 1.7 g (76%) (S)-1-Methyl-2-(7'-methyl-1',4'-dihydro-spiro[cyclohexan-1,4'-indeno[1,2-b]pyrrol]-1-yl)-ethylamin fumarat (1:1) mit Smp. 195-196°.

Beispiel **42** 44

a) Eine Lösung von 17.0 g (84.8 mmol) 5'-Methyl-2',3'-dihydro-spiro[cyclopentan-1,1'-[1H]inden]-3'-on, 17.5 ml (204 mmol) 3-Buten-2-ol und 170 mg p-Toluolsulfonsäure in 170 ml 2,2-Dimethoxy-propan wurde 71 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt neben 4.0 g Edukt 12.7 g (59%) (RS)-2'-(2-Buten-1-yl)-5'-methyl-2',3'-dihydro-spiro[cyclopentan-1,1'-[1H]inden]-3'-on als gelbes Öl.

b) Durch eine auf -70° gekühlte Lösung von 12.7 g (50.1 mmol) (RS)-2'-(2-Buten-1-yl)-5'-methyl-2',3'-dihydro-spiro[cyclopentan-1,1'-[1H]inden]-3'-on in 250 ml wasserfreiem Dichlormethan und 50 ml wasserfreiem Methanol leitete man unter Rühren während 60 Minuten einen Ozonstrom (3 g Ozon/Stunde). Anschliessend spülte man die Lösung während 5 Minuten mit Sauerstoff und während 10 Minuten mit Argon. Nach Zugabe von 5.54 ml (75.5 mmol) Dimethylsulfid rührte man 17 Stunden bei Raumtemperatur. Das Reaktionsgemisch wurde im Vakuum eingedampft, der Rückstand mit 160 ml Dichlormethan versetzt und nach Zugabe von 15 ml Wasser und 15 ml Trifluoressigsäure 2.5 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 12.0 g (99%) (RS)-2'-(2-Oxoethyl)-5'-methyl-2',3'-dihydro-spiro[cyclopentan-1,1'-[1H]inden]-3'-on als schwach rotes Öl.

c) Eine Lösung von 2.42 g (10 mmol) (RS)-2'-(2-Oxoethyl)-5'-methyl-2',3'-dihydro-spiro[cyclopentan-1,1'-[1H]inden]-3'-on und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 3:2) gereinigt. Man erhielt 2.06 g (73%) (R)-1-[7'-Methyl-1',4'-dihydro-spiro[cyclopentan-1,4'-indeno[1,2-b]pyrrol]-1'-yl]-propan-2-ol als rotes Öl.

d) Zu einer auf 0° gekühlten Lösung von 2.06 g (7.32 mmol) (R)-1-[7'-Methyl-1',4'-dihydro-spiro[cyclopentan-1,4'-indeno[1,2-b]pyrrol]-1'-yl]-propan-2-ol und 4.03 ml (14.6 mmol) Triethylamin in 50 ml Dichlormethan gab man tropfenweise unter Rühren 1.15 ml (14.6 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene grüne Öl wurde in 75 ml wasserfreiem Dimethylformamid gelöst, mit 0.95 g (14.6 mmol) Natriumazid versetzt und das Reaktionsgemisch unter Rühren 5 Stunden auf 70° erwärmt. Nach dem Abkühlen goss man die Lösung auf 100 ml Wasser und extrahierte dreimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Hexan/Essigester 4:1) gereinigt. Man erhielt 1.34 g (60%) (S)-1'-(2-Azido-propyl)-7'-methyl-1',4'-dihydro-spiro[cyclopentan]-1,4'-indeno[1,2-b]pyrrol als schwach rotes Öl.

e) 1.34 g (4.37 mmol) (S)-1'-(2-Azido-propyl)-7'-methyl-1',4'-dihydro-spiro[cyclopentan]-1,4'-indeno[1,2-b]pyrrol gelöst in 75 ml wasserfreiem Ethanol wurden an 135 mg Platinoxid 4 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 507 mg (4.37 mmol) Fumarsäure in 10 ml Methanol versetzt. Man rührte 15 Stunden bei Raumtemperatur und filtrierte anschliessend die weissen Kristalle ab. Man erhielt 1.23 g (71%) (S)-1-Methyl-2-(7'-methyl-1',4'-dihydro-spiro[cyclopentan-1,4'-indeno[1,2-b]pyrrol]-1-yl)-ethylamin fumarat (1:1) mit Smp. 192°.

Beispiel **43**

a) Eine Lösung von 1.05 g (4.13 mmol) (RS)-1-(2-Azido-propyl)-7-hydroxy-1,4-dihydro-indeno[1,2-b]pyrrol und 0.77 ml (8.26 mmmol) Isopropylbromidbromid, 1.14 g (8.26 mmol) Kaliumcarbonat in 30 ml N,N-Dimethylformamid wurde 48 Stunden auf 50° erhitzt. Nach dem Abkühlen goss man die Lösung auf 150 ml Wasser und extrahierte zweimal mit jeweils 150 ml Essigester. Die vereinigten organischen Phasen wusch man einmal mit 70 ml halbgesättigter Natriumchloridlösung, trocknete über Magnesiumsulfat und engte die Lösung im Vakuum ein. Das Rohprodukt wurde durch Säulenchromatographie an Kieselgel (Toluol) gereinigt. Man erhielt 0.35 g (28%) (RS)-1-(2-Azido-propyl)-7-iso-propoxy-1,4-dihydro-indeno[1,2-b]pyrrol als oranges Öl, welches direkt in die nächste Reaktion eingesetzt wurde.

b) 0.35 g (1.17 mmol) (RS)-1-(2-Azido-propyl)-7-iso-propoxy-1,4-dihydro-indeno[1,2-b]-pyrrol gelöst in 40 ml wasserfreiem Ethanol wurden an 40 mg Platinoxid 4 Stunden hydriert. Es wurde anschliessend vom Katalysator abfiltriert, mit Ethanol nachgewaschen und das Lösungsmittel im Vakuum abgezogen. Das erhaltene firblose Öl wurde in 70 ml wasserfreiem Diethylether gelöst, filtriert und unter Rühren mit einer Lösung von 68 mg (0.58 mmol) Fumarsäure in 10 ml Methanol versetzt. Man rührte 17 Stunden bei Raumtemperatur und filtrierte anschliessend die weissen Kristalle ab. Man erhielt 0.22 mg (57%) (RS)-2-(7-Iso-propoxy-1,4-dihydro-indeno[1,2-b]pyrrol-1-yl)-1-methyl-ethylamin fumarat (1:0.5) mit Smp. 192°.

Beispiel **44**

a) Eine Lösung von 8.0 g (54 mmol) 6-Hydroxy-1-indanon, 6.3 ml (59.4 mmmol) Cyclopentylbromid, 16.4 g (119 mmol) Kaliumcarbonat und 10 ml N,N-Dimethylformamid in 100 ml Aceton wurde 35 Stunden auf 75° erhitzt. Nach dem Abkühlen goss man die Lösung auf 150 ml Wasser und extrahierte zweimal mit jeweils 200 ml Essigester. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das Rohprodukt wurde durch Säulenchromatographie an Kieselgel (Hexan/Essigester 3:1) gereinigt. Man erhielt 9.45 g (81%) 6-Cyclopentoxy-1-indanon als oranges Öl, welches direkt in die nächste Reaktion eingesetzt wurde.

b) Eine Lösung von 9.45 g (43.7 mmol) 6-Cyclopentoxy-1-indanon , 9.0 ml (105 mmol) 3-Buten-2-ol und 100 mg p-Toluolsulfonsäure in 100 ml 2,2-Dimethoxy-propan wurde 63 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 6:1). Man erhielt 8.1 g (69%) (RS)-2-(2-Buten-1-yl)-6-cyclopentoxy-1-indanon als gelbes Öl.

c) Durch eine auf -70° gekühlte Lösung von 8.1 g (29.9 mmol) (RS)-2-(2-Buten-1-yl)-6-cyclopentoxy-1-indanon in 150 ml wasserfreiem Dichlormethan und 30 ml wasserfreiem Methanol leitete man unter Rühren während 60 Minuten einen Ozonstrom (1.5 g Ozon/Stunde). Anschliessend spülte man die Lösung während 5 Minuten mit Sauerstoff und während 10 Minuten mit Argon. Nach Zugabe von 3.29 ml (44.9 mmol) Dimethylsulfid rührte man 21 Stunden bei Raumtemperatur. Das Reaktionsgemisch wurde im Vakuum eingedampft, der Rückstand mit 75 ml

Dichlormethan versetzt und nach Zugabe von 12.5 ml Wasser und 12.5 ml Trifluoressigsäure 5 Stunden bei Raumtemperatur gerührt. Man goss das Gemisch anschliessend auf 150 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 5.63 g (73%) (RS)-2-(2-Oxoethyl)-6-cyclopentoxy-1-indanon als oranges Öl.

d) Eine Lösung von 2.58 g (10 mmol) (RS)-2-(2-Oxoethyl)-6-cyclopentoxy-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.44 g (48%) (RS)-1-(7-Cyclopentoxy-1,4-dihydro-indeno[1,2-b]pyrrol-1-yl)-propan-2-ol als schwach gelbes Öl.

e) Zu einer auf 0° gekühlten Lösung von 1.44 g (4.8 mmol) (RS)-1-(7-Cyclopentoxy-1,4-dihydro-indeno[1,2-b]pyrrol-1-yl)-propan-2-ol und 2.66 ml (19.3 mmol) Triethylamin in 55 ml Dichlormethan gab man tropfenweise unter Rühren 0.75 ml (9.6 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene grüne Öl wurde in 30 ml wasserfreiem Dimethylformamid gelöst, mit 0.62 g (9.6 mmol) Natriumazid versetzt und das Reaktionsgemisch unter Rühren 16 Stunden auf 60° erwärmt. Nach dem Abkühlen goss man die Lösung auf 100 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Hexan 1:4) gereinigt. Man erhielt 1.27 g (82%) (RS)-1-(2-Azido-propyl)-7-cyclopentoxy-1,4-dihydro-indeno[1,2-b]pyrrol als schwach gelblichen Feststoff, der direkt in die nächste Reaktion eingesetzt wurde..

f) 1.27 g (3.93 mmol) (RS)-1-(2-Azido-propyl)-7-cyclopentoxy-1,4-dihydro-indeno[1,2-b]-pyrrol gelöst in 75 ml wasserfreiem Ethanol wurden an 125 mg Platinoxid 16 Stunden hydriert. Es wurde anschliessend vom Katalysator abfiltriert, mit Ethanol nachgewaschen und das Lösungsmittel im Vakuum abgezogen. Das erhaltene schwach rote Öl wurde in 80 ml wasserfreiem Diethylether gelöst, filtriert und unter Rühren mit einer Lösung von 198 mg (1.7 mmol) Fumarsäure in 20 ml Methanol versetzt. Man rührte 17 Stunden bei Raumtemperatur und filtrierte anschliessend die leicht rosafarbenen Kristalle ab. Man erhielt 926 mg (80%) (RS)-2-(7-Cyclopenttoxy-1,4-dihydro-indeno[1,2-b]pyrrol-1-yl)-1-methyl-ethylamin fumarat (1:0.5) mit Smp. 196-198°.

Beispiel **45**

a) Zu einer auf 0° gekühlten Lösung von 12.0 g (81 mmol) 6-Hydroxy-1-indanon und 45.2 ml (162 mmol) Triethylamin in 350 ml Dichlormethan gab man tropfenweise unter Rühren 12.6 ml (162 mmol) Methansulfonylchlorid und rührte weitere 1.5 Stunden bei dieser Temperatur. Das Reaktionsgemisch wurde anschliessend mit 200 ml Dichlormethan verdünnt, zweimal mit jeweils 150 ml gesättigter Natriumhydrogencarbonatlösung gewaschen und die vereinigten Wasserphasen einmal mit 100 ml Dichlormethan extrahiert. Die vereinigten organischen Phasen wurden mit 200 ml gesättigter Natriumchloridlösung gewaschen, über Magnesiumsulfat getrocknet und im Vakuum eingedampft. Man erhielt 18.3 g (99%) 6-Mesyloxy-1-indanon als braunen Feststoff, der direkt in die nächste Reaktion eingesetzt wurde.

b) Eine Lösung von 18.3 g (80.9 mmol) 6-Mesyloxy-1-indanon, 16.7 ml (194 mmol) 3-Buten-2-ol und 300 mg p-Toluolsulfonsäure in 400 ml 2,2-Dimethoxy-propan wurde 46 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Essigester 4:1). Man erhielt neben 8.31 g Edukt 11.3 g (50%) (RS)-2-(2-Buten-1-yl)-6-mesyloxy-1-indanon als gelbes Öl.

c) Durch eine auf -70° gekühlte Lösung von 11.3 g (40.3 mmol) (RS)-2-(2-Buten-1-yl)-6-mesyloxy-1-indanon in 300 ml wasserfreiem Dichlormethan und 60 ml wasserfreiem Methanol leitete man unter Rühren während 55 Minuten einen Ozonstrom (2 g Ozon/Stunde). Anschliessend spülte man die Lösung während 5 Minuten mit Sauerstoff und während 10 Minuten mit Argon. Nach Zugabe von 4.51 ml (61.5 mmol) Dimethylsulfid rührte man 15 Stunden bei Raumtemperatur. Das Reaktionsgemisch wurde im Vakuum eingedampft, der Rückstand mit 250 ml Dichlormethan versetzt und nach Zugabe von 25 ml Wasser und 25 ml Trifluoressigsäure 4 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 50 ml Wasser zugegeben, die Phasen getrennt

und die Wasserphase zweimal mit jeweils 200 ml Dichlormethan extrahiert. Die vereinigten organischen Phasen wurden über Magnesiumsulfat getrocknet und im Vakuum eingeengt. Man erhielt 9.38 g (85%) (RS)-2-(2-Oxoethyl)-6-mesyloxy-1-indanon als schwach braunen Feststoff mit Smp. 85-87°.

d) Eine Lösung von 2.31 g (8.59 mmol) (RS)-2-(2-Oxoethyl)-6-mesyloxy-1-indanon und 110 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.58 g (34.4 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Essigester/Hexan 2:3) gereinigt. Man erhielt 0.74 g (28%) (RS)-1-(7-Mesyloxy-1,4-dihydro-indeno[1,2-b]pyrrol-1-yl)-propan-2-ol als schwach braunen Feststoff, der direkt in die nächste Reaktion eingesetzt wurde.

e) Zu einer auf 0° gekühlten Lösung von 0.72 g (2.38 mmol) (RS)-1-(7-Mesyloxy-1,4-dihydro-indeno[1,2-b]pyrrol-1-yl)-propan-2-ol und 1.33 ml (9.53 mmol) Triethylamin in 30 ml Dichlormethan gab man tropfenweise unter Rühren 0.37 ml (4.77 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene grüne Öl wurde in 30 ml wasserfreiem Dimethylformamid gelöst, mit 0.62 g (4.77 mmol) Natriumazid versetzt und das Reaktionsgemisch unter Rühren 16 Stunden auf 60° erwärmt. Nach dem Abkühlen goss man die Lösung auf 70 ml Wasser und extrahierte zweimal mit jeweils 70 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Hexan 1:4) gereinigt. Man erhielt 0.68 g (86%) (RS)-1-(2-Azido-propyl)-7-mesyloxy-1,4-dihydro-indeno[1,2-b]pyrrol als schwach gelblichen Feststoff, der direkt in die nächste Reaktion eingesetzt wurde.

f) 0.66 g (2.0 mmol) (RS)-1-(2-Azido-propyl)-7-mesyloxy-1,4-dihydro-indeno-[1,2-b]-pyrrol gelöst in 30 ml wasserfreiem Ethanol wurden an 66 mg Platinoxid 4 Stunden hydriert. Es wurde anschliessend vom Katalysator abfiltriert, mit Ethanol nachgewaschen und das Lösungsmittel im Vakuum abgezogen. Das erhaltene schwach braune Öl wurde in 50 ml wasserfreiem Diethylether gelöst, filtriert und unter Rühren mit einer Lösung von 116 mg (1.0 mmol) Fumarsäure in 10 ml Methanol versetzt. Man rührte 17 Stunden bei Raumtemperatur und filtrierte anschliessend die leicht rosafarbenen Kristalle ab. Man erhielt 400 mg (55%) (RS)-2-(7-Mesyloxy-1,4-dihydro-indeno[1,2-b]pyrrol-1-yl)-1-methyl-ethylamin fumarat (1:0.5) mit Smp. 201°.

Beispiel **46**

a) Zu einer auf -70° gekühlten Lösung von 3.24 g (20.0 mmol) 5-Methoxy-1-indanon in 350 ml wasserfreiem Tetrahydrofuran gab man unter Rühren tropfenweise eine aus 4.25 ml (30.0 mmol) Diisopropylamin und 18.7 ml (30 mmol) 1.6 N n-Butyllithium in Hexan frisch hergestellte Lithiumdiisopropylamid-lösung in 60 ml wasserfreiem Tetrahydrofuran. Man rührte weitere 45 Minuten bei dieser Temperatur und tropfte anschliessend über 15 Minuten eine Lösung von 1.6 ml (20.0 mmol) Chloraceton in 60 ml wasserfreiem Tetrahydrofuran gelöst hinzu. Man liess das Reaktionsgemisch über 100 Minuten auf Raumtemperatur kommen und rührte weitere 45 Minuten bei dieser Temperatur. Anschliessend goss man das Reaktionsgemisch auf 150 ml Eis, gab 150 ml gesättigte Natriumchloridlösung dazu und trennte die organische Phase ab. Die Wasserphase wurde einmal mit 400 ml Diethylether extrahiert, die vereinigten organischen Phasen einmal mit 100 ml gesättigter Natriumchloridlösung gewaschen, über Magnesiumsulfat getrocknet und im Vakuum eingeengt. Das erhaltene rote Öl wurde durch Säulenchromatographie an Kieselgel (Hexan/Diethylether 3:7) gereinigt. Man erhielt 1.67 g Rohprodukt, welches aus Diethylether/Hexan umkristallisiert wurde. Die Kristallisation ergab 1.21 g (56%) (RS)-5-Methoxy-2-(2-oxopropyl)-1-indanon als schwach gelben Feststoff mit Smp. 73°.

b) Eine Lösung von 1.2 g (5.5 mmol) (RS)-5-Methoxy-2-(2-oxopropyl)-1-indanon und 60 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 1.65 g (22.0 mmol) (RS)-1-Amino-2-propanol in 20 ml wasserfreiem Toluol. Anschliessend wurde weitere 90 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 1.17 g (82%) (RS)-1-(6-Methoxy-2-methyl-1,4-dihydro-indeno[1,2-b]pyrrol-1-yl)-propan-2-ol als gelben Feststoff, der ohne weitere Umkristallisation in die nächste Reaktion eingesetzt wurde.

c) Zu einer auf 0° gekühlten Lösung von 1.16 g (4.51 mmol) (RS)-1-(6-Methoxy-2-methyl-1,4-dihydro-indeno[1,2-b]pyrrol-1-yl)-propan-2-ol und 2.5 ml (18.0 mmol) Triethylamin in 50 ml Dichlormethan gab man tropfenweise unter Rühren 0.7 ml (9.0 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 60 ml gesättigter Natriumhydrogencarbonatlösung gewaschen und die vereinigten Wasserphasen einmal mit 60 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.58 g (9.0 mmol) Natriumazid versetzt und das Reaktionsgemisch unter Rühren 16 Stunden auf 80° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.86 g (68%) (RS)-1-(2-Azido-propyl)-6-methoxy-2-methyl-1,4-dihydro-indeno[1,2-b]pyrrol als gelbes Öl.

d) 0.85 g (3.01 mmol) (RS)-1-(2-Azido-propyl)-6-methoxy-2-methyl-1,4-dihydro-indeno-[1,2-b]-pyrrol gelöst in 50 ml wasserfreiem Ethanol wurden an 85 mg Platinoxid 17 Stunden hydriert. Es wurde anschliessend vom Katalysator abfiltriert, mit Ethanol nachgewaschen und das Lösungsmittel im Vakuum abgezogen. Das erhaltene schwach gelbe Öl wurde in 70 ml wasserfreiem Diethylether gelöst, filtriert und unter Rühren mit einer Lösung von 175 mg (1.51 mmol) Fumarsäure in 15 ml Methanol versetzt. Man rührte 19 Stunden bei Raumtemperatur und filtrierte anschliessend die leicht weissen Kristalle ab. Man erhielt 779 mg (82%) (RS)-2-(6-Methoxy-2-methyl-1,4-dihydro-indeno[1,2-b]pyrrol-1-yl)-1-methyl-ethylamin fumarat (1:0.5) mit Smp. 218°.

<u>Beispiel **47**</u>

a) Eine Lösung von 12.3 g (61.2 mmol) 5,6-Dichlor-1-indanon, 12.6 ml (0.15 mol) 3-Buten-2-ol und 125 mg p-Toluolsulfonsäure in 125 ml 2,2-Dimethoxy-propan wurde 68 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt neben 4.3 g Edukt 10.8 g (69%) (RS)-2-(2-Buten-1-yl)-5,6-dichlor-1-indanon als gelbes Öl.

b) Durch eine auf -70° gekühlte Lösung von 10.8 g (42.4 mmol) (RS)-2-(2-Buten-1-yl)-5,6-dichlor-1-indanon in 150 ml wasserfreiem Dichlormethan und 30 ml wasserfreiem Methanol leitete man unter Rühren während 45 Minuten einen Ozonstrom (3.5 g Ozon/Stunde). Anschliessend spülte man die Lösung während 5 Minuten mit Sauerstoff und während 10 Minuten mit Argon. Nach Zugabe von 4.66 ml (63.6 mmol) Dimethylsulfid rührte man 3 Stunden bei Raumtemperatur. Das Reaktionsgemisch wurde im Vakuum eingedampft, der Rückstand mit 150 ml Dichlormethan versetzt und nach Zugabe von 20 ml Wasser und 20 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet und im Vakuum eingeengt. Man erhielt 11.6 g Rohprodukt, welches aus Hexan Essigester kristallisiert wurde. Die Kristallisation ergab 7.59 g (73%) (RS)-2-(2-Oxoethyl)-5,6-dichlor-1-indanon als schwach gelben Feststoff mit Smp. 93-96°.

c) Eine Lösung von 2.0 g (8.23 mmol) (RS)-2-(2-Oxoethyl)-5,6-dichlor-1-indanon und 70 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.47 g (32.9 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 4:1) gereinigt. Man erhielt 0.62 g (27%) (RS)-1-(6,7-Dichlor-1,4-dihydro-indeno[1,2-b]pyrrol-1-yl)-propan-2-ol als braunes Öl.

d) Zu einer auf 0° gekühlten Lösung von 0.6 g (2.12 mmol) (RS)-1-(6,7-Dichlor-1,4-dihydro-indeno[1,2-b]pyrrol-1-yl)-propan-2-ol und 1.18 ml (8.5 mmol) Triethylamin in 30 ml Dichlormethan gab man tropfenweise unter Rühren 0.33 ml (4.25 mmol) Methansulfonylchlorid und rührte weitere 1.5 Stunden bei dieser Temperatur. Das Reaktionsgemisch wurde anschliessend mit 50 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 275 mg (4.24 mmol) Natriumazid versetzt und das Reaktionsgemisch unter Rühren 17 Stunden auf 60° erwärmt. Nach dem Abkühlen goss man die Lösung auf 60 ml Wasser und extrahierte dreimal mit jeweils 90 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.45 g (69%) (RS)-1-(2-Azido-propyl)-6,7-dichlor-1,4-dihydro-indeno[1,2-b]pyrrol als schwach farbloses Öl.

e) 0.44 g (1.43 mmol) (RS)-1-(2-Azido-propyl)-6,7-dichlor-1,4-dihydro-indeno-[1,2-b]-pyrrol gelöst in 30 ml wasserfreiem Ethanol wurden an 45 mg Platinoxid 3 Stunden hydriert. Es wurde anschliessend vom Katalysator abfiltriert,

mit Ethanol nachgewaschen und das Lösungsmittel im Vakuum abgezogen. Das erhaltene farblose Öl wurde in 30 ml wasserfreiem Diethylether gelöst, filtriert und unter Rühren mit einer Lösung von 83 mg (0.72 mmol) Fumarsäure in 5 ml Methanol versetzt. Man rührte 17 Stunden bei Raumtemperatur und filtrierte anschliessend die weissen Kristalle ab. Man erhielt 395 mg (81%) (RS)-2-(6,7-Dichlor-1,4-dihydro-indeno[1,2-b]pyrrol-1-yl)-1-methylethylamin fumarat (1:0.5) mit Smp. 203°.

Beispiel **48**

a) Eine Lösung von 13.0 g (89 mmol) 4-Methyl-1-indanon, 19.2 ml (0.22 mol) 3-Buten-2-ol und 170 mg p-Toluolsulfonsäure in 170 ml 2,2-Dimethoxy-propan wurde 46 Stunden an einem mit Molekularsieb (0.4 nm, 2 mm Perlform) gefüllten Wasserabscheider unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt 12.0 g (67%) (RS)-2-(2-Buten-1-yl)-4-methyl-1-indanon als gelbes Öl.

b) Durch eine auf -70° gekühlte Lösung von 12.0 g (60 mmol) (RS)-2-(2-Buten-1-yl)-4-methyl-1-indanon in 220 ml wasserfreiem Dichlormethan und 45 ml wasserfreiem Methanol leitete man unter Rühren während 90 Minuten einen Ozonstrom (3 g Ozon/Stunde). Anschliessend spülte man die Lösung während 5 Minuten mit Sauerstoff und während 10 Minuten mit Argon. Nach Zugabe von 6.6 ml (90 mmol) Dimethylsulfid rührte man 15 Stunden bei Raumtemperatur. Das Reaktionsgemisch wurde im Vakuum eingedampft, der Rückstand mit 160 ml Dichlormethan versetzt und nach Zugabe von 20 ml Wasser und 20 ml Trifluoressigsäure 2 Stunden bei Raumtemperatur gerührt. Man goss das Gemisch anschliessend auf 200 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 200 ml Dichlormethan extrahiert. Die vereinigten organischen Phasen wurden über Magnesiumsulfat getrocknet und im Vakuum eingeengt. Man erhielt 10.6 g (94%) (RS)-2-(2-Oxoethyl)-4-methyl-1-indanon als schwach gelbes Öl.

c) Eine Lösung von 1.9 g (10.0 mmol) (RS)-2-(2-Oxoethyl)-4-methyl-1-indanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 45 Minuten gekocht, wobei man das Lösungsmittel auf ein Volumen von 20 ml reduzierte. Das erkaltete Reaktionsgemisch wurde durch Säulenchromatographie an Kieselgel (Diethylether/Hexan 7:3) gereinigt. Man erhielt 0.63 g (28%) (RS)-1-(5-Methyl-1,4-dihydro-indeno[1,2-b]pyrrol-1-yl)-propan-2-ol als weissen Feststoff, der direkt in die nächste Reaktion eingesetzt wurde.

d) Zu einer auf 0° gekühlten Lösung von 0.63 g (2.8 mmol) (RS)-1-(5-Methyl-1,4-dihydro-indeno[1,2-b]pyrrol-1-yl)-propan-2-ol und 1.6 ml (11.2 mmol) Triethylamin in 25 ml Dichlormethan gab man tropfenweise unter Rühren 0.44 ml (5.6 mmol) Methansulfonylchlorid und rührte weitere 1.5 Stunden bei dieser Temperatur. Das Reaktionsgemisch wurde anschliessend mit 50 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Der erhaltene grüne Feststoff wurde in 30 ml wasserfreiem Dimethylformamid gelöst, mit 0.36 g (5.6 mmol) Natriumazid versetzt und das Reaktionsgemisch unter Rühren 16 Stunden auf 60° erwärmt. Nach dem Abkühlen goss man die Lösung auf 80 ml Wasser und extrahierte dreimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.69 g (98%) (RS)-1-(2-Azido-propyl)-5-methyl-1,4-dihydro-indeno[1,2-b]pyrrol als schwach gelbes Öl.

e) 0.69 g (2.7 mmol) (RS)-1-(2-Azido-propyl)-5-methyl-1,4-dihydro-indeno-[1,2-b]pyrrol gelöst in 30 ml wasserfreiem Ethanol wurden an 70 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 157 mg (1.35 mmol) Fumarsäure in 15 ml Methanol versetzt. Man rührte 18 Stunden bei Raumtemperatur und filtrierte anschliessend die weissen Kristalle ab. Man erhielt 0.34 g (44%) (RS)-2-(5-Methyl-1,4-dihydro-indeno[1,2-b]pyrrol-1-yl)-1-methylethylamin fumarat (1:0.5) mit Smp. 214°.

Beispiel **49**

a) Eine Lösung von 1.9 g (10 mmol) (RS)-2-(2-Oxoethyl)-4-chromanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (R)-1-Amino-2-propanol in 20 ml wasserfreiem Toluol. Anschliessend wurde weitere 35 Minuten gekocht, wobei man das Lösungsmittel auf ein Volumen von 20 ml reduzierte. Das erkaltete Reaktionsgemisch wurde durch Säulenchromato-graphie an Kieselgel (Essigester/Toluol 2:3) gereinigt. Man erhielt 1.9 g (83%) (R)-1-(1,4-Dihydro-[1]benzopyrano[4,3-b]pyrrol-1-yl)-propan-2-ol als gelbes Öl.

b) Zu einer auf 0° gekühlten Lösung von 1.88 g (8.2 mmol) (R)-1-(1,4-Dihydro-[1]benzopyrano[4,3-b]pyrrol-1-yl)-propan-2-ol und 4.57 ml (32.8 mmol) Triethylamin in 50 ml wasserfreiem Dichlormethan gab man tropfenweise unter Rühren 1.27 ml (16.4 mmol) Methansulfonylchlorid und rührte weitere 1.5 Stunden bei dieser Temperatur. Das Reaktionsgemisch wurde anschliessend mit 280 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 140 ml gesättigter Natrium-chloridlösung gewaschen, über Magnesiumsulfat getrocknet und im Vakuum eingedampft. Der erhaltene braune Feststoff wurde in 50 ml wasserfreiem Dimethylformamid gelöst, mit 1.0 g (15.2 mmol) Natriumazid versetzt und das Reaktionsgemisch unter Rühren 18 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.67 g (80%) (S)-1-(2-Azido-propyl)-1,4-dihydro-[1]benzopyrano[4,3-b]-pyrrol als farbloses Öl.

c) 1.65 g (6.5 mmol) (S)-1-(2-Azido-propyl)-1,4-dihydro-[1]benzopyrano[4,3-b]pyrrol gelöst in 60 ml wasserfreiem Ethanol wurden an 170 mg Platinoxid 4 Stunden hydriert. Es wurde anschliessend vom Katalysator abfiltriert, mit Ethanol nachgewaschen und das Lösungsmittel im Vakuum abgezogen. Das so erhaltene farblose Öl wurde in 100 ml wasserfreiem Diethylether gelöst, filtriert und unter Rühren mit einer Lösung von 309 mg (2.67 mmol) Fumarsäure in 20 ml Methanol versetzt. Man rührte 18 Stunden bei Raumtemperatur und filtrierte anschliessend die weissen Kristalle ab. Man erhielt 1.35 g (73%) (S)-1-(1,4-Dihydro-[1]benzopyrano[4,3-b]pyrrol-1-yl)-1-methyl-ethylamin fumarat (1:0.5) mit Smp. 194-195°.

Beispiel **50**

a) Eine Lösung von 25 g (0.15 mol) 5-Chlor-1-indanon, 31 ml (0.36 mol) 3-Buten-2-ol und 250 mg p-Toluolsulfonsäure in 31 ml 2,2-Dimethoxypropan und 250 ml wasserfreiem Toluol wurde 17 Stunden unter Rückfluss gekocht. Das Reaktionsgemisch wurde anschliessend im Vakuum eingeengt und durch Säulenchromatographie an Kieselgel gereinigt (Hexan/Diethylether 5:1). Man erhielt 11.9 g (36%) (RS)-2-(2-Buten-1-yl)-5-chlor-1-indanon als gelbes Öl.

b) Durch eine auf -70° gekühlte Lösung von 11.9 g (53.9 mmol) (RS)-2-(2-Buten-1-yl)-5-chlor-1-indanon in 200 ml wasserfreiem Dichlormethan und 100 ml wasserfreiem Methanol leitete man unter Rühren während 60 Minuten einen Ozonstrom (3 g Ozon/Stunde). Anschliessend spülte man die Lösung während 5 Minuten mit Sauerstoff und während 10 Minuten mit Argon. Nach Zugabe von 5.9 ml (80.9 mmol) Dimethylsulfid rührte man 16 Stunden bei Raumtemperatur. Das Reaktionsgemisch wurde im Vakuum eingedampft, der Rückstand mit 50 ml Dichlormethan versetzt und nach Zugabe von 12 ml Wasser und 12 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt aus Essigester/Hexan kristallisiert. Man erhielt 8.98 g (80%) (RS)-2-(2-Oxoethyl)-6-chlor-1-indanon als weissen Feststoff mit Smp. 66°.

c) Eine Lösung von 2 g (9.6 mmol) (RS)-2-(2-Oxoethyl)-5-chlor-1-indanon und 100 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 2.88 g (38.3 mmol) (RS)-1-Amino-2-propanol in 20 ml wasserfreiem Toluol. Anschliessend wurde weitere 35 Minuten gekocht, wobei man das Lösungsmittel auf ein Volumen von 30 ml reduzierte. Das erkaltete Reaktionsgemisch wurde durch Säulenchromatographie an Kieselgel (Essigester/Toluol 1:1) gereinigt. Man erhielt 1.9 g (80%) (RS)-1-(6-Chlor-1,4-dihydro-indeno[1,2-b]pyrrol-1-yl)-propan-2-ol als braunes Öl, welches direkt in die nächste Reaktion eingesetzt wurde.

d) Zu einer auf 0° gekühlten Lösung von 1.9 g (7.7 mmol) (RS)-1-(6-Chlor-1,4-dihydro-indeno[1,2-b]pyrrol-1-yl)-propan-2-ol und 4.3 ml (30.6 mmol) Triethylamin in 60 ml Dichlormethan gab man tropfenweise unter Rühren 1.2 ml (15.3 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 1.0 g (15.3 mmol) Natriumazid versetzt und das Reaktionsgemisch unter Rühren 17 Stunden auf 50° erwärmt. Nach dem Abkühlen goss man die Lösung auf 140 ml Wasser und extrahierte zweimal mit jeweils 140 ml Diethylether und einmal mit 140 ml Essigester. Die vereinigten organischen Phasen wusch man

je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.8 g (38%) (RS)-1-(2-Azido-propyl)-6-chlor-1,4-dihydro-indeno[1,2-b]pyrrol als gelbliches Öl.

e) 0.8 g (2.9 mmol) (RS)-1-(2-Azido-propyl)-6-chlor-1,4-dihydro-indeno[1,2-b]pyrrol gelöst in 80 ml wasserfreiem Ethanol wurden an 80 mg Platinoxid 3 Stunden hydriert. Es wurde anschliessend vom Katalysator abfiltriert, mit Ethanol nachgewaschen und das Lösungsmittel im Vakuum abgezogen. Das erhaltene farblose Öl wurde in 150 ml wasserfreiem Diethylether gelöst, filtriert und unter Rühren mit einer Lösung von 170 mg (1.46 mmol) Fumarsäure in 15 ml Methanol versetzt. Man rührte 4 Stunden bei Raumtempera-tur und filtrierte anschliessend die weissen Kristalle ab. Man erhielt 780 mg (87%) (RS)-2-(6-Chlor-1,4-dihy-dro-indeno[1,2-b]pyrrol-1-yl)-1-methyl-ethylamin fumarat (1:0.5) mit Smp. 212°.

Beispiel **51**

a) Eine Lösung von 11.9 g (66.7 mmol) 7-Methoxy-4-chromanon, 13.8 ml (0.16 mol) 3-Buten-2-ol und 120 mg p-Toluolsulfonsäure in 14 ml 2,2-Dimethoxypropan und 120 ml wasserfreiem Toluol wurde 24 Stunden unter Rückfluss gekocht. Das Reaktionsgemisch wurde anschliessend im Vakuum eingeengt und durch Säulenchromatographie an Kieselgel gereinigt (Hexan/Diethylether 4:1). Man erhielt 6.3 g (41%) (RS)-2-(2-Buten-1-yl)-7-methoxy-4-chromanon als gelbes Öl.

b) Durch eine auf -70° gekühlte Lösung von 6.25 g (26.9 mmol) (RS)-2-(2-Buten-1-yl)-7-methoxy-4-chromanon in 90 ml wasserfreiem Dichlormethan und 30 ml wasserfreiem Methanol leitete man unter Rühren während 1 Stunde einen Ozonstrom (3 g Ozon/Stunde). Anschliessend spülte man die Lösung während 5 Minuten mit Sauerstoff und während 15 Minuten mit Argon. Nach Zugabe von 3 ml (40.5 mmol) Dimethylsulfid rührte man 16 Stunden bei Raumtemperatur. Das Reaktionsgemisch wurde anschliessend im Vakuum eingedampft, der Rückstand mit 60 ml Dichlormethan versetzt und nach Zugabe von 15 ml Wasser und 15 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 70 ml Wasser zugegeben, die Phasen getrennt und die Wasserphase zweimal mit jeweils 100 ml Dichlormethan extrahiert. Die vereinigten organischen Phasen wurden über Magnesiumsulfat getrocknet im Vakuum eingeengt. Man erhielt 2.9 g (49%) 2-(2-Oxoethyl)-7-methoxy-4-chromanon als gelbes Öl.

c) Eine Lösung von 2.38 g (10.8 mmol) (RS)-2-(2-Oxoethyl)-7-methoxy-4-chromanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.25 g (43.2 mmol) (RS)-1-Amino-2-propanol in 20 ml wasserfreiem Toluol. Anschliessend wurde weitere 35 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 2:3) gereinigt. Man erhielt 1.95 g (70%) (RS)-1-(1,4-Dihydro-8-methoxy[1]-benzopyrano[4,3-b]pyrrol-1-yl)-propan-2-ol als braunes Öl.

d) Zu einer auf 0° gekühlten Lösung von 1.92 g (7.4 mmol) (RS)-1-(1,4-Dihydro-8-methoxy[1]benzopyrano[4,3-b]pyrrol-1-yl)-propan-2-ol und 4.13 ml (29.6 mmol) Triethylamin in 50 ml wasserfreiem Dichlormethan gab man tropfenweise unter Rühren 1.15 ml (14.8 mmol) Methansulfonylchlorid und rührte weitere 1.5 Stunden bei dieser Temperatur. Das Reaktionsgemisch wurde anschliessend mit 280 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 140 ml gesättigter Natriumchloridlösung gewaschen, über Magnesiumsulfat getrocknet und im Vakuum eingedampft. Das erhaltene braune Öl wurde in 50 ml wasser-freiem Dimethylformamid gelöst, mit 0.96 g (14.8 mmol) Natriumazid versetzt und das Reakfionsgemisch unter Rühren 18 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.58 g (75%) (RS)-1-(2-Azido-propyl)-1,4-dihydro-8-methoxy-[1]benzopyrano[4,3-b]pyrrol als farbloses Öl.

e) 1.57 g (5.5 mmol) (RS)-1-(2-Azido-propyl)-1,4-dihydro-8-methoxy-[1]benzopyrano[4,3-b]pyrrol gelöst in 60 ml wasserfreiem Ethanol wurden an 160 mg Platinoxid 17 Stunden unter hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 281 mg (2.45 mmol) Fumarsäure in 20 ml Methanol versetzt. Man rührte 18 Stunden bei Raumtemperatur und filtrierte anschliessend die weissen Kristalle ab. Man erhielt 1.42 g (86%) (RS)-1-(1,4-Dihydro-[1]benzopyrano-[4,3-b]pyrrol-1-yl)-1-methyl-ethylamin fumarat (1:0.5) mit Smp. 197-198°.

Beispiel 52

a) Eine Lösung von 50.0 g (0.31 mol) 5-Methoxy-1-indanon, 80 ml (0.92 mol) 3-Buten-2-ol, 132 ml (1.08 mol) 2,2-Dimethoxypropan und 600 mg p-Toluolsulfonsäure in 500 ml Toluol wurde zum Sieden gebracht. Das entstehende Methanol/Aceton-Gemisch wurde abdestilliert und die Reaktionslösung anschliessend noch 48 Stunden unter Rückfluss gekocht. Nach Abkühlen wurde die Lösung im Vakuum eingedampft. Reinigung an Kieselgel (Hexan/Diethylether 5:1) lieferte 19.2 g (31%) (RS)-2-(2-Buten-1-yl)-5-methoxy-1-indanon als hellgelbes Öl.

b) Durch eine auf -70° abgekühlte Lösung von 19.2 g (89 mmol) (RS)-2-(2-Buten-1-yl)-5-methoxy-1-indanon in 600 ml wasserfreiem Methanol leitete man unter Rühren 85 Minuten Ozon (3 g Ozon/Stunde). Anschliessend spülte man die Lösung mit Sauerstoff und gab dann 9.1 ml (0.12 mol) Dimethylsulfid in die kalte Lösung. Über Nacht kam die Lösung auf Raumtemperatur und wurde im Vakuum eingedampft. Der Rückstand wurde über eine Säule mit an Kieselgel adsorbierter Oxalsäurelösung (600 g Kieselgel 100 ml 10%iger Oxalsäurelösung) chromatographiert (Dichlormethan). Man erhielt 14.1 g (78%) (RS)-2-(2-Oxoethyl)-5-methoxy-1-indanon als gelbes Öl.

c) Durch eine auf -70° gekühlte Lösung von 13.3 g (61.5 mmol) (RS)-2-(2-Buten-1-yl)-5-methoxy-1-indanon in 200 ml wasserfreiem Dichlormethan und 100 ml wasserfreiem Methanol leitete man unter Rühren während 60 Minuten einen Ozonstrom (3 g Ozon/Stunde). Anschliessend spülte man die Lösung während 5 Minuten mit Sauerstoff und während 10 Minuten mit Argon. Nach Zugabe von 6.82 ml (92.2 mmol) Dimethylsulfid rührte man 16 Stunden bei Raumtemperatur. Das Reaktionsgemisch wurde im Vakuum eingedampft, der Rückstand mit 200 ml Dichlormethan versetzt und nach Zugabe von 25 ml Wasser und 25 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 11.6 g (92%) (RS)-2-(2-Oxoethyl)-5-methoxy-1-indanon als gelbes, Öl, welches ohne weitere Reinigung in die nächste Reaktion eingesetzt wurde.

d) Eine Lösung von 2 g (9.8 mmol) (RS)-2-(2-Oxoethyl)-5-methoxy-1-inda-non und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 2.94 g (39.2 mmol) (RS)-1-Amino-2-propanol in 20 ml wasserfreiem Toluol. Anschliessend wurde weitere 35 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:1) gereinigt. Man erhielt 1.6 g (67%) (RS)-1-(6-Methoxy-1,4-dihydro-indeno[1,2-b]pyrrol-1-yl)-propan-2-ol als braunes Öl, welches direkt in die nächste Reaktion eingesetzt wurde.

e) Zu einer auf 0° gekühlten Lösung von 1.41 g (5.8 mmol) (RS)-1-(6-Methoxy-1,4-dihydro-indeno[1,2-b]pyrrol-1-yl)-propan-2-ol und 3.24 ml (23.2 mmol) Triethylamin in 50 ml Dichlormethan gab man tropfenweise unter Rühren 1.9 ml (11.6 mmol) Methansulfonylchlorid und rührte weitere 1.5 Stunden bei dieser Temperatur. Das Reaktionsgemisch wurde anschlies-send mit 150 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 558 mg (8.6 mmol) Natriumazid versetzt und das Reaktionsgemisch unter Rühren 7 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.75 g (48%) (RS)-1-(2-Azido-propyl)-6-methoxy-1,4-dihydro-indeno[1,2-b]pyrrol als farbloses Öl.

f) 0.75 g (2.8 mmol) (RS)-1-(2-Azido-propyl)-6-methoxy-1,4-dihydro-indeno[1,2-b]pyrrol gelöst in 30 ml wasserfreiem Ethanol wurden an 60 mg Platinoxid 16 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 324 mg (2.79 mmol) Fumarsäure in 50 ml Methanol versetzt. Man rührte 16 Stunden bei Raumtemperatur und filtrierte anschliessend die weissen Kristalle ab. Man erhielt 530 mg (63%) (RS)-2-(6-Methoxy-1,4-dihydro-indeno[1,2-b]pyrrol-1-yl)-1-methyl-ethylamin fumarat (1:0.5) mit Smp. 189°.

Beispiel 53

a) Eine Lösung von 1.9 g (10 mmol) (RS)-2-(2-Oxoethyl)-4-chromanon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 3.0 g (40 mmol) (S)-1-Amino-2-propanol in 20 ml wasserfreiem Toluol.

Anschliessend wurde weitere 35 Minuten gekocht, wobei man das Lösungsmittel auf ein Volumen von 20 ml reduzierte. Das erkaltete Reaktionsgemisch wurde durch Säulenchromato-graphie an Kieselgel (Essigester/Toluol 2:3) gereinigt. Man erhielt 1.73 g (76%) (S)-1-(1,4-Dihydro-[1]benzopyrano[4,3-b]pyrrol-1-yl)-propan-2-ol als gelbes Öl.

b) Zu einer auf 0° gekühlten Lösung von 1.7 g (7.4 mmol) (S)-1-(1,4-Dihydro-[1]benzopyrano[4,3-b]pyrrol-1-yl)-propan-2-ol und 4.13 ml (29.7 mmol) Triethylamin in 50 ml wasserfreiem Dichlormethan gab man tropfenweise unter Rühren 1.15 ml (14.8 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 140 ml gesättigter Natrium-chloridlösung gewaschen, über Magnesiumsulfat getrocknet und im Vakuum eingedampft. Der erhaltene braune Feststoff wurde in 50 ml wasserfreiem Dimethylformamid gelöst, mit 0.88 g (13.5 mmol) Natriumazid versetzt und das Reaktionsgemisch unter Rühren 18 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.49 g (79%) (R)-1-(2-Azido-propyl)-1,4-dihydro-[1]benzopyrano[4,3-b]-pyrrol als farbloses Öl.

c) 1.47 g (5.8 mmol) (R)-1-(2-Azido-propyl)-1,4-dihydro-[1]benzopyrano[4,3-b]-pyrrol gelöst in 60 ml wasserfreiem Ethanol wurden an 150 mg Platinoxid 18 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 294 mg (2.53 mmol) Fumarsäure in 20 ml Methanol versetzt. Man rührte 18 Stunden bei Raumtemperatur und filtrierte anschliessend die weissen Kristalle ab. Man erhielt 1.3 g (79%) (R)-1-(1,4-Dihydro-[1]benzopyrano[4,3-b]pyrrol-1-yl)-1-methyl-ethylamin fumarat (1:0.5) mit Smp. 194-195°.

Beispiel **54**

a) Eine Lösung von 23.9 g (0.145 mol) 4-Thiochromanon, 30 ml (0.35 mol) 3-Buten-2-ol und 240 mg p-Toluolsulfonsäure in 30 ml 2,2-Dimethoxypropan und 240 ml wasserfreiem Toluol wurde 20 Stunden unter Rückfluss gekocht. Das Reaktionsgemisch wurde anschliessend im Vakuum eingeengt und durch Säulenchromatographie an Kieselgel (Hexan/Diethylether 5:1) gereinigt. Man erhielt 15.3 g (48%) (RS)-2-(2-Buten-1-yl)-4-thiochroma-non als gelbes Öl.

b) Durch eine auf -70° gekühlte Lösung von 3 g (13.7 mmol) (RS)-2-(2-Buten-1-yl)-4-thiochromanon in 100 ml wasserfreiem Dichlormethan und 30 ml wasserfreiem Methanol leitete man unter Rühren während 15 Minuten einen Ozonstrom (3 g Ozon/Stunde). Anschliessend spülte man die Lösung während 5 Minuten mit Sauerstoff und während 10 Minuten mit Argon. Nach Zugabe von 2 ml (20.5 mol) Dimethylsulfid rührte man 16 Stunden bei Raumtemperatur. Das Reaktionsgemisch wurde anschliessend im Vakuum eingedampft, der Rückstand mit 50 ml Dichlormethan versetzt und nach Zugabe von 5 ml Wasser und 5 ml Trifluoressigsäure 1 Stunde bei Raumtemperatur gerührt. Man goss das Gemisch anschliessend auf 50 ml Wasser und neutralisierte unter Rühren durch spatelweise Zugabe von Natriumhydrogencarbonat. Es wurden weitere 50 ml Wasser zugegeben, die Phasen getrennt und die Wasserphase zweimal mit jeweils 80 ml Dichlormethan extrahiert. Die vereinigten organischen Phasen wurden über Magnesiumsulfat getrocknet und im Vakuum eingeengt. Man erhielt 2.8 g (99%) 2-(2-Oxoethyl)-4-thiochromanon als gelbes Öl, welches ohne weitere Umkristallisation in die nächste Reaktion eingesetzt wurde.

c) Eine Lösung von 2 g (9.7 mmol) (RS)-2-(2-Oxoethyl)-4-thiochromanon und 120 mg p-Toluolsulfonsäure in 100 ml wasserfreiem Toluol wurde am Wasserabscheider erhitzt. Zu der siedenden Lösung tropfte man über einen Zeitraum von 5 Minuten eine Lösung von 2.9 g (38.6 mmol) (RS)-1-Amino-2-propanol in 20 ml wasserfreiem Toluol. Anschliessend wurde weitere 35 Minuten gekocht, wobei man das Lösungsmittel auf ein Volumen von 20 ml reduzierte. Das erkaltete Reaktionsgemisch wurde durch Säulenchromatographie an Kieselgel (Essigester/Toluol 1:1) gereinigt. Man erhielt 1.8 g (76%) (RS)-1-(1,4-Dihydro-[1]benzothiopyrano[4,3-b]pyrrol-1-yl)-propan-2-ol als hellbraunen Feststoff, der ohne weitere Umkristallisation in die nächste Reaktion eingesetzt wurde.

d) Zu einer auf 0° gekühlten Lösung von 1.8 g (7.3 mmol) (RS)-1-(1,4-Dihydro-[1]benzothiopyrano[4,3-b]pyrrol-1-yl)-propan-2-ol und 4.1 ml (29.3 mmol) Triethylamin in 50 ml wasserfreiem Dichlormethan gab man tropfenweise unter Rühren 1.15 ml (14.7 mmol) Methansulfonylchlorid und rührte weitere 1.5 Stunden bei dieser Temperatur. Das Reaktionsgemisch wurde anschliessend mit 180 ml Diethylether verdünnt, zweimal mit jeweils 70 ml gesättigter Natriumhydrogencarbonatlösung gewaschen und die vereinigten Wasserphasen einmal mit 70 ml Diethylether extrahiert. Die vereinigten organischen Phasen wurden mit 140 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.96 g (14.7 mmol) Natriumazid versetzt und das Reaktionsgemisch unter

Rühren 17 Stunden auf 60° erwärmt. Nach dem Abkühlen goss man die Lösung auf 100 ml Wasser und extrahierte zweimal mit jeweils 120 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.17 g (59%) (RS)-1-(2-Azido-propyl)-1,4-dihydro-[1]benzothiopyrano[4,3-b]pyrrol als schwach gelbes Öl.

e) Zu einer Suspension von 247 mg (6.49 mmol) Lithiumaluminiumhydrid in 30 ml wasserfreiem Tetrahydrofuran gab man tropfenweise unter Rühren 1.17 g (4.3 mmol) (RS)-1-(2-Azido-propyl)-1,4-dihydro-[1]benzothiopyrano[4,3-b]pyrrol gelöst in 30 ml wasserfreiem Tetrahydrofuran Es wurde anschliessend 2 Stunden unter Rückfluss gekocht. Die Hydrolyse erfolgte mit 20%-wässrigem Tetrahydrofuran. Die flüssige Phase wurde abgetrennt und der Rückstand nochmals 15 Minuten mit 50 ml 20%-wässrigem Tetrahydrofuran ausgekocht. Die vereinigten Lösungen wurden mit 100 ml Diethylether versetzt, die organische Phase wurde abgetrennt und die Wasserphase zweimal mit jeweils 50 ml Diethylether extrahiert. Die vereinigten organischen Phasen wurden mit 100 ml gesättigter Natriumchloridlösung gewaschen, über Magnesiumsulfat getrocknet und im Vakuum eingeengt. Man erhielt 970 mg (92%) eines farblosen Öles, welches in 100 ml wasserfreiem Diethylether gelöst, filtriert und unter Rühren mit einer Lösung von 461 mg (3.97 mmol) Fumarsäure in 20 ml Methanol versetzt wurde. Man rührte 3 Stunden bei Raumtemperatur und filtrierte anschliessend die schwach gelben Kristalle ab. Man erhielt 1.2 g (77%) (RS)-1-(1,4-Dihydro-[1]benzothiopyrano[4,3-b]pyrrol-1-yl)-1-methyl-ethylamin fumarat (1:1) mit Smp. 201°.

Beispiel **55**

a) Eine Lösung von 26.4 g (0.16 mol) 7-Methoxy-1-indanon, 33.6 ml (0.39 mol) 3-Buten-2-ol und 265 mg p-Toluolsulfonsäure in 33.6 ml 2,2-Dimethoxypropan und 265 ml wasserfreiem Toluol wurde 17 Stunden unter Rück-fluss gekocht. Das Reaktionsgemisch wurde anschliessend im Vakuum eingeengt und durch Säulenchromatographie an Kieselgel gereinigt (Hexan/Diethylether 5:1). Man erhielt 9.9 g (28%) (RS)-2-(2-Buten-1-yl)-7-methoxy-1-indanon als gelbes Öl.

b) Durch eine auf -70° gekühlte Lösung von 9.9 g (45.8 mmol) (RS)-2-(2-Buten-1-yl)-7-methoxy-1-indanon in 200 ml wasserfreiem Dichlormethan und 100 ml wasserfreiem Methanol leitete man unter Rühren während 45 Minuten einen Ozonstrom (3 g Ozon/Stunde). Anschliessend spülte man die Lösung während 5 Minuten mit Sauerstoff und während 10 Minuten mit Argon. Nach Zugabe von 5 ml (67.7 mmol) Dimethylsulfid rührte man 16 Stunden bei Raumtemperatur. Das Reaktionsgemisch wurde im Vakuum eingedampft, der Rückstand mit 200 ml Dichlormethan versetzt und nach Zugabe von 20 ml Wasser und 20 ml Trifluoressigsäure 3 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt aus Essigester/Hexan kristallisiert. Man erhielt 6.6 g (71%) (RS)-2-(2-Oxoethyl)-7-methoxy-1-indanon als weissen Feststoff mit Smp.102°.

c) Eine Lösung von 2.04 g (10 mmol) (RS)-2-(2-Oxoethyl)-7-methoxy-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.0 g (40 mmol) (RS)-1-Amino-2-propanol in 20 ml wasserfreiem Toluol. Anschliessend wurde weitere 35 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 2:3) gereinigt. Man erhielt 1.1 g (45%) (RS)-1-(8-Methoxy-1,4-dihydro-indeno[1,2-b]pyrrol-1-yl)-propan-2-ol als braunes Öl, welches direkt in die nächste Reaktion eingesetzt wurde.

d) Zu einer auf 0° gekühlten Lösung von 1.1 g (4.5 mmol) (RS)-1-(8-Methoxy-1,4-dihydro-indeno[1,2-b]pyrrol-1-yl)-propan-2-ol und 2.5 ml (18.0 mmol) Triethylamin in 25 ml Dichlormethan gab man tropfenweise unter Rühren 0.7 ml (9.0 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 140 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 526 mg (8.1 mmol) Natriumazid versetzt und das Reaktionsgemisch unter Rühren 29 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.68 g (56%) (RS)-1-(2-Azido-propyl)-8-methoxy-1,4-dihydro-indeno[1,2-b]-pyrrol als farbloses Öl.

e) 0.67 g (2.5 mmol) (RS)-1-(2-Azido-propyl)-8-methoxy-1,4-dihydro-indeno[1,2-b]pyrrol gelöst in 25 ml wasser-

freiem Ethanol wurden an 67 mg Platinoxid 5 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 273 mg (2.35 mmol) Fumarsäure in 10 ml Methanol versetzt. Man rührte 22 Stunden bei Raumtemperatur und filtrierte anschliessend die weissen Kristalle ab. Man erhielt 559 mg (74%) (RS)-2-(8-Methoxy-1,4-dihydro-indeno[1,2-b]pyrrol-1-yl)-1-methyl-ethylamin fumarat (1:0.5) mit Smp. 193°.

Beispiel **56**

a) Eine Lösung von 23.3 g (0.105 mol) (RS)-3-Phenyl-1-tetralon, 21.6 ml (0.25 mol) 3-Buten-2-ol und 230 mg p-Toluolsulfonsäure in 21.6 ml 2,2-Dimethoxypropan und 230 ml wasserfreiem Toluol wurde 30 Stunden unter Rückfluss gekocht. Das Reaktionsgemisch wurde anschliessend im Vakuum eingeengt und durch Säulenchromatographie an Kieselgel (Hexan/Diethylether 4:1) gereinigt. Man erhielt 11.8 g (41%) (2RS/3RS)-2-(2-Buten-1-yl)-3-phenyl-1-tetralon als rotes Öl.
b) Durch eine auf -70° gekühlte Lösung von 11.8 g (42.7 mmol) (2RS/3RS)-2-(2-Buten-1-yl)-3-phenyl-1-tetralon in 300 ml wasserfreiem Dichlormethan und 100 ml wasserfreiem Methanol leitete man unter Rühren während 40 Minuten einen Ozonstrom (3 g Ozon/Stunde). Anschliessend spülte man die Lösung während 5 Minuten mit Sauerstoff und während 10 Minuten mit Argon. Nach Zugabe von 4.45 ml (60.8 mmol) Dimethylsulfid rührte man 17 Stunden bei Raumtemperatur. Das Reaktionsgemisch wurde im Vakuum eingedampft, der Rückstand mit 300 ml Dichlormethan versetzt und nach Zugabe von 30 ml Wasser und 30 ml Trifluoressigsäure 2 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt durch Säulenchromatographie an Kieselgel (Toluol/ Essigester 9:1) gereinigt. Man erhielt 9.7 g (86%) (2RS/3RS)-2-(2-Oxoethyl)-3-phenyl-1-tetralon als gelbes Öl.
c) Eine Lösung von 2 g (7.6 mmol) (2RS/3RS)-2-(2-Oxoethyl)-3-phenyl-1-tetralon und 80 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.18 g (29.0 mmol) (RS)-1-Amino-2-propanol in 20 ml wasserfreiem Toluol. Anschliessend wurde weitere 30 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:9) gereinigt. Man erhielt 1.04 g (45%) (2RS/4RS)-1-(4,5-Dihydro-4-phenyl-1-H-benz[g]indol-1-yl)-propan-2-ol als braunes Öl, welches direkt in die nächste Reaktion eingesetzt wurde.
d) Zu einer auf 0° gekühlten Lösung von 1.04 g (3.4 mmol) (2RS/ 4RS)-1-(4,5-Dihydro-4-phenyl-1-H-benz[g]indol-1-yl)-propan-2-ol und 1.9 ml (13.6 mmol) Triethylamin in 30 ml Dichlormethan gab man tropfenweise unter Rühren 0.53 ml (6.8 mmol) Methansulfonylchlorid und rührte weitere 1.5 Stunden bei dieser Temperatur. Das Reaktionsgemisch wurde an-schliessend mit 200 ml Diethylether verdünnt, zweimal mit jeweils 70 ml gesättigter Natriumhydrogencarbonatlösung gewaschen und die vereinigten Wasserphasen einmal mit 130 ml Diethylether extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 25 ml wasserfreiem Dimethylformamid gelöst, mit 0.45 g (6.8 mmol) Natriumazid versetzt und das Reaktionsgemisch unter Rühren 6 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 100 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 473 mg (42%) (2RS/4RS)-1-(2-Azido-propyl)-4,5-dihydro-4-phenyl-1H-benz[g]indol als farbloses Öl.
e) 473 mg (1.44 mmol) (2RS/4RS)-1-(2-Azido-propyl)-4,5-dihydro-4-phenyl-1-H-benz[g]indol gelöst in 20 ml wasserfreiem Ethanol wurden an 50 mg Platinoxid 5 Stunden hydriert Es wurde anschliessend vom Katalysator abfiltriert, mit Ethanol nachgewaschen und das Lösungsmittel im Vakuum abgezogen. Das erhaltene farblose Öl wurde in 40 ml wasserfreiem Diethylether gelöst, filtriert und unter Rühren mit einer Lösung von 148 mg (1.28 mmol) Fumarsäure in 5 ml Methanol versetzt. Man rührte 7 Stunden bei Raumtemperatur und filtrierte anschliessend die weissen Kristalle ab. Man erhielt 446 mg (74%) (2RS/4RS)-2-(4,5-Dihydro-4-phenyl-1H-benz[g]indol-1-yl)-1-methyl-ethylamin fumarat (1:1) mit Smp. 187°.

Beispiel **57**

a) Eine Lösung von 20 g (96 mmol) (RS)-3-Phenyl-1-indanon, 20 ml (0.23 mol) 3-Buten-2-ol und 200 mg p-Toluolsulfonsäure in 20 ml 2,2-Dimethoxy-propan und 200 ml wasserfreiem Toluol wurde 22 Stunden unter Rückfluss

gekocht. Das Reaktionsgemisch wurde anschliessend im Vakuum einge-engt und durch Säulenchromatographie an Kieselgel gereinigt (Hexan/ Essigester 5:1). Man erhielt 10 g (40%) (2RS/3RS)-2-(2-Buten-1-yl)-3-phenyl-1-indanon als gelbes Öl.

b) Durch eine auf -70° gekühlte Lösung von 10 g (38.1 mmol) (2RS/3RS)-2-(2-Buten-1-yl)-3-phenyl-1-indanon in 200 ml wasserfreiem Dichlormethan und 70 ml wasserfreiem Methanol leitete man unter Rühren während 40 Minuten einen Ozonstrom (3 g Ozon/Stunde). Anschliessend spülte man die Lösung während 5 Minuten mit Sauerstoff und während 10 Minuten mit Argon. Nach Zugabe von 4.3 ml (58.2 mmol) Dimethylsulfid rührte man 16 Stunden bei Raumtemperatur. Das Reaktionsgemisch wurde im Vakuum eingedampft, der Rückstand mit 150 ml Dichlormethan versetzt und nach Zugabe von 20 ml Wasser und 20 ml Trifluoressigsäure 1 Stunde bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt und die Wasserphase zweimal mit jeweils 150 ml Dichlormethan extrahiert. Die vereinigten organischen Phasen wurden über Magnesiumsulfat getrocknet und im Vakuum eingeengt. Man erhielt 9.5 g (99%) (2RS/3RS)-2-(2-Oxoethyl)-3-phenyl-1-indanon als Öl, welches ohne weitere Umkristallisation in die nächste Reaktion eingesetzt wurde.

c) Eine Lösung von 3.0 g (12 mmol) (2RS/3RS)-2-(2-Oxoethyl)-3-phenyl-1-indanon und 100 mg p-Toluolsulfonsäure in 100 ml wasserfreiem Toluol wurde am Wasserabscheider erhitzt. Zu der siedenden Lösung tropfte man über einen Zeitraum von 5 Minuten eine Lösung von 3.6 g (48 mmol) (RS)-1-Amino-2-propanol in 20 ml wasserfreiem Toluol. Anschliessend wurde weitere 35 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:1) gereinigt. Man erhielt 2.4 g (69%) (2RS/4RS)-1-(4-Phenyl-1,4-dihydro-indeno[1,2-b]pyrrol-1-yl)-propan-2-ol als Öl, welches direkt in die nächste Reaktion eingesetzt wurde.

d) Zu einer auf 0° gekühlten Lösung von 2.3 g (7.9 mmol) (2RS/4RS)-1-(4-Phenyl-1,4-dihydro-indeno[1,2-b]pyrrol-1-yl)-propan-2-ol und 4.46 ml (31.8 mmol) Triethylamin in 60 ml Dichlormethan gab man tropfenweise unter Rühren 1.24 ml (15.9 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 140 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 60 ml wasserfreiem Dimethylformamid gelöst, mit 884 mg (13.6 mmol) Natriumazid versetzt und das Reaktionsgemisch unter Rühren 16 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 1.22 g (49%) (2RS/4RS)-1-(2-Azido-propyl)-4-phenyl-1,4-dihydro-indeno[1,2-b]-pyrrol als leicht braunes Öl.

e) 1.2 g (3.8 mmol) (2RS/4RS)-1-(2-Azido-propyl)-4-phenyl-1,4-dihydro-indeno[1,2-b]-pyrrol gelöst in 50 ml wasserfreiem Ethanol wurden an 120 mg Platinoxid 5 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 480 mg (4.13 mmol) Fumarsäure in 20 ml Methanol versetzt. Man rührte 16 Stunden bei Raumtemperatur und filtrierte anschliessend die Kristalle ab. Man erhielt 1.04 g (65%) (2RS/4RS)-2-(4-Phenyl-1,4-dihydro-indeno[1,2-b]pyrrol-1-yl)-1-methylethylamin fumarat (1:0.86) mit Smp. 191°.

Beispiel **58**

a) Eine Lösung von 21.5 g (0.13 mol) 4-Methoxy-1-indanon, 27.4 ml (0.32 mol) 3-Buten-2-ol und 210 mg p-Toluolsulfonsäure in 27.4 ml 2,2-Dimethoxypropan und 210 ml wasserfreiem Toluol wurde 16 Stunden unter Rückfluss gekocht. Das Reaktionsgemisch wurde anschliessend im Vakuum eingeengt und durch Säulenchromatographie an Kieselgel gereinigt (Hexan/Essigester 9:1). Man erhielt 6.42 g (23%) (RS)-2-(2-Buten-1-yl)-4-methoxy-1-indanon als gelbes Öl.

b) Durch eine auf -70° gekühlte Lösung von 6.42 g (29.7 mmol) (RS)-2-(2-Buten-1-yl)-4-methoxy-1-indanon in 180 ml wasserfreiem Dichlormethan und 60 ml wasserfreiem Methanol leitete man unter Rühren während 35 Minuten einen Ozonstrom (3 g Ozon/Stunde). Anschliessend spülte man die Lösung während 5 Minuten mit Sauerstoff und während 10 Minuten mit Argon. Nach Zugabe von 3.3 ml (44.7 mmol) Dimethylsulfid rührte man 16 Stunden bei Raumtemperatur. Das Reaktionsgemisch wurde im Vakuum eingedampft, der Rückstand mit 200 ml Dichlormethan versetzt und nach Zugabe von 20 ml Wasser und 20 ml Trifluoressigsäure 2 Stunden bei Raumtemperatur gerührt. Man goss das Gemisch anschliessend auf 100 ml Wasser und neutralisierte unter Rühren durch spatelweise Zugabe von Natriumhydrogencarbonat. Es wurden weitere 100 ml Wasser zugegeben, die Phasen getrennt

und die Wasserphase zweimal mit jeweils 150 ml Dichlormethan extrahiert. Die vereinigten organischen Phasen wurden über Magnesiumsulfat getrocknet, im Vakuum eingeengt und das erhaltene Rohprodukt aus Essigester/Hexan kristallisiert. Man erhielt 5.1 g (84%) (RS)-2-(2-Oxoethyl)-4-methoxy-1-indanon als weissen Feststoff mit Smp.71°.

c) Eine Lösung von 2.04 g (10 mmol) (RS)-2-(2-Oxoethyl)-4-methoxy-1-indanon und 80 mg p-Toluolsulfonsäure in 90 ml wasserfreiem Toluol wurde am Wasserabscheider erhitzt. Zu der siedenden Lösung tropfte man über einen Zeitraum von 5 Minuten eine Lösung von 3.13 g (41.7 mmol) (RS)-1-Amino-2-propanol in 20 ml wasserfreiem Toluol. Anschliessend wurde weitere 35 Minuten gekocht, wobei man das Lösungsmittel auf ein Volumen von 25 ml reduzierte. Das erkaltete Reaktionsgemisch wurde durch Säulenchromatographie an Kieselgel (Essigester/Toluol 1:4) gereinigt. Man erhielt 0.7 g (29%) (RS)-1-(5-Methoxy-1,4-dihydro-indeno[1,2-b]pyrrol-1-yl)-propan-2-ol als braunes Öl, welches direkt in die nächste Reaktion eingesetzt wurde.

d) Zu einer auf 0° gekühlten Lösung von 0.7 g (2.9 mmol) (RS)-1-(5-Methoxy-1,4-dihydro-indeno[1,2-b]pyrrol-1-yl)-propan-2-ol und 1.6 ml (11.5 mmol) Triethylamin in 20 ml Dichlormethan gab man tropfenweise unter Rühren 0.45 ml (5.77 mmol) Methansulfonylchlorid und rührte weitere 1.5 Stunden bei dieser Temperatur. Das Reaktionsgemisch wurde anschliessend mit 200 ml Diethylether verdünnt, zweimal mit jeweils 70 ml gesättigter Natriumhydrogencarbonatlösung gewaschen und die vereinigten Wasserphasen einmal mit 140 ml Diethylether extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 20 ml wasserfreiem Dimethylformamid gelöst, mit 366 mg (5.6 mmol) Natriumazid versetzt und das Reaktionsgemisch unter Rühren 18 Stunden auf 60° erwärmt. Nach dem Abkühlen goss man die Lösung auf 140 ml Wasser und extrahierte zweimal mit jeweils 140 ml Diethylether. Die vereinigten organischen Phasen wusch man je einmal mit 140 ml Wasser und mit 140 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.55 g (73%) (RS)-1-(2-Azido-propyl)-5-methoxy-1,4-dihydro-indeno[1,2-b]-pyrrol als farbloses Ol.

e) 0.54 g (2.0 mmol) (RS)-1-(2-Azido-propyl)-5-methoxy-1,4-dihydro-indeno[1,2-b]-pyrrol gelöst in 20 ml wasserfreiem Ethanol wurde an 54 mg Platinoxid 17 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 110 mg (0.95 mmol) Fumarsäure in 10 ml Methanol versetzt. Man rührte 22 Stunden bei Raumtemperatur und filtrierte anschliessend die weissen Kristalle ab. Man erhielt 500 mg (83%) (RS)-2-(5-Methoxy-1,4-dihydro-indeno[1,2-b]pyrrol-1-yl)-1-methyl-ethylamin fumarat (1:0.5) mit Smp. 194°.

Beispiel **59**

a) Eine Lösung von 0.95 g (3.1 mmol) (2RS/4RS)-2-(4,5-Dihydro-4-phenyl-1-H-benz[g]indol-1-yl)-1-methyl-ethylamin in 15 ml wasserfreiem Pyridin wurde mit 15 ml Essigsäureanhydrid versetzt und 30 Minuten unter Rühren auf 50° erhitzt. Das Reaktionsgemisch wurde anschliessend auf Eis gegossen und mit 70 ml gesättigter Natriumhydrogencarbonatlösung versetzt. Man extrahierte zweimal mit jeweils 100 ml Dichlormethan, wusch die vereinigten organischen Phasen einmal mit kalter 3N Schwefelsäure und einmal mit kalter gesättigter Natriumhydrogencarbonatlösung. Nachdem man die Lösung über Magnesiumsulfat getrocknet hatte, wurde das Lösungsmittel im Vakuum abgezogen und der Rückstand mit 40 ml wasserfreiem Dioxan aufgenommen. Man gab 729 mg (3.21 mmol) DDQ hinzu und kochte 1 Stunde unter Rückfluss. Anschliessend engte man das Reaktionsgemisch im Vakuum ein und reinigte den Rückstand durch Säu-lenchromatographie an Kieselgel (Dichlormethan/Methanol 20:1). Man erhielt 0.69 g (64%) (RS)-N-[2-(4-Phenyl-1-H-benz[g]indol-1-yl)-1-methyl-ethyl]-acetamid als hellbraunen Feststoff, der ohne weitere Umkristallisation in die nächste Reaktion eingesetzt wurde.

b) Eine Mischung aus 670 mg (1.96 mmol) (RS)-N-[2-(4-Phenyl-1-H-benz[g]indol-1-yl)-1-methyl-ethyl]-acetamid, 1.32 g (23.4 mmol) Kaliumhydroxid in 15 ml Wasser und 30 ml Ethylenglykol wurde 46 Stunden unter Rückfluss gekocht. Das Reaktionsgemisch wurde anschliessend in 80 ml gesättigte Natriumchloridlösung gegossen und zweimal mit jeweils 80 ml Essigester extrahiert. Die vereinigten organischen Phasen wurden einmal mit 100 ml gesättigter Natriumchloridlösung gewaschen und über Magnesiumsulfat getrocknet. Nach dem Einengen im Vakuum, wurde der Rückstand säulenchromatographisch an Kieselgel (Dichlormethan/Methanol 9:1) gereinigt und das erhaltene Öl [308 mg (1.03 mmol)] in 30 ml wasserfreiem Diethylether gelöst, filtriert und unter Rühren mit einer Lösung von 119 mg (1.03 mmol) Fumarsäure in 6 ml Methanol versetzt. Man rührte 20 Stunden bei Raumtemperatur und filtrierte anschliessend die weissen Kristalle ab. Man erhielt 308 mg (41%) (RS)-2-(4-Phenyl-1-H-benz[g]indol-1-yl)-1-methyl-ethylamin fumarat (1:0.7) mit Smp. >230°.

Beispiel 60

a) Zu einer auf -70° gekühlten Lösung von 2.96 g (18.3 mmol) 6-Methoxy-1-indanon in 300 ml wasserfreiem Tetrahydrofuran tropfte man unter Rühren während 10 Minuten eine bei 0° aus 3.12 ml (22 mmol) Diisopropylamin und 13.8 ml (22 mmol) n-Butyllithium (1.6 N in Hexan) hergestellte Lösung von LDA in 40 ml wasserfreiem Tetrahydrofuran. Nach 15 Minuten tropfte man während 5 Minuten eine Lösung von 1.62 ml (20.2 mmol) Chloraceton in 40 ml wasserfreiem Tetrahydrofuran zu der Lösung und rührte anschlies-send 2 Stunden bei Raumtemperatur. Man goss das Reaktionsgemisch auf 150 ml Eis, gab 150 ml gesättigte Natriumchloridlösung hinzu und extrahierte zweimal mit jeweils 300 ml Diethylether. Die vereinigten organischen Phasen wusch man einmal mit 200 ml gesättigter Natriumchloridlösung, trocknete über Magnesiumsulfat und engte im Vakuum ein. Das erhaltene Rohprodukt wurde durch Säulenchromatographie an Kieselgel (Hexan/ Diethylether 3:2, dann 2:3) gereinigt. Man erhielt 1.8 g (45%) (RS)-6-Methoxy-2-(2-oxopropyl)-1-indanon als rotes Öl.
b) Eine Lösung von 1.8 g (8.3 mmol) (RS)-6-Methoxy-2-(2-oxopropyl)-1-indanon und 70 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 2.48 g (33 mmol) (RS)-1-Amino-2-propanol in 20 ml wasserfreiem Toluol. Anschliessend wurde weitere 3 Stunden gekocht, wobei man das Lösungsmittel auf ein Volumen von 30 ml reduzierte. Das erkaltete Reaktionsgemisch wurde durch Säulenchromatographie an Kieselgel (Diethylether/Hexan 7:3) gereinigt. Man erhielt 1.37 g (65%) (RS)-1-(7-Methoxy-2-methyl-1,4-dihydro-indeno[1,2-b]pyrrol-1-yl)-propan-2-ol als Feststoff mit Smp. 110°.
c) Zu einer auf 0° gekühlten Lösung von 1.35 g (5.3 mmol) (RS)-1-(7-Methoxy-2-methyl-1,4-dihydro-indeno[1,2-b]pyrrol-1-yl)-propan-2-ol und 2.9 ml (20.9 mmol) Triethylamin in 40 ml Dichlormethan gab man tropfenweise unter Rühren 0.81 ml (10.5 mmol) Methansulfonylchlorid und rührte weitere 1.5 Stunden bei dieser Temperatur. Das Reaktionsgemisch wurde anschliessend auf 70 ml Wasser gegeben, zweimal mit jeweils 100 ml Dichlormethan extrahiert und die vereinigten organischen Phasen je einmal mit 70 ml gesättigter Natriumhydrogencarbonatlösung und 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.68 g (10.5 mmol) Natriumazid versetzt und das Reaktionsgemisch unter Rühren 23 Stunden auf 80° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.93 g (62%) (RS)-1-(2-Azido-propyl)-7-methoxy-2-methyl-1,4-dihydro-indeno[1,2-b]pyrrol als farbloses Öl.
d) 0.92 g (3.3 mmol) (RS)-1-(2-Azido-propyl)-7-methoxy-2-methyl-1,4-dihydro-indeno[1,2-b]pyrrol gelöst in 70 ml wasserfreiem Ethanol wurde an 90 mg Platinoxid 16 Stunden hydriert. Es wurde anschliessend vom Kataly-sator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 160 mg (1.4 mmol) Fumarsäure in 15 ml Methanol versetzt. Man rührte 17 Stunden bei Raumtemperatur und filtrierte anschliessend die weissen Kristalle ab. Man erhielt 800 mg (78%) (RS)-2-(7-Methoxy-2-methyl-1,4-dihydro-indeno[1,2-b]pyrrol-1-yl)-1-methyl-ethylamin fumarat (1:0.5) mit Smp. 187-188°.

Beispiel 61

a) Zu einer auf -70° gekühlten Lösung von 3.24 g (20 mmol) 5-Methoxy-1-indanon in 350 ml wasserfreiem Tetrahydrofuran tropfte man unter Rühren während 15 Minuten eine bei 0° aus 4.25 ml (30 mmol) Diisopropylamin und 18.8 ml (30 mmol) n-Butyllithium (1.6 N in Hexan) hergestellte Lösung von LDA in 60 ml wasserfreiem Tetrahydrofuran. Nach 45 Minuten tropfte man während 15 Minuten eine Lösung von 1.6 ml (20 mmol) Chloraceton in 60 ml wasserfreiem Tetrahydrofuran zu der Lösung und rührte anschliessend 2 Stunden bei Raumtemperatur. Man goss das Reaktionsgemisch auf 150 ml Eis, gab 150 ml gesättigte Natriumchloridlösung hinzu und extrahierte zweimal mit jeweils 300 ml Diethylether. Die vereinigten organischen Phasen wusch man einmal mit 200 ml gesättigter Natriumchloridlösung, trocknete über Magnesiumsulfat und engte im Vakuum ein. Das erhaltene Rohprodukt wurde durch Säulenchromatographie an Kieselgel (Hexan/ Diethylether 3:7) gereinigt. Man erhielt 1.4 g (32%) (RS)-5-Methoxy-2-(2-oxopropyl)-1-indanon als Feststoff mit Smp. 73°.
b) Eine Lösung von 1.2 g (5.5 mmol) (RS)-5-Methoxy-2-(2-oxopropyl)-1-indanon und 60 mg p-Toluolsulfonsäure in 70 ml wasserfreiem Toluol wurde am Wasserabscheider erhitzt. Zu der siedenden Lösung tropfte man über einen Zeitraum von 5 Minuten eine Lösung von 1.65 g (22 mmol) (RS)-1-Amino-2-propanol in 20 ml wasserfreiem Toluol. Anschliessend wurde weitere 3 Stunden gekocht, wobei man das Lösungsmittel auf ein Volumen von 30 ml reduzierte. Das erkaltete Reaktionsgemisch wurde durch Säulenchromatographie an Kieselgel (Diethylether/Hexan 7:3) gereinigt. Man erhielt 1.17 g (82%) (RS)-1-(6-Methoxy-2-methyl-1,4-dihydro-indeno[1,2-b]pyrrol-1-yl)-propan-2-ol als Öl.

c) Zu einer auf 0° gekühlten Lösung von 1.16 g (4.5 mmol) (RS)-1-(6-Methoxy-2-methyl-1,4-dihydro-indeno[1,2-b]pyrrol-1-yl)-propan-2-ol und 2.5 ml (18 mmol) Triethylamin in 50 ml Dichlormethan gab man tropfenweise unter Rühren 0.7 ml (9.0 mmol) Methansulfonylchlorid und rührte weitere 1.5 Stunden bei dieser Temperatur. Das Reaktionsgemisch wurde anschliessend auf 70 ml Wasser gegeben, zweimal mit jeweils 100 ml Dichlormethan extrahiert und die vereinigten organischen Phasen je einmal mit 70 ml gesättigter Natriumhydrogencarbonatlösung und 70 ml gesättigter Natriumchloridlösung gewaschen, über Magnesiumsulfat getrocknet und im Vakuum eingedampft. Das erhaltene braune Öl wurde in 40 ml wasserfreiem Dimethylformamid gelöst, mit 0.58 g (9.0 mmol) Natriumazid versetzt und das Reaktionsgemisch unter Rühren 16 Stunden auf 80° erwärmt. Nach dem Abkühlen goss man die Lösung auf 70 ml Wasser und extrahierte zweimal mit jeweils 100 ml Essigester. Die vereinigten organischen Phasen wusch man je einmal mit 70 ml Wasser und mit 70 ml gesättigter Natriumchloridlösung, trocknete über Magnesiumsulfat und engte die Lösung im Vakuum ein. Das erhaltene braune Öl wurde durch Säulenchromatographie an Kieselgel (Toluol) gereinigt. Man erhielt 0.86 g (55%) (RS)-1-(2-Azido-propyl)-6-methoxy-2-methyl-1,4-dihydro-indeno[1,2-b]pyrrol als farbloses Öl.

d) 0.85 g (3.0 mmol) (RS)-1-(2-Azido-propyl)-6-methoxy-2-methyl-1,4-dihydro-indeno-[1,2-b]-pyrrol gelöst in 50 ml wasserfreiem Ethanol wurde an 85 mg Platinoxid 17 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 155 mg (1.3 mmol) Fumarsäure in 15 ml Methanol versetzt. Man rührte 19 Stunden bei Raumtemperatur und filtrierte anschliessend die weissen Kristalle ab. Man erhielt 780 mg (83%) (RS)-2-(6-Methoxy-2-methyl-1,4-dihydro-indeno[1,2-b]pyrrol-1-yl)-1-methyl-ethylamin fumarat (1:0.5) mit Smp. 215°.

Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| | Tablettengewicht 250 |

Beispiel B

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |
| Na-carboxymethylstärke | 20 |
| Magnesiumstearat | 4 |

(fortgesetzt)

| | mg/Tablette |
|---|---|
| | Tablettengewicht 400 |

Beispiel C

Es werden Kapseln folgender Zusammensetzung hergestellt:

| | mg/Kapsel |
|---|---|
| Wirkstoff | 50 |
| Lactose krist. | 60 |
| Mikrokristalline Cellulose | 34 |
| Talk | 5 |
| Magnesiumstearat | 1 |
| | Kapselfüllgewicht 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

**Patentansprüche**

**1.** Verbindungen der allgemeinen Formel

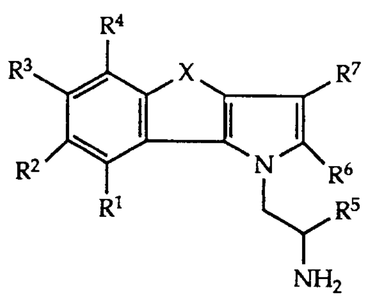

I

worin

| | |
|---|---|
| $R^1$ bis $R^4$ | je Wasserstoff, Halogen, **$C_1$-$C_7$**-Alkyl, Phenyl, **$C_5$-$C_6$**-Cycloalkyl oder **$C_1$-$C_7$**-Alkoxy und $R^2$ noch zusätzlich **$C_1$-$C_7$**-Alkoxycarbonyl, **Acetyloxy** oder Mesyloxy; |
| **$R^5$** | **$C_1$-$C_7$-Alkyl;** |
| **$R^6$ und** $R^7$ | je Wasserstoff oder **$C_1$-$C_7$**-Alkyl; |
| X | $-CH_2CH(C_6H_5)-$, $-CH=C(C_6H_5)-$, $-YCH_2-$, $-CH=CH-$ oder $-(CR^{11}R^{12})_n-$; |
| $R^{11}$ und $R^{12}$ | je Wasserstoff, Phenyl, **$C_1$-$C_7$**-Alkyl oder Halogen; |
| n | 1 bis 3 und |
| Y | O oder S bedeuten, |

sowie pharmazeutisch annehmbare Salze von basischen Verbindungen der Formel I mit Säuren.

**2.** Verbindungen nach Anspruch **1**, worin $R^5$ Methyl bedeutet.

3. Verbindungen nach Anspruch 2, worin $R^1$, $R^3$ und $R^4$ Wasserstoff und $R^2$ Halogen, $C_1$-$C_7$-Alkyl oder Methoxy bedeuten.

4. (S)-2-(4,4,7-Trimethyl-1,4-dihydro-indeno[1,2-b]pyrrol-1-yl)-1-methyl-ethylamin.

5. (S)-2-(7-Methoxy-4,4-dimethyl-1,4-dihydro-indeno[1,2-b]pyrrol-1-yl)-1-methyl-ethylamin.

6. (S)-2-(7-Ethyl-4,4-dimethyl-1,4-dihydro-indeno[1,2-b]pyrrol-1-yl)-1-methyl-ethylamin.

7. (S)-2-(7-Methyl-1,4-dihydro-indeno[1,2-b]pyrrol-1-yl)-1-methyl-ethylamin.

8. (S)-2-(7-Brom-1,4-dihydro-indeno[1,2-b]pyrrol-1-yl)-1-methyl-ethylamin.

9. (S)-2-(7-Methoxy-1,4-dihydro-indeno[1,2-b]pyrrol-1-yl)-1-methyl-ethylamin.

10. (S)-2-(7-Chlor-1,4-dihydro-indeno[1,2-b]pyrrol-1-yl)-1-methyl-ethyl amin.

11. (S)-2-(8-Methoxy-1H-benz[g]indol-1-yl)-1-methyl-ethylamin.

12. Verbindungen der allgemeinen Formel

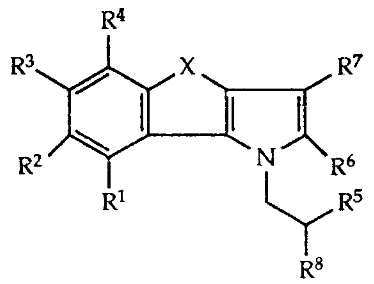

II

worin $R^1$ bis $R^7$ die in Anspruch 1 angegebenen Bedeutungen haben und $R^8$ eine in eine Aminogruppe überführbarer Rest oder eine Hydroxygruppe bedeutet.

13. Verbindungen nach einem der Ansprüche 1 bis 11 zur Anwendung als therapeutische Wirkstoffe.

14. Verbindungen nach einem der Ansprüche 1-11 zur Anwendung als therapeutische Wirkstoffe zur Behandlung oder Verhütung von zentralnervösen Störungen, wie Depressionen, bipolare Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

15. Verfahren zur Herstellung von Verbindungen nach einem der Ansprüche 1 bis 11 dadurch gekennzeichnet, dass man

a) eine Verbindung der allgemeinen Formel

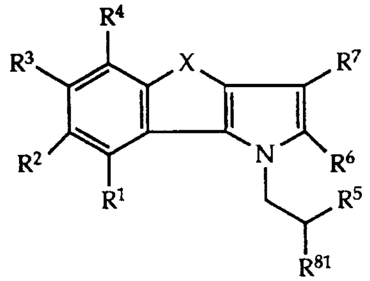

IIa

worin $R^1$ bis $R^7$ die in Anspruch 1 angegebenen Bedeutungen haben und $R^{81}$ einen in eine Aminogruppe überführbaren Rest bedeutet,

in eine entsprechende Aminoverbindung überführt und
b) erwünschtenfalls die erhaltene Verbindung der Formel I in ein pharmazeutisch annehmbares Salz überführt.

**16.** Arzneimittel, enthaltend eine Verbindung nach einem der Ansprüche 1 bis **11** und ein therapeutisch inertes Trägermaterial.

**17.** Arzneimittel nach Anspruch **16** zur Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts.

## Claims

**1.** Compounds of the general formula

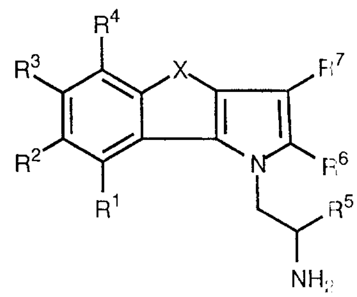

I

wherein

| | |
|---|---|
| $R^1$ to $R^4$ | each signify hydrogen, halogen, $C_1$-$C_7$-alkyl, phenyl, $C_5$-$C_6$-cycloalkyl or $C_1$-$C_7$-alkoxy and $R^2$ additionally signifies $C_1$-$C_7$-alkoxycarbonyl, acetyloxy or mesyloxy; |
| $R^5$ | $C_1$-$C_7$-alkyl |
| $R^6$ and $R^7$ | each signify hydrogen or $C_1$-$C_7$-alkyl; |
| X | signifies $-CH_2CH(C_6H_5)-$, $-CH{=}C(C_6H_5)-$, $-YCH_2-$, $-CH{=}CH-$ or $-(CR^{11}R^{12})_n-$; |
| $R^{11}$ and $R^{12}$ | each signify hydrogen, phenyl, $C_1$-$C_7$-alkyl or halogen; |
| n | signifies 1 to 3 and |

Y signifies O or S,

as well as pharmaceutically usable salts of basic compounds of formula I with acids.

**2.** Compounds according to claim 1, wherein $R^5$ signifies methyl.

**3.** Compounds according to claim 2, wherein $R^1$, $R^3$ and $R^4$ signify hydrogen and $R^2$ signifies halogen, $C_1$-$C_7$-alkyl or methoxy.

**4.** (S)-2-(4,4,7-Trimethyl-1,4-dihydro-indeno[1,2-b]pyrrol-1-yl)-1-methyl-ethylamine.

**5.** (S)-2-(7-Methoxy-4,4-dimethyl-1,4-dihydro-indeno[1,2-b]pyrrol-1-yl)-1-methyl-ethylamine.

**6.** (S)-2-(7-Ethyl-4,4-dimethyl-1,4-dihydro-indeno[1,2-b]pyrrol-1-yl)-1-methyl-ethylamine.

**7.** (S)-2-(7-Methyl-1,4-dihydro-indeno[1,2-b]pyrrol-1-yl)-1-methyl-ethylamine.

**8.** (S)-2-(7-Brom-1,4-dihydro-indeno[1,2-b]pyrrol-1-yl)-1-methyl-ethylamine.

**9.** (S)-2-(7-Methoxy-1,4-dihydro-indeno[1,2-b]pyrrol-1-yl)-1-methyl-ethylamine.

**10.** (S)-2-(7-Chloro-1,4-dihydro-indeno[1,2-b]pyrrol-1-yl)-1-methyl-ethylamine.

**11.** (S)-2-(8-Methoxy-1H-benz[g]indol-1-yl)-1-methyl-ethylamine.

**12.** Compounds of the general formula

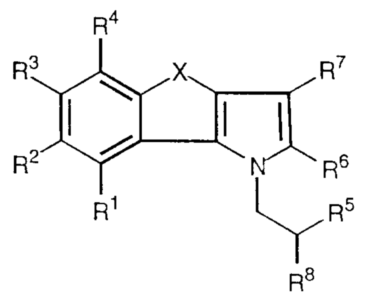

II

wherein $R^1$ to $R^7$ and X have the significances given in claim 1 and $R^8$ signifies a residue convertible into an amino group or a hydroxy group.

**13.** Compounds according to any one of claims 1 to 11 for use as therapeutically active substances.

**14.** Compounds according to any one of claims 1-11 for use as therapeutically active substances for the treatment or prevention of central nervous disorders such as depressions, bipolar disorders, anxiety states, sleep and sexual disorders, psychoses, schizophrenia, migraine and other conditions associated with cephalic pain or pain of a different kind, personality disorders or obsessive compulsive disorders, social phobias or panic states, mental organic disorders, mental disorders in childhood, aggressivity, age-related memory disorders and behavioural disorders, addiction, obesity, bulimia etc.; damages of the nervous system by trauma, stroke, neurodegenerative diseases etc.; cardiovascular disorders such as hypertension, thrombosis, stroke etc.; and gastrointestinal disorders such as dysfunction of the gastrointestinal tract motility

**15.** A process for the manufacture of compounds according to any one of claims 1 to 11, which process comprises

a) converting a compound of the general formula

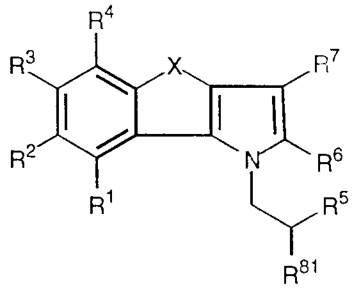

IIa

wherein $R^1$ to $R^7$ and X have the significances given in claim 1 and $R^{81}$ signifies a residue convertible into an amino group,

into a corresponding amino compound and

b) if desired, converting the compound of formula I obtained into a pharmaceutically acceptable salt.

**16.** A medicament containing a compound according to any one of claims 1 to 11 and a therapeutically inert carrier material.

**17.** A medicament according to claim 16 for the treatment or prevention of central nervous disorders such as depressions, bipolar disorders, anxiety states, sleep and sexual disorders, psychoses, schizophrenia, migraine and other conditions associated with cephalic pain or pain of a different kind, personality disorders or obsessive compulsive disorders, social phobias or panic states, mental organic disorders, mental disorders in childhood, aggressivity, age-related memory disorders and behavioural disorders, addiction, obesity, bulimia etc.; damages of the nervous system by trauma, stroke, neurodegenerative diseases etc.; cardiovascular disorders such as hypertension, thrombosis, stroke etc.; and gastrointestinal disorders such as dysfunction of the gastrointestinal tract motility.

## Revendications

**1.** Composés de formule générale

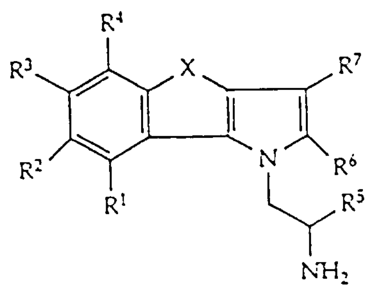

I

dans laquelle

| | |
|---|---|
| $R^1$ à $R^4$ | représentent chacun un hydrogène, un halogène, un alkyle en $C_1$ à $C_7$ , un phényle, un cycloalkyle en $C_5$ à $C_6$ ou un alcoxy en $C_1$ à $C_7$ et $R^2$ représente en outre un alcoxycarbonyle en $C_1$-$C_7$, un acétyloxy ou un mésyloxy; |
| $R^5$ | représente un alkyle en $C_1$ à $C_7$; |
| $R^6$ et $R^7$ | représentent chacun un hydrogène ou un alkyle en $C_1$ à $C_7$ ; |
| X | représente $-CH_2CH(C_6H_5)-$, $-CH=C(C_6H_5)-$, $-YCH_2-$, $-CH=CH-$ ou $-(CR^{11}R^{12})_n$; |

$R^{11}$ et $R^{12}$ représentent chacun un hydrogène, un phényle, un alkyle en $C_1$ à $C_7$ ou un halogène;
n vaut de 1 à 3 et
Y représente O ou S,

ainsi que les sels pharmaceutiquement acceptables de composés basiques de formule I avec des acides.

2. Composés selon la revendication 1, dans laquelle $R^5$ représente un méthyle.

3. Composés selon la revendication 2, dans laquelle $R^1$, $R^3$ et $R^4$ représentent un hydrogène et $R^2$ représente un halogène, un alkyle en $C_1$ à $C_7$ ou un méthoxy.

4. (S)-2-(4,4,7-triméthyl-1,4-dihydro-indéno[1,2-b]-pyrrol-1-yl)-1-méthyl-èthylamine.

5. (S)-2-(7-méthoxy-4,4-diméthyl-1,4-dihydro-indéno[1,2-b]-pyrrol-1-yl)-1-méthyl-éthylamine.

6. (S)-2-(7-éthyl-4,4-diméthy1-1,4-dihydro-indéno[1,2-b]-pyrrol-1-yl)-1-méthyl-éthylamine.

7. (S)-2-(7-méthyl-1,4-dihydro-indéno[1,2-b]-pyrrol-1-yl)-1-méthyl-éthylamine.

8. (S)-2-(7-bromo-1,4-dihydro-indéno[1,2-b]-pyrrol-1-yl)-1-méthyl-éthylamine.

9. (S)-2-(7-méthoxy-1,4-dihydro-indéno[1,2-b]-pyrrol-1-yl)-1-méthyl-éthylamine.

10. (S)-2-(7-chloro-1,4-dihydro-indéno[1,2-b]-pyrrol-1-yl)-1-méthyl-éthylamine.

11. (S)-2-(8-méthoxy-1H-benz[g]-indol-1-yl)-1-méthyl-éthylamine.

12. Composés de formule générale

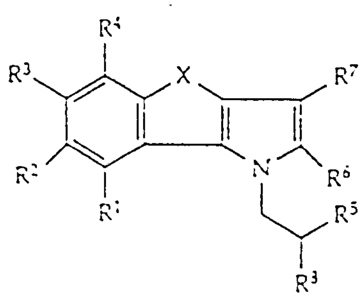

II

dans laquelle $R^1$ à $R^7$ ont les significations données dans la revendication 1 et $R^8$ représente un groupe transformable en un groupe amino ou un groupe hydroxy.

13. Composés selon l'une des revendications 1 à 11 aux fins d'application comme substances actives thérapeutiques.

14. Composés selon l'une des revendications 1-11 aux fins d'application comme substances actives thérapeutiques pour traiter ou prévenir les maladies du système nerveux central comme les dépressions, les désordres bipolaires, les états d'angoisse, les désordres du sommeil et de la sexualité, les psychoses, la schizophrénie, la migraine et d'autres états liés à des maux de tête ou à des douleurs d'autres types, les désordres de la personnalité ou les désordres obsessivo-compulsifs, les phobies sociales ou les accès de panique, les désordres organiques mentaux, les désordres mentaux de l'enfance, l'agressivité, les désordres de la mémoire liés à l'âge et les désordres du comportement, la manie, l'obésité, la boulimie, etc; les désordres du système nerveux par traumatisme, l'apoplexie cérébrale, les maladies neuro-dégénératives, etc; les maladies cardio-vasculaires comme l'hypertension artérielle, la thrombose, l'apoplexie cérébrale, etc; et les maladies gastro-intestinales, comme le dysfonctionnement de la motilité de l'appareil gastro-intestinal.

15. Procédé de préparation de composés selon l'une des revendications 1 à 11, caractérisé en ce que

a) on transforme un composé de formule générale

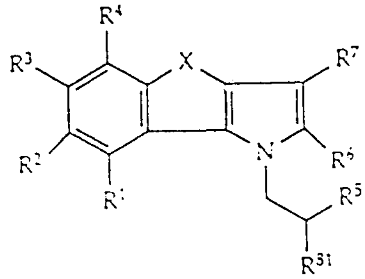

IIa

dans laquelle $R^1$ à $R^7$ ont les significations données dans la revendication 1 et $R^{81}$ représente un radical transformable en un groupe amino et

b) si on le désire on transforme le composé de formule I obtenu en un sel pharmaceutiquement acceptable.

**16.** Médicament contenant un composé selon l'une des revendications 1 à 11 et un support thérapeutiquement inerte.

**17.** Médicament selon la revendication 16 pour traiter ou prévenir les maladies du système nerveux central comme les dépressions, les désordres bipolaires, les états d'angoisse, les désordres du sommeil et de la sexualité, les psychoses, la schizophrénie, la migraine et d'autres états liés à des maux de tête ou à des douleurs d'autres types, les désordres de la personnalité ou les désordres obsessivo-compulsifs, les phobies sociales ou les accès de panique, les désordres organiques mentaux, les désordres mentaux de l'enfance, l'agressivité, les désordres de la mémoire liés à l'âge et les désordres du comportement, la manie, l'obésité, la boulimie, etc; les désordres du système nerveux par traumatisme, l'apoplexie cérébrale, les maladies neuro-dégénératives, etc; les maladies cardio-vasculaires comme l'hypertension artérielle, la thrombose, l'apoplexie cérébrale, etc; et les maladies gastro-intestinales, comme le dysfonctionnement de la motilité de l'appareil gastro-intestinal.